(19) Europäisches Patentamt
European Patent Office
Office européen des brevets

(11) EP 4 095 139 A1

(12) EUROPEAN PATENT APPLICATION
published in accordance with Art. 153(4) EPC

(43) Date of publication:
30.11.2022 Bulletin 2022/48

(21) Application number: 21743887.8

(22) Date of filing: 22.01.2021

(51) International Patent Classification (IPC):
*C07D 473/32* (2006.01) *A61K 31/52* (2006.01)
*A61K 31/5377* (2006.01) *A61P 35/00* (2006.01)

(52) Cooperative Patent Classification (CPC):
A61K 31/52; A61K 31/5377; A61P 35/00;
C07D 473/32

(86) International application number:
PCT/CN2021/073285

(87) International publication number:
WO 2021/147996 (29.07.2021 Gazette 2021/30)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
KH MA MD TN

(30) Priority: 22.01.2020 CN 202010072960

(71) Applicant: CSPC Zhongqi Pharmaceutical Technology
(Shijiazhuang) Co., Ltd.
Shijiazhuang, Hebei 050035 (CN)

(72) Inventors:
- JI, Dehua
Shijiazhuang, Hebei 050035 (CN)
- YANG, Shengyong
Shijiazhuang, Hebei 050035 (CN)
- GUO, Xiaofeng
Shijiazhuang, Hebei 050035 (CN)
- ZHANG, Chen
Shijiazhuang, Hebei 050035 (CN)
- LI, Linli
Shijiazhuang, Hebei 050035 (CN)
- MA, Yuxiu
Shijiazhuang, Hebei 050035 (CN)
- SUN, Xiaowei
Shijiazhuang, Hebei 050035 (CN)
- CUI, Qiaoli
Shijiazhuang, Hebei 050035 (CN)
- GUO, Feng
Shijiazhuang, Hebei 050035 (CN)
- ZHANG, Haohao
Shijiazhuang, Hebei 050035 (CN)

(74) Representative: Johnson, Stephen William et al
Venner Shipley LLP
Byron House
Cambridge Business Park
Cowley Road
Cambridge CB4 0WZ (GB)

# (54) SALT OF ARYLAMINOPURINE DERIVATIVE, PREPARATION METHOD THEREFOR AND USE THEREOF

(57) Provided in the present invention are a salt of an arylaminopurine derivative represented by Formula (2), a preparation method therefor and the use thereof. The salt obtained in the present invention has good crystallinity and significantly improved solubility relative to that in the free form, and the preferred salt and crystal form have low hygroscopicity and can exist stably. Therefore, compared with the free form of arylaminopurine derivatives or other salts, it is easier to prepare same into a medicine.

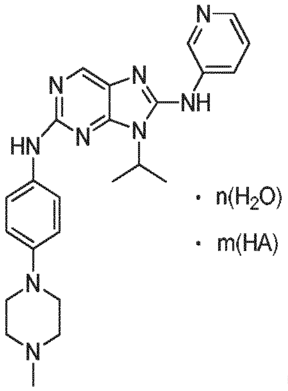

(2)

EP 4 095 139 A1

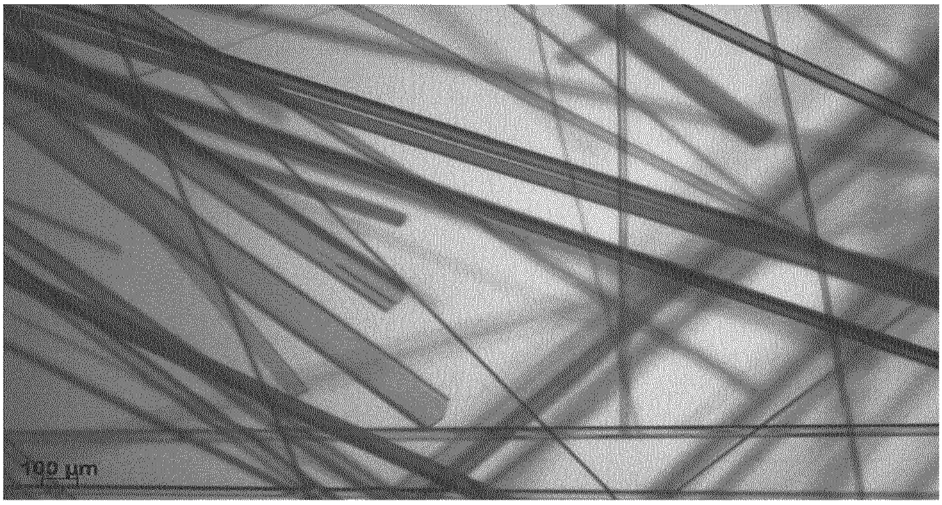


Figure 2

## Description

### Technical Field

The present invention belongs to the field of pharmaceutical chemistry, and in particular, relates to a salt of an arylaminopurine derivative and a preparation method therefor and use thereof.

### Background technology

Compound 1, having a chemical name of 9-isopropyl-2-(4-(4-methylpiperazin-1-yl)anilino)-8-(pyridine-3-amino)-9 H-purine, an arylaminopurine derivative, is a novel multi-targeted protein kinase inhibitor, and its main targets include FLT3, EGFR, Abl, Fyn, Hck, Lck, Lyn, Ret, Yes, and the like. Preclinical pharmacological experiments show that it has a good inhibitory effect and good tolerance on leukemia, non-small cell lung cancer, and other tumors, especially FLT3 mutation-positive, such as FLT3-ITD (internal tandem duplication) acute myeloid leukemia (AML) and non-small cell lung cancer (NSCLC) with EGFR activating mutations. Its mechanism of action is to exert its anti-tumor effect by inhibiting multiple targets or signaling pathways. In particular, for the AML, its anti-leukemia effect is mainly exerted by inhibiting the FLT3 signaling pathway, and for the NSCLC, its anti-tumor effect is mainly exerted by inhibiting the EGFR signaling pathway. It has remarkable efficacy on human leukemia (MV4-11, K562) and lung cancer (HCC827, PC-9) transplantation tumors in nude mice. In terms of anti-leukemia, its activity is better than that of Sunitinib; and in terms of anti-lung cancer, its activity is comparable to that of Gefitinib.

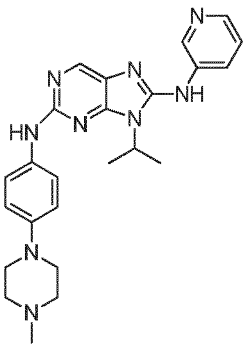

(1)

WO 2011/147066 relates to arylaminopurine derivatives, and discloses the preparation methods and medicinal uses of the free forms of the derivatives, but does not describe and prepare the salts of the compounds of the general formula and the salts of the specific compounds.

The present inventors found that the compound represented by Formula 1 is insoluble in water, which seriously affects its druggability. Therefore, it is necessary to improve the structure of the compound represented by Formula 1 to meet pharmaceutical needs.

### Summary of the Invention

To solve the above problems, the present inventors have made extensive studies on salts of the arylaminopurine derivative represented by Formula 1 to find the pharmaceutical form(s) satisfying pharmaceutical requirements with good solubility, low hygroscopicity, and good stability.

Therefore, one aspect of the present invention provides a salt of the arylaminopurine derivative, wherein said salt is represented by Formula 2:

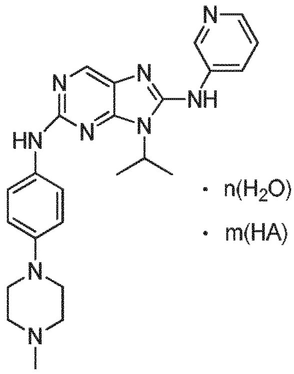

(2)

wherein,

HA is an acid;
$H_2O$ is the water of crystallization;
m is an integer or half-integer from 1 to 4, namely, m=1, 1.5, 2, 2.5, 3, 3.5 or 4;
n is an integer or half-integer from 0 to 5, namely, n=0, 0.5, 1, 1.5, 2, 2.5, 3, 3.5, 4, 4.5, or 5.

Preferably, the acid is selected from a group consisting of hydrochloric acid, methanesulfonic acid, L-malic acid, L-tartaric acid, oxalic acid, succinic acid, acetic acid, or sulfuric acid; preferably hydrochloric acid, L-malic acid, L-tartaric acid, oxalic acid, succinic acid, acetic acid, or sulfuric acid; more preferably hydrochloric acid, L-malic acid, L-tartaric acid, oxalic acid, succinic acid or acetic acid; further preferably hydrochloric acid.
In one embodiment of the present invention, the salt of the arylaminopurine derivative is characterized in that the salt is a hydrochloride represented by Formula 3:

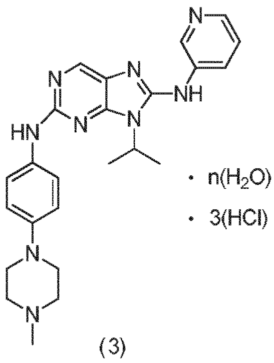

(3) ,

n is 0, 0.5, 1, 1.5, 2, 2.5, 3, 3.5, 4, 4.5, or 5;
preferably, the salt is a hydrochloride represented by Formula 3':

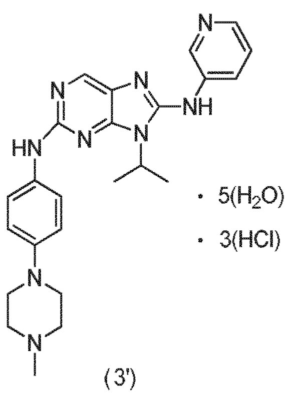

(3') ;

preferably, the hydrochloride represented by Formula 3 or Formula 3' has an X-ray powder diffraction pattern comprising peaks at 2θ values of 8.5±0.2°, 11.8±0.2°, 19.6±0.2°, 25.2±0.2°, 27.2±0.2° as measured with CuKα radiation; more preferably, the hydrochloride represented by Formula 3 or Formula 3' has an X-ray powder diffraction pattern comprising peaks at 2θ values of 8.5±0.2°, 11.8±0.2°, 12.6±0.2°, 19.6±0.2°, 20.0±0.2°, 23.7±0.2°, 25.2±0.2°, 27.2±0.2° as measured with CuKα radiation; further preferably, the hydrochloride represented by Formula 3 or Formula 3' has an X-ray powder diffraction pattern comprising peaks at 2θ values of 7.3±0.2°, 8.5±0.2°, 9.0±0.2°, 11.8±0.2°, 12.6±0.2°, 14.3±0.2°, 18.1±0.2°, 19.6±0.2°, 20.0±0.2°, 21.1±0.2°, 21.9±0.2°, 23.7±0.2°, 25.2±0.2°, 26.1±0.2°, 27.2±0.2° or at 2θ values of 7.3±0.2°, 8.5±0.2°, 9.1±0.2°, 11.8±0.2°, 12.6±0.2°, 14.3±0.2°, 18.1±0.2°, 19.6±0.2°, 20.0±0.2°, 21.1±0.2°, 21.9±0.2°, 23.7±0.2°, 25.2±0.2°, 26.1±0.2°, 27.2±0.2° as measured with CuKα radiation; more further preferably, the hydrochloride represented by Formula 3 or Formula 3' has an X-ray powder diffraction pattern substantially as shown in Figure 1 or Figure 3, as measured with CuKα radiation.

Preferably, the single crystal of the hydrochloride represented by Formula 3 or Formula 3', as measured with CuKα radiation, belongs to the triclinic system, space group $P\bar{1}$, and has the unit cell parameters: {a=7.04142(7)Å, b=12.15291(7)Å, c=18.13188(10)Å, α=93.2215(5)°, β=95.3039(6)°, γ=91.9554(6)°, V=1541.32(2)Å$^3$}.

In one embodiment of the present invention, the salt of the arylaminopurine derivative is a mesylate represented by Formula 4, Formula 5, or Formula 6:

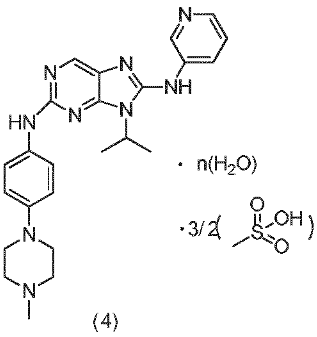
(4) ,
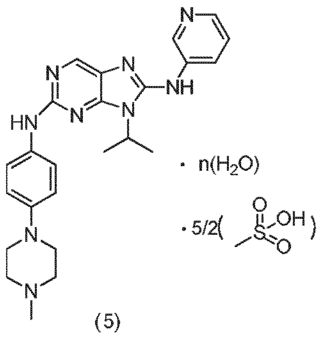
(5) ,
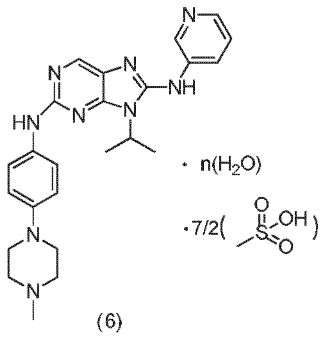
(6) ,

n is 0, 0.5, 1, 1.5, 2, 2.5, 3, 3.5, 4, 4.5, or 5;

preferably, the salt is a mesylate represented by Formula 4', Formula 5', or Formula 6':

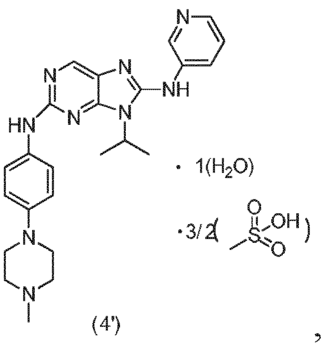
(4') ,
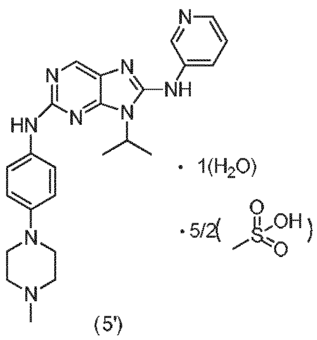
(5') ,
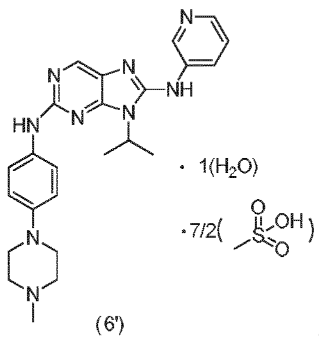
(6') ;

preferably, the mesylate represented by Formula 4 or Formula 4' has an X-ray powder diffraction pattern comprising peaks at 2θ values of 6.8±0.2°, 15.1±0.2°, 16.3±0.2°, 21.0±0.2°, 25.0±0.2° as measured with CuKα radiation; more preferably, the mesylate represented by Formula 4 or Formula 4' has an X-ray powder diffraction pattern comprising peaks at 2θ values of 6.8±0.2°, 8.6±0.2°, 10.7±0.2°, 12.6±0.2°, 13.1±0.2°, 13.4±0.2°, 15.1±0.2°, 16.3±0.2°, 17.7±0.2°, 19.0±0.2°, 19.9±0.2°, 21.0±0.2°, 25.0±0.2° as measured with CuKα radiation; further preferably, the mesylate represented by Formula 4 or Formula 4' has an X-ray powder diffraction pattern substantially as shown in Figure 4, as measured with CuKα radiation.

Or, the mesylate represented by Formula 5 or Formula 5' has an X-ray powder diffraction pattern comprising peaks at 2θ values of 6.1±0.2°, 6.4±0.2°, 17.4±0.2°, 18.9±0.2°, 19.3±0.2°, 24.4±0.2°, 26.4±0.2° or at 2θ values of 6.1±0.2°, 6.4±0.2°, 17.5±0.2°, 18.9±0.2°, 19.3±0.2°, 24.4±0.2°, 26.4±0.2° as measured with CuKα radiation; preferably, the mesylate represented by Formula 5 or Formula 5' has an X-ray powder diffraction pattern comprising peaks

at 2θ values of 6.1±0.2°, 6.4±0.2°, 11.7±0.2°, 12.4±0.2°, 16.0±0.2°, 16.6±0.2°, 16.9±0.2°, 17.4±0.2°, 18.0±0.2°, 18.9±0.2°, 19.3±0.2°, 19.9±0.2°, 20.2±0.2°, 23.4±0.2°, 24.4±0.2°, 26.4±0.2°, 27.3±0.2° or at 2θ values of 6.1±0.2°, 6.4±0.2°, 11.7±0.2°, 12.4±0.2°, 16.0±0.2°, 16.6±0.2°, 16.9±0.2°, 17.5±0.2°, 18.0±0.2°, 18.9±0.2°, 19.3±0.2°, 19.9±0.2°, 20.2±0.2°, 23.4±0.2°, 24.4±0.2°, 26.4±0.2°, 27.3±0.2° as measured with CuKα radiation; further preferably, the mesylate represented by Formula 5 or Formula 5' has an X-ray powder diffraction pattern substantially as shown in Figure 5, as measured with CuKα radiation.

Or, the mesylate represented by Formula 6 or Formula 6' has an X-ray powder diffraction pattern comprising peaks at 2θ values of 4.9±0.2°, 11.5±0.2°, 14.5±0.2°, 18.5±0.2°, 18.9±0.2° as measured with CuKα radiation; preferably, the mesylate represented by Formula 6 or Formula 6' has an X-ray powder diffraction pattern comprising peaks at 2θ values of 4.9±0.2°, 6.0±0.2°, 9.7±0.2°, 10.5±0.2°, 11.5±0.2°, 12.3±0.2°, 14.5±0.2°, 15.1±0.2°, 16.8±0.2°, 18.5±0.2°, 18.9±0.2°, 21.6±0.2°, 22.0±0.2°, 22.3±0.2°, 22.8±0.2°, 23.4±0.2°, 24.3±0.2°, 25.4±0.2°, 26.7±0.2°, 27.3±0.2° as measured with CuKα radiation; further preferably, the mesylate represented by Formula 6 or Formula 6' has an X-ray powder diffraction pattern substantially as shown in Figure 6, as measured with CuKα radiation.

In one embodiment of the present invention, the salt of the arylaminopurine derivative is an L-malate represented by Formula 7:

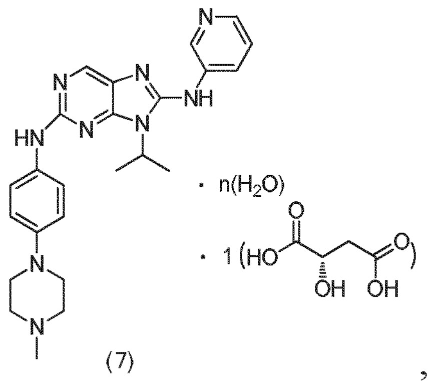

(7)

,

n is 0, 0.5, 1, 1.5, 2, 2.5, 3, 3.5, 4, 4.5, or 5;
preferably, the salt is an L-malate represented by Formula 7':

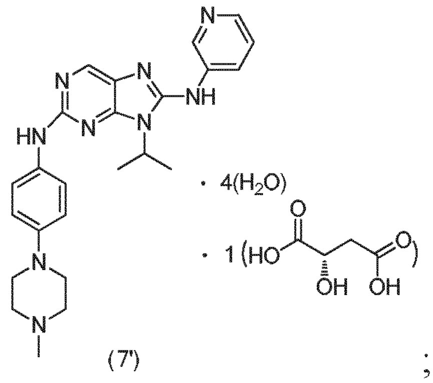

(7')

;

preferably, the L-malate represented by Formula 7 or Formula 7' has an X-ray powder diffraction pattern comprising peaks at 2θ values of 7.0±0.2°, 9.3±0.2°, 17.6±0.2°, 19.7±0.2°, 25.9±0.2° as measured with CuKα radiation; more preferably, the L-malate represented by Formula 7 or Formula 7' has an X-ray powder diffraction pattern comprising peaks at 2θ values of 7.0±0.2°, 9.3±0.2°, 12.0±0.2°, 12.9±0.2°, 14.0±0.2°, 16.6±0.2°, 17.6±0.2°, 18.5±0.2°, 19.7±0.2°, 24.2±0.2°, 25.2±0.2°, 25.9±0.2°, 27.5±0.2° or at 2θ values of 7.0±0.2°, 9.3±0.2°, 12.0±0.2°, 12.9±0.2°, 14.0±0.2°, 16.6±0.2°, 17.6±0.2°, 18.5±0.2°, 19.7±0.2°, 23.0±0.2°, 24.2±0.2°, 25.2±0.2°, 25.9±0.2°, 27.5±0.2° as measured with CuKα radiation; further preferably, the L-malate represented by Formula 7 or Formula 7' has an X-ray powder diffraction pattern substantially as shown in Figure 7, as measured with CuKα radiation.

In one embodiment of the present invention, the salt of the arylaminopurine derivative is an L-tartrate represented

by Formula 8, Formula 9, or Formula 10:

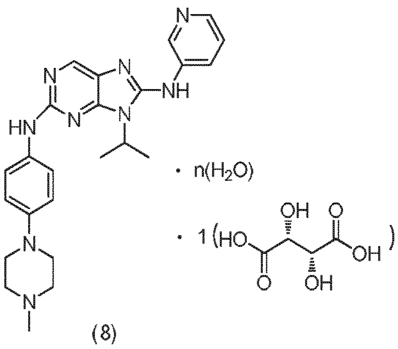

(8) ,

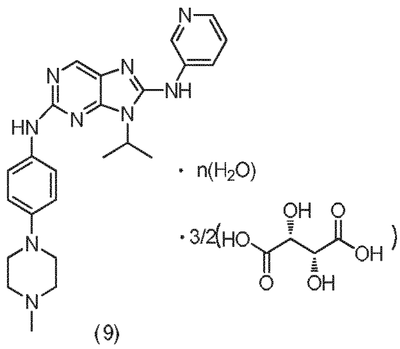

(9) ,

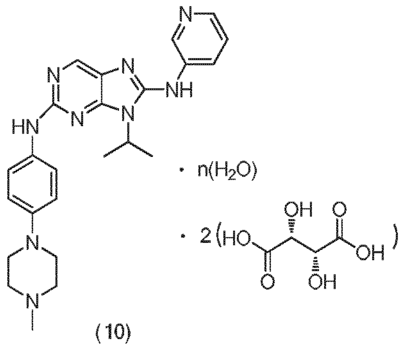

(10) ,

n is 0, 0.5, 1, 1.5, 2, 2.5, 3, 3.5, 4, 4.5, or 5;

preferably, the salt is an L-tartrate represented by Formula 8', Formula 9', or Formula 10':

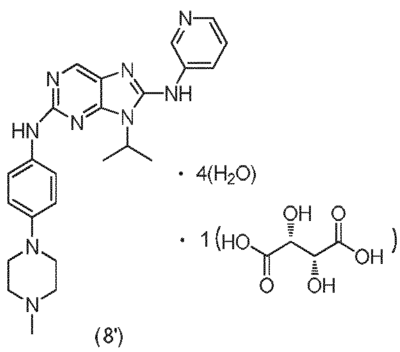

(8') ,

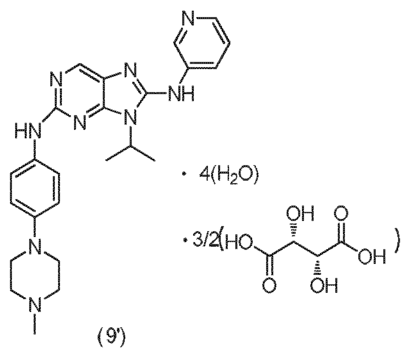

(9') ,

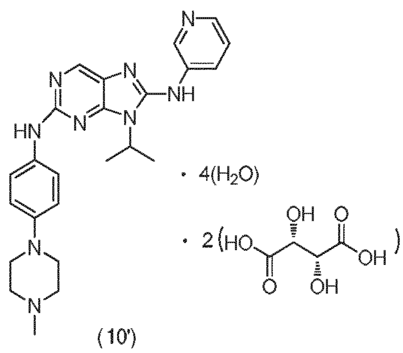

(10') ;

preferably, the L-tartrate represented by Formula 8 or Formula 8' has an X-ray powder diffraction pattern comprising

peaks at 2θ values of 6.9±0.2°, 9.1±0.2°, 17.8±0.2°, 19.4±0.2°, 25.5±0.2° as measured with CuKα radiation; more preferably, the L-tartrate represented by Formula 8 or Formula 8' has an X-ray powder diffraction pattern comprising peaks at 2θ values of 6.9±0.2°, 9.1±0.2°, 12.9±0.2°, 13.8±0.2°, 16.5±0.2°, 17.8±0.2°, 19.4±0.2°, 20.1±0.2°, 25.5±0.2°, 26.9±0.2° as measured with CuKα radiation; further preferably, the L-tartrate represented by Formula 8 or Formula 8' has an X-ray powder diffraction pattern substantially as shown in Figure 8, as measured with CuKα radiation.

Or, the L-tartrate represented by Formula 9 or Formula 9' has an X-ray powder diffraction pattern comprising peaks at 2θ values of 8.5±0.2°, 14.8±0.2°, 17.1±0.2°, 18.8±0.2°, 24.6±0.2°, 26.1±0.2° as measured with CuKα radiation; the L-tartrate represented by Formula 9 or Formula 9' has an X-ray powder diffraction pattern comprising peaks at 2θ values of 8.5±0.2°, 9.8±0.2°, 10.1±0.2°, 11.3±0.2°, 13.7±0.2°, 14.8±0.2°, 15.4±0.2°, 16.3±0.2°, 17.1±0.2°, 17.6±0.2°, 18.8±0.2°, 20.5±0.2°, 22.3±0.2°, 24.6±0.2°, 26.1±0.2° as measured with CuKα radiation; further preferably, the L-tartrate represented by Formula 9 or Formula 9' has an X-ray powder diffraction pattern substantially as shown in Figure 9, as measured with CuKα radiation.

Or, the L-tartrate represented by Formula 10 or Formula 10' has an X-ray powder diffraction pattern comprising peaks at 2θ values of 8.3±0.2°, 8.9±0.2°, 9.5±0.2°, 14.8±0.2°, 17.7±0.2°, 21.0±0.2°, 24.0±0.2° as measured with CuKα radiation; preferably, the L-tartrate represented by Formula 10 or Formula 10' has an X-ray powder diffraction pattern comprising peaks at 2θ values of 7.0±0.2°, 8.3±0.2°, 8.9±0.2°, 9.5±0.2°, 12.5±0.2°, 13.1±0.2°, 14.8±0.2°, 16.0±0.2°, 17.7±0.2°, 18.1±0.2°, 19.2±0.2°, 21.0±0.2°, 23.6±0.2°, 24.0±0.2°, 25.3±0.2°, 26.7±0.2° as measured with CuKα radiation; further preferably, the L-tartrate represented by Formula 10 or Formula 10' has an X-ray powder diffraction pattern substantially as shown in Figure 10, as measured with CuKα radiation.

In one embodiment of the present invention, the salt of the arylaminopurine derivative is an oxalate represented by Formula 11, or Formula 12:

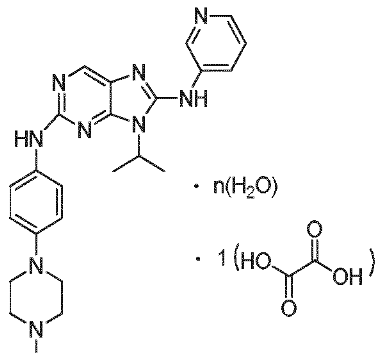
(11) ,

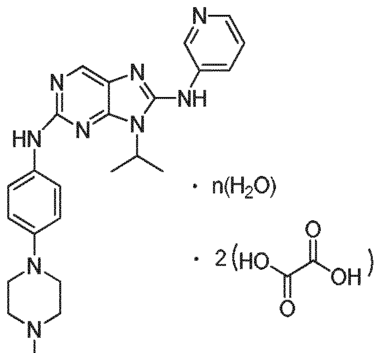
(12) ,

n is 0, 0.5, 1, 1.5, 2, 2.5, 3, 3.5, 4, 4.5, or 5;

preferably, the salt is an oxalate represented by Formula 11', or Formula 12':

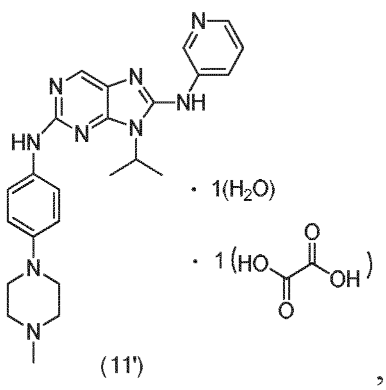
(11') ,

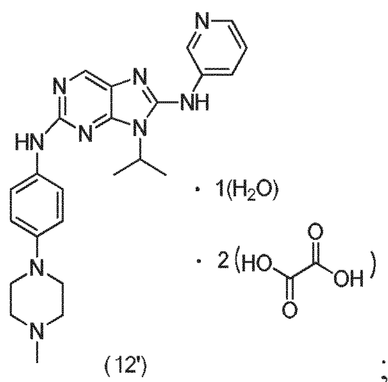
(12') ;

preferably, the oxalate represented by Formula 11 or Formula 11' has an X-ray powder diffraction pattern comprising peaks at 2θ values of 8.1±0.2°, 8.4±0.2°, 9.0±0.2°, 14.1±0.2°, 16.7±0.2°, 25.6±0.2° as measured with CuKα radiation; preferably, the oxalate represented by Formula 11 or Formula 11' has an X-ray powder diffraction pattern

comprising peaks at 2θ values of 8.1±0.2°, 8.4±0.2°, 9.0±0.2°, 14.1±0.2°, 14.8±0.2°, 16.7±0.2°, 17.9±0.2°, 18.5±0.2°, 19.6±0.2°, 23.6±0.2°, 25.6±0.2° as measured with CuKα radiation; further preferably, the oxalate represented by Formula 11 or Formula 11' has an X-ray powder diffraction pattern substantially as shown in Figure 11, as measured with CuKα radiation.

Or, the oxalate represented by Formula 12 or Formula 12' has an X-ray powder diffraction pattern comprising peaks at 2θ values of 7.1±0.2°, 12.2±0.2°, 14.2±0.2°, 16.4±0.2°, 17.7±0.2°, 19.0±0.2°, 24.4±0.2° as measured with CuKα radiation; preferably, the oxalate represented by Formula 12 or Formula 12' has an X-ray powder diffraction pattern comprising peaks at 2θ values of 7.1±0.2°, 8.3±0.2°, 12.2±0.2°, 14.2±0.2°, 16.4±0.2°, 17.7±0.2°, 18.6±0.2°, 19.0±0.2°, 24.4±0.2° as measured with CuKα radiation; further preferably, the oxalate represented by Formula 12 or Formula 12' has an X-ray powder diffraction pattern substantially as shown in Figure 12, as measured with CuKα radiation.

In one embodiment of the present invention, the salt of the arylaminopurine derivative is a succinate represented by Formula 13, or Formula 14:

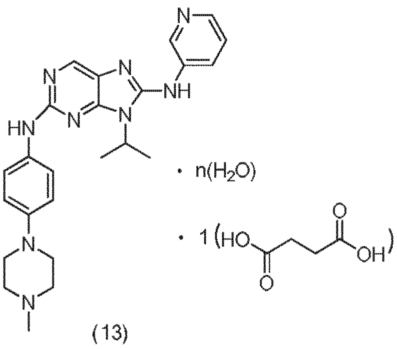
(13) ,
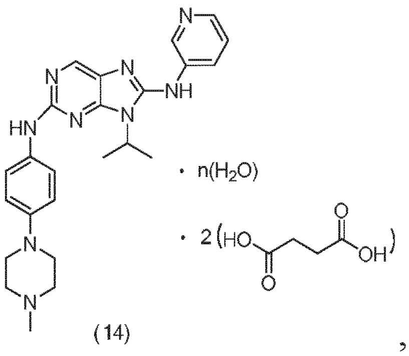
(14) ,

n is 0, 0.5, 1, 1.5, 2, 2.5, 3, 3.5, 4, 4.5, or 5;

preferably, the salt is a succinate represented by Formula 13', or Formula 14':

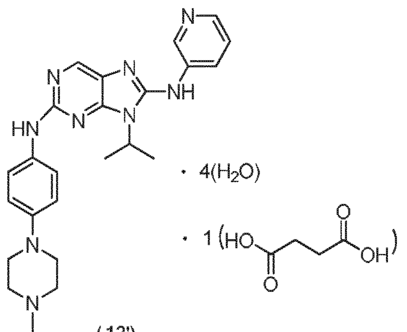
(13') ,
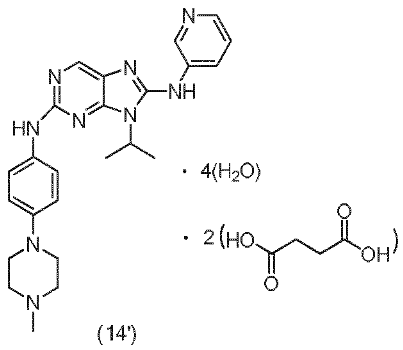
(14') ;

preferably, the succinate represented by Formula 13 or Formula 13' has an X-ray powder diffraction pattern comprising peaks at 2θ values of 7.0±0.2°, 9.1±0.2°, 11.3±0.2°, 16.8±0.2°, 20.4±0.2°, 21.0±0.2°, 22.4±0.2° or at 2θ values of 7.0±0.2°, 9.1±0.2°, 18.5±0.2°, 20.4±0.2°, 21.0±0.2°, 22.4±0.2°, 27.1±0.2° as measured with CuKα radiation; preferably, the succinate represented by Formula 13 or Formula 13' has an X-ray powder diffraction pattern comprising peaks at 2θ values of 7.0±0.2°, 9.1±0.2°, 11.3±0.2°, 13.1±0.2°, 13.8±0.2°, 14.4±0.2°, 16.0±0.2°, 16.8±0.2°, 17.7±0.2°, 18.5±0.2°, 20.4±0.2°, 21.0±0.2°, 22.4±0.2°, 24.2±0.2°, 25.9±0.2°, 27.1±0.2° as measured with CuKα radiation; further preferably, the succinate represented by Formula 13 or Formula 13' has an X-ray powder diffraction pattern substantially as shown in Figure 13, as measured with CuKα radiation.

Or, the succinate represented by Formula 14 or Formula 14' has an X-ray powder diffraction pattern comprising peaks at 2θ values of 7.0±0.2°, 9.2±0.2°, 17.6±0.2°, 18.4±0.2°, 19.7±0.2°, 25.8±0.2°, 27.3±0.2° as measured with CuKα radiation; preferably, the succinate represented by Formula 14 or Formula 14' has an X-ray powder diffraction pattern comprising peaks at 2θ values of 7.0±0.2°, 9.2±0.2°, 11.9±0.2°, 16.7±0.2°, 17.6±0.2°, 18.4±0.2°, 19.7±0.2°, 23.0±0.2°, 24.1±0.2°, 25.2±0.2°, 25.8±0.2°, 27.3±0.2° or at 2θ values of 7.0±0.2°, 9.2±0.2°, 11.9±0.2°, 16.7±0.2°, 17.6±0.2°, 18.4±0.2°, 19.7±0.2°, 20.3±0.2°, 23.0±0.2°, 24.1±0.2°, 25.2±0.2°, 25.8±0.2°, 27.3±0.2° as measured

with CuKα radiation; further preferably, the succinate represented by Formula 14 or Formula 14' has an X-ray powder diffraction pattern substantially as shown in Figure 14, as measured with CuKα radiation.

In one embodiment of the present invention, the salt of the arylaminopurine derivative is an acetate represented by Formula 15, or Formula 16:

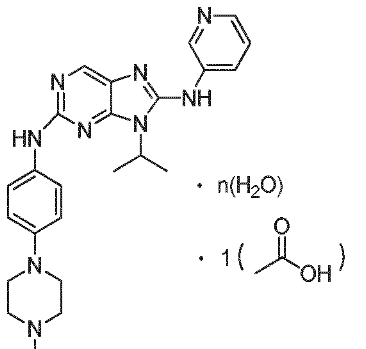

(15) ,

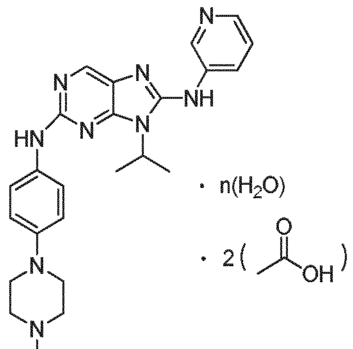

(16) ,

n is 0, 0.5, 1, 1.5, 2, 2.5, 3, 3.5, 4, 4.5, or 5;

preferably, the salt is an acetate represented by Formula 15', or Formula 16':

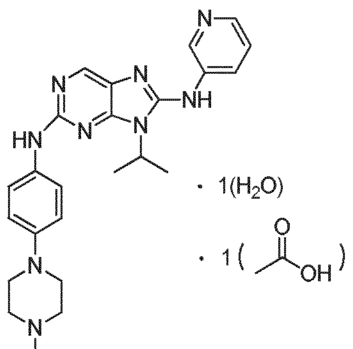

(15') ,

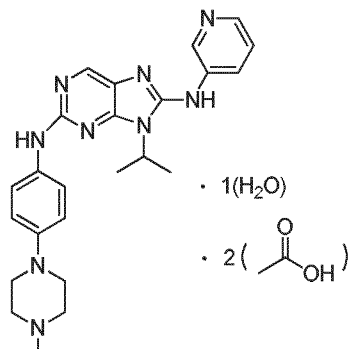

(16') ;

preferably, the acetate represented by Formula 15 or Formula 15' has an X-ray powder diffraction pattern comprising peaks at 2θ values of 10.9±0.2°, 12.6±0.2°, 15.1±0.2°, 17.8±0.2°, 19.2±0.2°, 19.6±0.2°, 21.0±0.2°, 21.8±0.2°, 22.3±0.2°, 24.6±0.2°, 25.4±0.2° as measured with CuKα radiation; preferably, the acetate represented by Formula 15 or Formula 15' has an X-ray powder diffraction pattern comprising peaks at 2θ values of 6.3±0.2°, 8.9±0.2°, 10.9±0.2°, 11.5±0.2°, 12.2±0.2°, 12.6±0.2°, 15.1±0.2°, 17.8±0.2°, 19.2±0.2°, 19.6±0.2°, 21.0±0.2°, 21.8±0.2°, 22.3±0.2°, 24.6±0.2°, 25.4±0.2° as measured with CuKα radiation; further preferably, the acetate represented by Formula 15 or Formula 15' has an X-ray powder diffraction pattern substantially as shown in Figure 15, as measured with CuKα radiation.

Or, the acetate represented by Formula 16 or Formula 16' has an X-ray powder diffraction pattern comprising peaks at 2θ values of 6.2±0.2°, 12.2±0.2°, 16.1±0.2°, 17.5±0.2°, 23.4±0.2°, 24.8±0.2° or at 2θ values of 6.2±0.2°, 12.2±0.2°, 17.5±0.2°, 21.5±0.2°, 23.4±0.2°, 24.8±0.2° as measured with CuKα radiation; preferably, the acetate represented by Formula 16 or Formula 16' has an X-ray powder diffraction pattern comprising peaks at 2θ values of 6.2±0.2°, 8.1±0.2°, 9.1±0.2°, 12.2±0.2°, 15.0±0.2°, 16.1±0.2°, 17.5±0.2°, 18.2±0.2°, 20.7±0.2°, 21.5±0.2°, 23.4±0.2°, 24.8±0.2°, 28.8±0.2° as measured with CuKα radiation; further preferably, the acetate represented by Formula 16 or Formula 16' has an X-ray powder diffraction pattern substantially as shown in Figure 16, as measured with CuKα radiation.

In one embodiment of the present invention, the salt of the arylaminopurine derivative is a sulfate represented by Formula 17, or Formula 18:

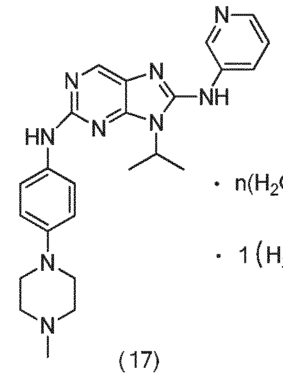

· n($H_2O$)

· 1 ($H_2SO_4$)

(17) ,

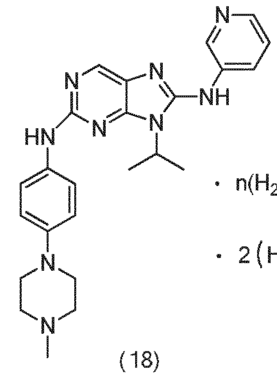

· n($H_2O$)

· 2 ($H_2SO_4$)

(18) ,

n is 0, 0.5, 1, 1.5, 2, 2.5, 3, 3.5, 4, 4.5, or 5;
preferably, the salt is a sulfate represented by Formula 17', or Formula 18':

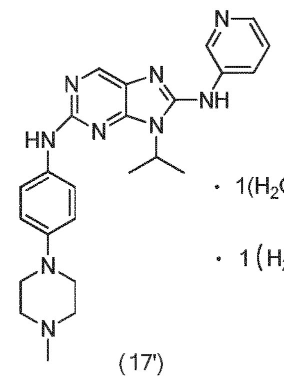

· 1($H_2O$)

· 1 ($H_2SO_4$)

(17') ,

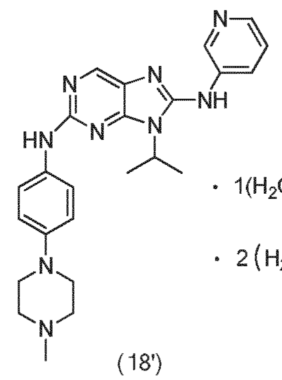

· 1($H_2O$)

· 2 ($H_2SO_4$)

(18') ;

preferably, the sulfate represented by Formula 17 or Formula 17' has an X-ray powder diffraction pattern comprising peaks at 2θ values of 4.8±0.2°, 7.0±0.2°, 9.5±0.2°, 13.6±0.2°, 15.7±0.2°, 18.6±0.2°, 21.6±0.2°, 25.7±0.2° or at 2θ values of 4.8±0.2°, 7.0±0.2°, 9.2±0.2°, 9.5±0.2°, 13.6±0.2°, 15.7±0.2°, 18.6±0.2°, 21.6±0.2°, 25.7±0.2° as measured with CuKα radiation; preferably, the sulfate represented by Formula 17 or Formula 17' has an X-ray powder diffraction pattern comprising peaks at 2θ values of 4.8±0.2°, 7.0±0.2°, 8.6±0.2°, 9.2±0.2°, 9.5±0.2°, 11.6±0.2°, 12.8±0.2°, 13.6±0.2°, 15.7±0.2°, 17.6±0.2°, 18.6±0.2°, 20.5±0.2°, 21.6±0.2°, 23.8±0.2°, 25.7±0.2° as measured with CuKα radiation; further preferably, the sulfate represented by Formula 17 or Formula 17' has an X-ray powder diffraction pattern substantially as shown in Figure 17, as measured with CuKα radiation.

Or, the sulfate represented by Formula 18 or Formula 18' has an X-ray powder diffraction pattern comprising peaks at 2θ values of 8.6±0.2°, 9.6±0.2°, 15.7±0.2°, 19.3±0.2°, 20.0±0.2°, 21.9±0.2°, 26.6±0.2° or at 2θ values of 8.6±0.2°, 9.6±0.2°, 15.7±0.2°, 17.1±0.2°, 19.3±0.2°, 20.0±0.2°, 26.6±0.2° as measured with CuKα radiation; the sulfate represented by Formula 18 or Formula 18' has an X-ray powder diffraction pattern comprising peaks at 2θ values of 8.6±0.2°, 9.6±0.2°, 15.7±0.2°, 16.5±0.2°, 17.1±0.2°, 19.3±0.2°, 20.0±0.2°, 21.9±0.2°, 23.5±0.2°, 24.4±0.2°, 26.6±0.2° as measured with CuKα radiation; further preferably, the sulfate represented by Formula 18 or Formula 18' has an X-ray powder diffraction pattern substantially as shown in Figure 18, as measured with CuKα radiation.

In another aspect, the present invention provides a pharmaceutical composition comprising the aforementioned salt represented by Formula 2 of the arylaminopurine derivative.

In another aspect, the present invention provides a pharmaceutical composition, comprising the aforementioned salt represented by Formula 2 of the arylaminopurine derivative, and a pharmaceutically acceptable adjuvant.

In another aspect, the present invention provides a pharmaceutical composition, comprising a pharmaceutically effective amount of the aforementioned salt represented by Formula 2 of the arylaminopurine derivative, and a pharmaceutically acceptable adjuvant. The pharmaceutically effective amount can be 0.1-99.9 wt%, for example, 1-90 wt%, 5-80 wt%, 5-65 wt%, 5-55 wt%, 5-45 wt%, or 5-40 wt%, based on the total weight of the pharmaceutical composition.

In the context of the present application, the term "pharmaceutically acceptable adjuvant" includes solvents, propellants, solubilizers, cosolvents, emulsifiers, colorants, binders, disintegrants, fillers, lubricants, wetting agents, osmotic pressure regulators, stabilizers, glidants, correctants, preservatives, suspending agents, coating materials,

flavoring agents, anti-adherents, antioxidants, chelating agents, penetration enhancers, pH regulators, buffering agents, plasticizers, surfactants, foaming agents, defoaming agents, thickeners, inclusion agents, humectants, absorbents, diluents, flocculating agents and deflocculating agents, filter aids, release retardants, and the like. Those skilled in the art can select specific pharmaceutically acceptable adjuvants according to actual requirements. Knowledge of adjuvants is well known to those skilled in the art, for example, with reference to "Pharmaceutics" (Editor-in-Chief by Cui Fude, 5th edition, People's Medical Publishing House, 2003).

In another aspect, the present invention provides use of the aforementioned salt represented by Formula 2 of the arylaminopurine derivative or the pharmaceutical composition containing the same in inhibiting the activity of one or more of FLT3, EGFR, Abl, Fyn, Hck, Lck, Lyn, Ret, Yes, VEGFR2, ALK, BTK, c-KIT, c-SRC, FGFR1, KDR, MET and PDGFR$\alpha$ kinases.

In another aspect, the present invention provides use of the aforementioned salt represented by Formula 2 of the arylaminopurine derivative or the pharmaceutical composition containing the same in manufacture of a medicament as the protein kinase inhibitor, wherein the kinase is selected from FLT3, EGFR, Abl, Fyn, Hck, Lck, Lyn, Ret, Yes, VEGFR2, ALK, BTK, c-KIT, c-SRC, FGFR1, KDR, MET or PDGFR$\alpha$, for example, the kinase is selected from FLT3, EGFR, Abl, Fyn, Hck, Lck, Lyn, Ret or Yes;

Preferably, the medicament as the protein kinase inhibitor is an antitumor drug, and the tumor is preferably leukemia or lung cancer, more preferably acute myeloid leukemia such as FLT3 mutation-positive acute myeloid leukemia (further such as FLT3-ITD acute myeloid leukemia), chronic myeloid leukemia (such as Ph-positive chronic myeloid leukemia), or non-small cell lung cancer (such as non-small cell lung cancer with EGFR activating mutations).

In another aspect, the present invention provides use of the aforementioned salt represented by Formula 2 of the arylaminopurine derivative or the pharmaceutical composition containing the same in manufacture of a medicament for treating or preventing a disorder; preferably, the disorder is a disorder caused by one or more of FLT3, EGFR, Abl, Fyn, Hck, Lck, Lyn, Ret, Yes, VEGFR2, ALK, BTK, c-KIT, c-SRC, FGFR1, KDR, MET and PDGFR$\alpha$ kinases; more preferably, the disorder is selected from non-small cell lung cancer, acute myeloid leukemia, chronic myelocytic leukemia, chronic myeloid leukemia, squamous cell carcinoma, mammary cancer, colorectal cancer, liver cancer, stomach cancer, and malignant melanoma; further preferably, the disorder is selected from human non-small cell lung cancer, human acute myeloid leukemia, human chronic myelocytic leukemia, human chronic myeloid leukemia, human squamous cell carcinoma, human mammary cancer, human colorectal cancer, human liver cancer, human stomach cancer, and human malignant melanoma.

In another aspect, the present invention provides use of the aforementioned salt represented by Formula 2 of the arylaminopurine derivative or the pharmaceutical composition containing the same in manufacture of a medicament for treating or preventing acute myeloid leukemia; preferably, the acute myeloid leukemia is selected from relapsed and/or refractory acute myeloid leukemia, or, the acute myeloid leukemia is selected from acute myeloid leukemia with FLT3-ITD mutations and/or TKD mutations, relapsed and/or refractory acute myeloid leukemia that had been unsuccessfully treated with Type II FLT3 inhibitor(s) (e.g. sorafenib), or, DEK-CAN positive acute myeloid leukemia with FLT3-ITD mutations; more preferably, the acute myeloid leukemia is acute myeloid leukemia with FLT3-ITD$^{high}$ mutations; and/or the unfavorable prognostic factors of the acute myeloid leukemia are 0-2; and/or the FAB classification of the acute myeloid leukemia is subtype M2, M4, or M5, preferably subtype M5. Further, the aforementioned use in manufacture of a medicament for treating or preventing acute myeloid leukemia is described in detail in patent application PCT/CN2020/127449, the disclosure of which is incorporated herein by reference as if set forth in this application.

In another aspect, the present invention provides a pharmaceutical composition for treating or preventing a disorder, wherein said composition contains a pharmaceutically effective amount of the aforementioned salt represented by Formula 2 of the arylaminopurine derivative and a pharmaceutically acceptable adjuvant; preferably, the disorder is a disorder caused by one or more of FLT3, EGFR, Abl, Fyn, Hck, Lck, Lyn, Ret, Yes, VEGFR2, ALK, BTK, c-KIT, c-SRC, FGFR1, KDR, MET and PDGFR$\alpha$ kinases; more preferably, the disorder is selected from non-small cell lung cancer, acute myeloid leukemia, chronic myelocytic leukemia, chronic myeloid leukemia, squamous cell carcinoma, mammary cancer, colorectal cancer, liver cancer, stomach cancer, and malignant melanoma; further preferably, the disorder is selected from human non-small cell lung cancer, human acute myeloid leukemia, human chronic myelocytic leukemia, human chronic myeloid leukemia, human squamous cell carcinoma, human mammary cancer, human colorectal cancer, human liver cancer, human stomach cancer, and human malignant melanoma. The pharmaceutically effective amount can be 0.1-99.9 wt%, for example, 1-90 wt%, 5-80 wt%, 5-65 wt%, 5-55 wt%, 5-45 wt%, or 5-40 wt%, based on the total weight of the pharmaceutical composition.

In another aspect, the present invention provides a method for preparing a salt represented by Formula 2 of the arylaminopurine derivative, which comprises a reaction of an arylaminopurine derivative represented by Formula 1 and an acid is performed in the presence of water and an organic solvent to obtain the salt represented by Formula 2 of the arylaminopurine derivative:

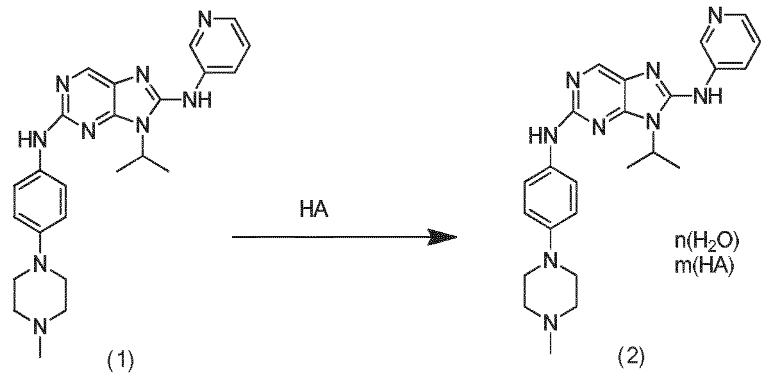


(1) (2) ,

wherein,

HA is an acid;
$H_2O$ is the water of crystallization;
m is an integer or half-integer from 1 to 4;
n is an integer or half-integer from 0 to 5.

According to the preparation method of the present invention, the molar ratio of the arylaminopurine derivative represented by Formula 1 to the acid is 1:1 to 1:4, preferably 1:1.2 to 1:3.5.

According to the preparation method of the present invention, the molar ratio of the arylaminopurine derivative represented by Formula 1 to water is not greater than 1:1 (i.e., 1:1 to 1:∞), preferably 1:4 to 1:200.

According to the preparation method of the present invention, the reaction temperature is 0-70°C, preferably 35-45°C.

According to the preparation method of the present invention, the reaction time is 0.5-10 hours, preferably 0.5-5 hours.

According to the preparation method of the present invention, the reaction is performed in the presence of the combination of water and one or more organic solvents selected from alcohols, ethers, esters, ketones, nitriles, and alkanes, preferably in the presence of $C_1$-$C_3$ lower alcohol and water, in the presence of a ketone and water, in the presence of nitrile and water, or the presence of ether and water, and more preferably in the presence of methanol-water, ethanol-water, isopropanol-water, tetrahydrofuran-water, dioxane-water, acetone-water or acetonitrile-water; and the ratio of the use amounts by volume of the organic solvent to water is 1:10 to 10:1, for example, 1:1 to 10:1 or 1:10 to 1:1, the organic solvent refers to the aforementioned other solvents except for water.

According to the preparation method of the present invention, after the reaction is finished, the temperature is reduced to 0-30°C, standing and crystallization are carried out for 0.5-24 hours, solids are separated, and dried to obtain the salt represented by Formula 2 of the arylaminopurine derivative. Preferably, the crystallization temperature is 5-15°C, and the crystallization time is 1-10 hours.

According to the preparation method of the present invention, the separation step includes separating the obtained salt represented by Formula 2 of the arylaminopurine derivative from the crystallization solution by using suitable processes such as filtration, e.g. suction filtration, and centrifugation.

According to the preparation method of the present invention, the drying process can adopt any suitable known process, preferably drying under reduced pressure (in a vacuum). The specific drying condition includes, for example, the temperature is preferably 35-70°C, more preferably 40-65°C; the pressure is preferably a vacuum degree>0.090 MPa; the drying time is preferably 5-50 hours, more preferably 5-10 hours. No matter what drying process is used, the residual solvent content in the obtained product should meet the quality standard.

In another aspect, the present invention provides a method for preparing a salt represented by Formula 2 of the arylaminopurine derivative, which comprises a reaction of an arylaminopurine derivative represented by Formula 1 and an acid is performed in the presence of water and an organic solvent to obtain the salt represented by Formula 2 of the arylaminopurine derivative:

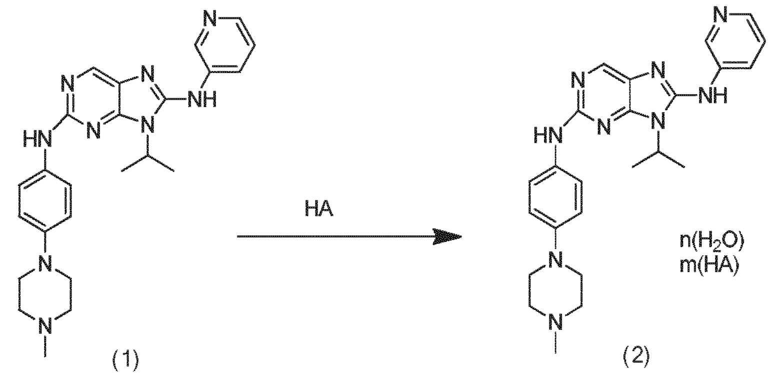


(1) (2) ,

wherein,

HA is an acid;
$H_2O$ is the water of crystallization;
m is an integer or half-integer from 1 to 4;
n is an integer or half-integer from 0 to 5; wherein:

the molar ratio of the arylaminopurine derivative represented by Formula 1 to the acid is 1:1 to 1:4, preferably 1:1.2 to 1:3.5;
the molar ratio of the arylaminopurine derivative represented by Formula 1 to water is not greater than 1:1, preferably 1:4 to 1:200;
the reaction temperature is 0-70°C, preferably 35-45°C;
the reaction time is 0.5-10 hours, preferably 0.5-5 hours;
the reaction is performed in the presence of the combination of water and one or more organic solvents selected from alcohols, ethers, esters, ketones, nitriles, and alkanes, preferably in the presence of $C_1$-$C_3$ lower alcohol and water, in the presence of a ketone and water, in the presence of nitrile and water, or the presence of ether and water, and more preferably in the presence of methanol-water, ethanol-water, isopropanol-water, tetrahydrofuran-water, dioxane-water, acetone-water or acetonitrile-water; and the ratio of the use amounts by volume of the organic solvent to water is 1:10 to 10:1, for example, 1:1 to 10:1 or 1:10 to 1:1, the organic solvent refers to the aforementioned other solvents except for water;
after the reaction is finished, the temperature is reduced to 0-30°C, preferably 5-15°C, the crystallization is carried out for 0.5-24 hours, preferably 1-10 hours, and solids are separated (for example by filtration, e.g. suction filtration, centrifugation and the like), and optionally dried (for example, the drying temperature is 35-70°C, preferably 40-65°C; the drying pressure is a vacuum degree>0.090 MPa; the drying time is 5-50 hours, preferably 5-10 hours) to obtain the salt represented by Formula 2 of the arylaminopurine derivative.

In a preferred method for preparing a salt represented by Formula 2 of the arylaminopurine derivative, the arylaminopurine derivative represented by Formula 1, purified water (the molar factor is 4-200) and a proper amount of an organic solvent (any of methanol, ethanol, isopropanol, tetrahydrofuran, dioxane, acetone or acetonitrile) are added into a reactor; the mixture is heated to 35-45°C under stirring; an acid (the molar factor is 1.2-3.5) is added into the reactor; after the acid is added, a proper amount of an organic solvent is added; while the temperature is kept at 35-45°C, the reaction is continued for 0.5-5 hours; then the reaction system is cooled to 5-15°C under stirring, crystallized for 1-10 hours, and filtered or centrifuged to obtain the salt represented by Formula 2 of the arylaminopurine derivative.
In one embodiment of the present invention, the salt of the arylaminopurine derivative is characterized in that the salt is a hydrochloride represented by Formula 3':

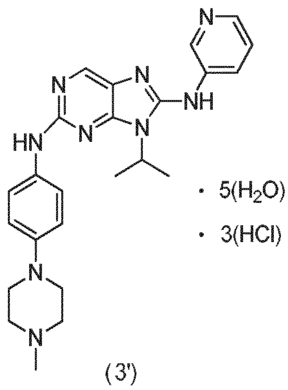

(3') ;

preferably, the hydrochloride represented by Formula 3' has an X-ray powder diffraction pattern comprising peaks at 2θ values of 7.3±0.2°, 8.5±0.2°, 9.0±0.2°, 11.8±0.2°, 12.6±0.2°, 14.3±0.2°, 18.1±0.2°, 19.6±0.2°, 20.0±0.2°, 21.1±0.2°, 21.9±0.2°, 23.7±0.2°, 25.2±0.2°, 26.1±0.2°, 27.2±0.2° or at 2θ values of 7.3±0.2°, 8.5±0.2°, 9.1±0.2°, 11.8±0.2°, 12.6±0.2°, 14.3±0.2°, 18.1±0.2°, 19.6±0.2°, 20.0±0.2°, 21.1±0.2°, 21.9±0.2°, 23.7±0.2°, 25.2±0.2°, 26.1±0.2°, 27.2±0.2° as measured with CuKα radiation;
the hydrochloride represented by Formula 3' is prepared in the following manner:

(1) an arylaminopurine derivative represented by Formula 1 is reacted with hydrochloric acid in the presence of water and an organic solvent,

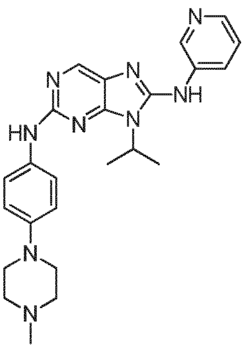

(1)

the molar ratio of the arylaminopurine derivative represented by Formula 1 to hydrochloric acid is 1:1 to 1:4, preferably 1:1.2 to 1:3.5;
preferably, the organic solvent is selected from acetone, isopropanol, tetrahydrofuran, and acetonitrile, and the volume ratio is 1:10 to 10:1, such as 1:1 to 10:1 or 1:10 to 1:1; the molar ratio of the arylaminopurine derivative represented by Formula 1 to water is not greater than 1:1 (that is, 1:1 to 1:∞), preferably 1:4 to 1:200;
the reaction temperature is 35-45°C;
the reaction time is 0.5-10 hours, preferably 0.5-5 hours;

(2) after the reaction is finished, the temperature is reduced to 5-15°C, the crystallization is carried out for 0.5-24 hours, and solids are separated (by filtration such as suction filtration, centrifugation, and the like), washed, and dried to obtain the hydrochloride represented by Formula 3'.

The arylaminopurine derivative represented by Formula 1 can be prepared with reference to the methods disclosed in the prior art, e.g. the methods described in the patent document WO2011/147066, the contents of which are incorporated herein by reference.

Beneficial Effect

The present invention provides the salts represented by Formula 2 of the arylaminopurine derivative, especially hydrochlorides, mesylates, L-malates, L-tartrates, oxalates, succinates, acetates, and sulfates. These salts can be

prepared into crystal forms, and their solubility is significantly improved in comparison to that of the arylaminopurine derivative represented by Formula 1. The preferred salts and crystal forms have low hygroscopicity and can exist stably, and therefore are easier to be formulated into drugs than the arylaminopurine derivative represented by Formula 1 or other salts.

**Brief description of the drawings**

Figure 1 is the XRPD pattern of the hydrochloride of the arylaminopurine derivative obtained in Example 1.
Figure 2 is the micrograph of a single crystal of the hydrochloride of the arylaminopurine derivative obtained in Example 2.
Figure 3 is the XRPD pattern of the single crystal hydrochloride of the arylaminopurine derivative obtained in Example 2.
Figure 4 is the XRPD pattern of the mesylate of the arylaminopurine derivative obtained in Example 3.
Figure 5 is the XRPD pattern of the mesylate of the arylaminopurine derivative obtained in Example 3.
Figure 6 is the XRPD pattern of the mesylate of the arylaminopurine derivative obtained in Example 5.
Figure 7 is the XRPD pattern of the L-malate of the arylaminopurine derivative obtained in Example 6.
Figure 8 is the XRPD pattern of the L-tartrate of the arylaminopurine derivative obtained in Example 7.
Figure 9 is the XRPD pattern of the L-tartrate of the arylaminopurine derivative obtained in Example 8.
Figure 10 is the XRPD pattern of the L-tartrate of the arylaminopurine derivative obtained in Example 9.
Figure 11 is the XRPD pattern of the oxalate of the arylaminopurine derivative obtained in Example 10.
Figure 12 is the XRPD pattern of the oxalate of the arylaminopurine derivative obtained in Example 11.
Figure 13 is the XRPD pattern of the succinate of the arylaminopurine derivative obtained in Example 12.
Figure 14 is the XRPD pattern of the succinate of the arylaminopurine derivative obtained in Example 13.
Figure 15 is the XRPD pattern of the acetate of the arylaminopurine derivative obtained in Example 14.
Figure 16 is the XRPD pattern of the acetate of the arylaminopurine derivative obtained in Example 15.
Figure 17 is the XRPD pattern of the sulfate of the arylaminopurine derivative obtained in Example 16.
Figure 18 is the XRPD pattern of the sulfate of the arylaminopurine derivative obtained in Example 17.
Figure 19 is the differential scanning calorimetry-thermogravimetric analysis (DSC-TGA) diagram of the hydrochloride of the arylaminopurine derivative obtained in Example 1.
Figure 20 is the differential scanning calorimetry (DSC) diagram of the mesylate of the arylaminopurine derivative obtained in Example 3.
Figure 21 is the thermogravimetric analysis (TGA) diagram of the mesylate of the arylaminopurine derivative obtained in Example 3.
Figure 22 is the differential scanning calorimetry (DSC) diagram of the mesylate of the arylaminopurine derivative obtained in Example 4.
Figure 23 is the thermogravimetric analysis (TGA) diagram of the mesylate of the arylaminopurine derivative obtained in Example 4.
Figure 24 is the differential scanning calorimetry (DSC) diagram of the mesylate of the arylaminopurine derivative obtained in Example 5.
Figure 25 is the thermogravimetric analysis (TGA) diagram of the mesylate of the arylaminopurine derivative obtained in Example 5.
Figure 26 is the differential scanning calorimetry-thermogravimetric analysis (DSC-TGA) diagram of the L-malate of the arylaminopurine derivative obtained in Example 6.
Figure 27 is the differential scanning calorimetry-thermogravimetric analysis (DSC-TGA) diagram of the L-tartrate of the arylaminopurine derivative obtained in Example 7.
Figure 28 is the differential scanning calorimetry-thermogravimetric analysis (DSC-TGA) diagram of the L-tartrate of the arylaminopurine derivative obtained in Example 8.
Figure 29 is the differential scanning calorimetry-thermogravimetric analysis (DSC-TGA) diagram of the L-tartrate of the arylaminopurine derivative obtained in Example 9.
Figure 30 is the differential scanning calorimetry-thermogravimetric analysis (DSC-TGA) diagram of the oxalate of the arylaminopurine derivative obtained in Example 10.
Figure 31 is the differential scanning calorimetry-thermogravimetric analysis (DSC-TGA) diagram of the oxalate of the arylaminopurine derivative obtained in Example 11.
Figure 32 is the differential scanning calorimetry (DSC) diagram of the succinate of the arylaminopurine derivative obtained in Example 12.
Figure 33 is the thermogravimetric analysis (TGA) diagram of the succinate of the arylaminopurine derivative obtained

in Example 12.

Figure 34 is the differential scanning calorimetry (DSC) diagram of the succinate of the arylaminopurine derivative obtained in Example 13.

Figure 35 is the thermogravimetric analysis (TGA) diagram of the succinate of the arylaminopurine derivative obtained in Example 13.

Figure 36 is the differential scanning calorimetry (DSC) diagram of the acetate of the arylaminopurine derivative obtained in Example 14.

Figure 37 is the thermogravimetric analysis (TGA) diagram of the acetate of the arylaminopurine derivative obtained in Example 14.

Figure 38 is the differential scanning calorimetry (DSC) diagram of the acetate of the arylaminopurine derivative obtained in Example 15.

Figure 39 is the thermogravimetric analysis (TGA) diagram of the acetate of the arylaminopurine derivative obtained in Example 15.

Figure 40 is the differential scanning calorimetry-thermogravimetric analysis (DSC-TGA) diagram of the sulfate of the arylaminopurine derivative obtained in Example 16.

Figure 41 is the differential scanning calorimetry-thermogravimetric analysis (DSC-TGA) diagram of the sulfate of the arylaminopurine derivative obtained in Example 17.

Figure 42 is the differential scanning calorimetry-thermogravimetric analysis (DSC-TGA) diagram of the arylaminopurine derivative obtained in Preparation Example 1.

## Detailed description

The technical solutions of the present invention will be further described in detail with reference to specific examples. The following examples are merely illustrative and explanatory of the present invention and should not be construed as limiting the scope of the present invention. All the techniques realized based on the above-mentioned contents of the present invention are covered in the protection scope of the present invention.

Unless otherwise specified, the raw materials and reagents used in the following examples are all commercially available products or can be prepared by known processes.

In the following examples, the analysis and detection conditions are as follows:

1. Moisture

Detection instrument: Karl Fischer moisture titrator/915 KF Ti-Touch

Test method: After the instrument was balanced, a proper amount (about 200 mg) of the test sample was taken, precisely weighed, and added to a titration cup, absolute methanol was used as the solvent, and a moisture titration solution was used for the direct measurement, and an average value was obtained by measuring each test sample twice.

2. Solubility

Detection instrument: Ultraviolet spectrophotometer/Evolution 300

Test method:

The following solutions with pH=1.2, pH=4.5, and pH=6.8 and water were used as the solvent, and the solvent preparation process was as follows:

(1) pH=1.2 hydrochloric acid solution: To 7.65 mL of hydrochloric acid was added 1000 mL of water, and the mixture was shaken uniformly to obtain the target solution.

(2) pH=4.5 phosphate buffer solution: 6.8g of potassium dihydrogen phosphate was taken and diluted with water to 1000 mL, and the mixture was shaken uniformly to obtain the target solution.

(2) pH=6.8 phosphate buffer solution: 6.8g of potassium dihydrogen phosphate and 0.896g of sodium hydroxide were taken and diluted with water to 1000 mL, and the mixture was shaken uniformly to obtain the target solution.

(4) Water: purified water

Sample preparation:

Test tubes with stopper were taken, and 10 mL of dissolution media at various pH values were precisely added to the test tubes respectively, and excessive stock drugs were added until supersaturated solutions were formed. The

adding amounts were recorded. The solutions were shaken uniformly, sealed with stoppers, and shaken for 24 hours in a shaker. 2 mL of solutions were taken out at different time points respectively, and centrifuged. The resulting supernatants were taken, filtered, and the subsequent filtrates were taken for later use.

The above-mentioned saturated solutions in different solvents were taken, and solvents were added to dilute the solutions to certain volumes. The absorbances were measured at a wavelength of 287 nm.

Preparation of a solution of the reference substance: an appropriate amount of the compound represented by Formula 1 was taken as the reference substance, and precisely weighed. A solvent was added to dissolve and dilute the reference compound to produce a solution containing about 10 μg of the compound represented by Formula 1 per 1 mL. The absorbance was measured at a wavelength of 287nm to calculate the solubility.

3. Hygroscopicity

Detection instrument: XPE105DR

Test method:

(1) A dry stoppered glass weighing bottle was taken, placed in a suitable constant-temperature desiccator at 25°C ± 1°C (with a saturated solution of ammonium chloride or ammonium sulfate in the lower part) or an artificial climate box (with the set temperature of 25°C±1°C and the relative humidity of 80%±2%) on the day before the test, and precisely weighed and recorded as the weight (m1).
(2) An appropriate amount of the test sample was taken and spread in the above-mentioned weighing bottle. The thickness of the test sample was generally about 1 mm, and the bottle was precisely weighed and recorded as the weight (m2).
(3) The stopper was removed to open the weighing bottle, and the opened weighing bottle and the stopper were placed under the above-mentioned constant temperature and humidity conditions for 24 hours.
(4) The opened weighing bottle was stoppered, precisely weighed, and recorded as the weight (m3).

$$\text{Percentage increase in mass} = (m3 - m2)/(m2 - m1) \times 100\%$$

(5) The description of hygroscopicity characteristics and the definition for the weight gain due to hygroscopicity:

Deliquescence: Sufficient water was absorbed to form a liquid.
Very hygroscopic: the weight gain due to hygroscopicity was not less than 15%.
Hygroscopic: the weight gain due to hygroscopicity was less than 15% but not less than 2%.
Slightly hygroscopic: the weight gain due to hygroscopicity was less than 2% but not less than 0.2%.
Not or nearly not hygroscopic: the weight gain due to hygroscopicity was less than 0.2%.

4. Content

Detection Instrument: High performance liquid chromatograph/Waters e2695-2489

Analysis method:
Octadecyl silane bonded to silica gel was used as a filler (the applicable range of the pH value should be greater than 10.0), 20 mmol/L of disodium hydrogen phosphate solution (the pH value was adjusted to 10.0 with sodium hydroxide)-acetonitrile (65:35) was used as the mobile phase; the detection wavelength was 287 nm, and the column temperature was 30°C. The number of theoretical plates should be not less than 3000.

Determination method: About 20 mg of the sample was taken and precisely weighed, put in a 100 mL volumetric flask. A diluent (50% methanol/water) was added to dissolve and dilute the sample to the scale. The content was shaken uniformly, and 10 μL was precisely metered and injected into a liquid chromatograph, and the chromatogram was recorded; another appropriate amount of the reference substance was taken, and the same method was used for determination. The result was obtained by calculating the peak area according to the external standard method.

5. X-Ray Powder Diffraction (XRPD)

(1) Examples 1 and 2

Detection instrument: PANalytical Empyrean type powder X-ray diffractometer

Test conditions:

Light tube type: Cu target, metal-ceramic X-ray tube;
X-ray wavelength: CuKα, $K\alpha_1$(Å): 1.540598, $K\alpha_2$(Å): 1.544426, $K\alpha_2/K\alpha_1$intensity ratio: 0.5;
Voltage and current: 45kV, 40mA;
Scanning range: 3-40°2θ;
Total scanning time: About 5 minutes.

(2) Examples 3-17

Detection instrument: BRUKER D2 PHASER powder X-ray diffractometer

Test conditions:

Light tube type: Cu target, ceramic X-ray tube;
X-ray wavelength: CuKα, $K\alpha_1$(Å): 1.540598, $K\alpha_2$(Å): 1.544426, $K\alpha_2/K\alpha_1$ intensity ratio: 0.5;
Voltage and current: 30kV, 10mA;
Scanning range: 4-40°2θ;
Total scanning time: 200.9S.

6. Differential scanning calorimetry-thermogravimetric analysis (DSC-TGA)

Detection instrument: NETZSCH STA 449F3

Test conditions:

Temperature range: 20°C-350°C;
Heating rate: 10.0 (K/min);
Sample holder/thermocouple: DSC/TG Cp S/S
Crucible: DSC/TG pan Al2O3
Atmosphere: N2, 20.0ml/min/N2, 50.0ml/min
Calibration/measurement range: 020/5000μV

7. Differential scanning calorimetry (DSC)

Detection instrument: NETZSCH DSC 214 Polyma

Test conditions:

Temperature range: 20°C-250°C;
Heating rate: 5.0(K/min);
Sample holder/thermocouple: DSC 214 Corona sensor/E
Crucible: Pan Al, pierced lid
Atmosphere: N2, 40.0ml/min/N2, 60.0ml/min
Calibration/measurement range: 000/5000μV

8. Thermogravimetric Analysis (TGA)

Detection instrument: METTLER and SDT Q600

Analysis method:

Temperature range: 20°C-250°C;
Heating rate: 5.0(K/min);

9. Nuclear Magnetic Resonance spectroscopy (NMRS)

Detection instrument: A VIII BRUKER 600 type superconducting nuclear magnetic resonance spectrometer
Contents and test solvent: $^1$H-NMR, the test solvent was $H_2D$.

10. Single-crystal

Single crystal diffraction data were collected using a Rigaka XtaLAB Synergy-R (Micro-Max007HF Cu mode, CuKα (λ=1.54184Å), Hypix 6000 HE detector) type single crystal diffractometer at a temperature of 120.00(10)K. The micrograph of the single crystal sample was taken by using a Shanghai CEWEI PXS9-T type stereoscopic microscope.

11. Acidity measurement

Detection instrument: Mettler Toledo S210-K pH meter

Test method: Based on the operation according to the pH value measurement method, 10 mg of a sample was precisely weighed, then 10 mL of freshly boiled and cooled purified water was added to dissolve the sample, and the mixture was shaken uniformly, and then the pH value was measured.

12. Measurement of related substances

Detection instrument: High performance liquid chromatograph/Waters e2695-2489

Chromatographic conditions:
Octadecylsilane bonded to silica gel was used as a filler (Model: Waters Xbridge C18 chromatographic column, with a length of 250 mm, an inner diameter of 4.6 mm, and a filler particle size of 5 μm), the detection wavelength was 250 nm, the column temperature was 35°C, and the flow rate was 1.0 mL/minute, the mobile phase A was 0.02 mol/L of a disodium hydrogen phosphate solution (the pH value was adjusted to 10.0 with a sodium hydroxide solution), the mobile phase B was acetonitrile, and the diluent was methanol, and the temperature of the sample plate was 4°C.
Test method: The system applicability test was performed according to the requirements, and the test sample solution, the control solution, and the sensitivity solution were prepared. Each 10 μL of the control solution and the test sample solution were precisely metered and injected into a liquid chromatograph, and the chromatogram was recorded. The result was obtained by calculating the peak area according to the self-dilution control method with the correction factor.

**Preparation Example 1: Preparation of the compound represented by Formula 1**

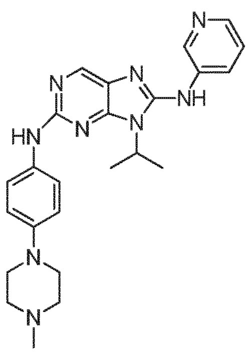

(1)

With reference to the method described in Example 90 of the patent document WO2011/147066, 100 g of the compound represented by Formula 1 was prepared.

**Example 1: Preparation of a hydrochloride of the arylaminopurine derivative**

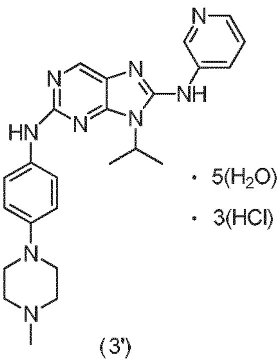

(3')

The arylaminopurine derivative (90g, 0.203mol) from Preparation Example 1, 800 mL of purified water, and 400 mL of acetone were added to the reactor. The mixture was heated to 40±5°C under stirring, and a stream of concentrated hydrochloric acid (74g, 0.731mol) was added to the reactor. After completing the addition of concentrated hydrochloric acid, 2L of acetone was added, and the reaction was continued for 1 hour while keeping the temperature at 40±5°C. Then the reaction mixture was cooled down to 10±5°C under stirring and crystallized for 2 hours. Suction filtration was performed. The filter cake was washed with 300 mL of acetone to produce a yellow or pale yellow hydrochloride (74.7g). $^1$H-NMR(600MHz, $D_2O$) δ: 1.556(d, 6H), δ: 2.896(s, 3H), δ: 3.058(t, 2H), δ: 3.187(t, 2H), δ: 3.586(d, 2H), δ: 3.749(d, 2H), δ: 4.701(s, 1H), δ: 7.062(d, 2H), δ: 7.377(d, 2H), δ: 7.968(t, 1H), δ: 8.086(s, 1H), δ: 8.431(d, 1H), δ: 8.636(d, 1H), δ: 9.171(s, 1H). The obtained hydrochloride exhibited good crystallinity, and its XRPD characterization pattern was shown in Figure 1. The main diffraction peak data were as follows:

| Peak position 2θ angle (°) | Relative peak intensity % |
|---|---|
| 7.300 | 20.96 |
| 8.504 | 40.4 |
| 9.052 | 14.65 |
| 11.814 | 34.65 |
| 12.579 | 13.44 |
| 14.300 | 15.86 |
| 18.136 | 18.09 |
| 19.641 | 29.87 |
| 20.027 | 26.40 |
| 21.140 | 22.06 |
| 21.913 | 14.4 |
| 23.701 | 25.54 |
| 25.162 | 62.26 |
| 26.137 | 15.54 |
| 27.165 | 100 |

It could be inferred from the calculation of the free base content by HPLC, the determination of the water content, and the hydrogen ratio in $^1$H-NMR of the nuclear magnetic resonances (see the table below) that the base/acid/$H_2O$ of the hydrochloride was 1:3:5.

| Name | Theoretical ratio (base/acid/ $H_2O$) | Theoretical water content (%) | Theoretical acid content (%) | Theoretical base content (%) | Measured water content (%) | Measured base content (%, HPLC) | Acid:base hydrogen atom number ratio ($^1$H-NMR) |
|---|---|---|---|---|---|---|---|
| Example 1 hydrochloride | 1:3:5 | 14.0% | 17.0% | 69.0% | 13.9% | 69.0% | N/A |

The process of Example 1 was repeated except for changing the amount of concentrated hydrochloric acid used in Example 1, and still, only the arylaminopurine derivative·trihydrochloride·pentahydrate obtained in Example 1 could be obtained. The process of Example 1 was repeated except for replacing acetone in Example 1 with isopropanol or tetrahydrofuran, and the arylaminopurine derivative·trihydrochloride·pentahydrate obtained in Example 1 could also be obtained.

**Example 2: Preparation of single crystal hydrochloride of the arylaminopurine derivative**

14.9 mg of the hydrochloride obtained in Example 1 was weighed and placed in a 3 mL glass bottle. 0.6 mL of acetonitrile/water (4:1, v/v) mixed solvent was added. The mixture was stirred to dissolve the hydrochloride and then placed in a 25 mL hydrothermal reaction vessel. The hydrothermal reaction vessel was sealed and placed in a temperature-controlled oven for the programmed temperature up and down. The temperature program was:

$$30°C \xrightarrow{120mins} 80°C \xrightarrow{300mins} 80°C \xrightarrow{5000mins} 30°C$$

After the completion of the experiment, it was found that a long and platy single crystal sample was precipitated in the system. A micrograph of the single crystal sample was shown in Figure 2. The single crystal X-ray diffraction characterization result showed that the crystal belonged to the triclinic system, space group $P\bar{1}$, and had the unit cell parameters: {a=7.04142(7)Å, b=12.15291(7)Å, c=18.13188(10)Å, α=93.2215(5)°, β=95.3039(6)°, γ=91.9554(6)°, V=1541.32(2)$Å^3$}. The asymmetric unit of the crystal consists of a cation of the compound represented by Formula 1, three chloride ions, and five water molecules. The single crystal was subjected to the XRPD measurement, and the obtained pattern was shown in Figure 3, and the main diffraction peak data were as follows:

| Peak position 2θ angle (°) | Relative peak intensity % |
|---|---|
| 7.292 | 26.96 |
| 8.507 | 66.86 |
| 9.041 | 12.81 |
| 11.815 | 51.74 |
| 12.558 | 11.89 |
| 14.281 | 16.05 |
| 18.109 | 14.25 |
| 19.633 | 41.79 |
| 20.033 | 30.29 |
| 21.125 | 11.68 |
| 21.919 | 22.19 |
| 23.727 | 28.37 |
| 25.166 | 42.06 |
| 26.131 | 15.26 |
| 27.177 | 100 |

It could be seen from the result of the comparison between Figure 1 and Figure 3 that the single crystal obtained in Example 2 was the same crystal form as that obtained in Example 1.

**Example 3: Preparation of a mesylate of the arylaminopurine derivative**

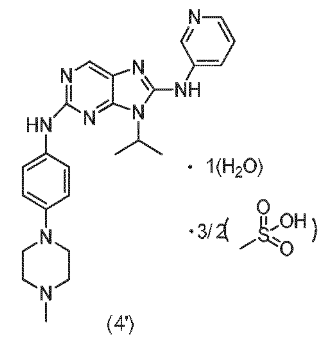

(4')

The arylaminopurine derivative (7g, 15.8mmol) from Preparation Example 1, 7 mL of purified water, and 28 mL of acetone were added to the reactor. The mixture was heated to 40±5°C under stirring, and methanesulfonic acid (1.82g, 18.9mmol) was added to the reactor. After completing the addition, 147 mL of acetone was added, and the reaction was continued for 1 hour while keeping the temperature at 40±5°C. Then the reaction mixture was cooled down to 10±5°C under stirring and crystallized for 2 hours. Suction filtration was performed. The filter cake was washed with 45 mL of acetone to produce a yellow or pale yellow mesylate (7.8g). $^1$H-NMR(600MHz, $D_2O$) δ: 1.500(d, 6H), δ: 2.783(s, 4H), δ: 2.888(m, 5H), δ: 3.085(m, 2H), δ: 3.511(m, 4H), δ: 4.489(m, 1H), δ: 6.817(d, 2H), δ: 7.200(d, 2H), δ: 7.404(m, 1H), δ: 7.906(m, 2H), δ: 8.122(d, 1H), δ: 8.567(s, 1H). The obtained mesylate exhibited good crystallinity, and its XRPD characterization pattern was shown in Figure 4. The main diffraction peak data were as follows:

| Peak position 2θ angle (°) | Relative peak intensity % |
|---|---|
| 6.803 | 36.6 |
| 8.599 | 20.2 |
| 10.679 | 16.0 |
| 12.633 | 19.7 |
| 13.112 | 36.1 |
| 13.434 | 26.1 |
| 15.136 | 47.0 |
| 16.271 | 55.4 |
| 17.734 | 34.4 |
| 19.009 | 40.7 |
| 19.913 | 41.3 |
| 20.967 | 100 |
| 25.008 | 55.5 |

It could be inferred from the calculation of the free base content by HPLC, the determination of the water content, and the hydrogen ratio in $^1$H-NMR of the nuclear magnetic resonances (see the table below) that the base/acid/$H_2O$ of the mesylate was 1:1.5:1.

| Name | Theoretical ratio (base/ acid/ $H_2O$) | Theoretical water content (%) | Theoretical acid content (%) | Theoretical base content (%) | Measured water content (%) | Measured base content (%, HPLC) | Acid:base hydrogen atom number ratio ($^1$H-NMR) |
|---|---|---|---|---|---|---|---|
| Example 3 mesylate | 1:1.5:1 | 3.0% | 23.8% | 73.2% | 4.1% | 77.5% | 1:1.5 |

**Example 4: Preparation of a mesylate of the arylaminopurine derivative**

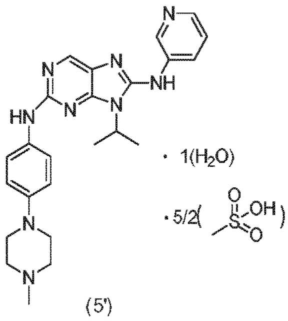

(5')

The preparation process of Example 3 was repeated except for changing the amount of methanesulfonic acid to (5.2g, 54.1mmol), and a yellow or pale yellow mesylate (8.8 g) was obtained. $^1$H-NMR(600MHz, $D_2O$) δ: 1.621(d, 6H), δ: 2.770(s, 8H), δ: 2.962(s, 3H), δ: 3.120(m, 2H), δ: 3.242(m, 2H), δ: 3.643(d, 2H), δ: 3.808(d, 2H), δ: 4.747(m, 1H), δ: 7.139(d, 2H), δ: 7.450(d, 2H), δ: 7.972(m, 1H), δ: 8.125(s, 1H), δ: 8.457(d, 1H), δ: 8.608(m, 1H), δ: 9.158(d, 1H). The obtained mesylate exhibited good crystallinity, and its XRPD characterization pattern was shown in Figure 5. The main diffraction peak data were as follows:

| Peak position 2θ angle (°) | Relative peak intensity % |
|---|---|
| 6.058 | 100 |
| 6.394 | 83.4 |
| 11.664 | 21.5 |
| 12.380 | 15.1 |
| 16.027 | 21.7 |
| 16.569 | 33. 2 |
| 16.915 | 29.1 |
| 17.450 | 55.4 |
| 18.033 | 33.2 |
| 18.911 | 55.7 |
| 19.271 | 58.6 |
| 19.896 | 26.4 |
| 20.219 | 29.9 |
| 23.368 | 26.5 |
| 24.382 | 47.7 |
| 26.375 | 38.7 |
| 27.339 | 26.2 |

It could be inferred from the calculation of the free base content by HPLC, the determination of the water content, and the hydrogen ratio in $^1$H-NMR of the nuclear magnetic resonances (see the table below) that the base/acid/$H_2O$ of the mesylate was 1:2.5:1.

| Name | Theoretical ratio (base/ acid/ $H_2O$) | Theoretical water content (%) | Theoretical acid content (%) | Theoretical base content (%) | Measured water content (%) | Measured base content (%, HPLC) | Acid:base hydrogen atom number ratio ($^1$H-NMR) |
|---|---|---|---|---|---|---|---|
| Example 4 mesylate | 1:2.5:1 | 2.6% | 34.2% | 63.2% | 3.2% | 64.1% | 1:2.5 |

**Example 5: Preparation of a mesylate of the arylaminopurine derivative**

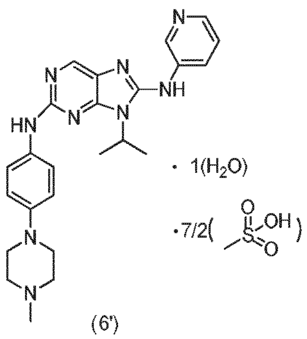

(6')

The preparation process of Example 3 was repeated except for changing the amount of methanesulfonic acid to (7.58g, 78.9mmol), and a yellow or pale yellow mesylate (11.2 g) was obtained. $^1$H-NMR(600MHz, $D_2O$) δ: 1.602(d, 6H), δ: 2.736(s, 11H), δ: 2.951(s, 3H), δ: 3.177(m, 2H), δ: 3.263(m, 2H), δ: 3.655(d, 2H), δ: 3.837(d, 2H), δ: 4.745(m, 1H), δ: 7.188(d, 2H), δ: 7.473(d, 2H), δ: 8.015(m, 1H), δ: 8.131(s, 1H), δ: 8.486(d, 1H), δ: 8.678(m, 1H), δ: 9.210(d, 1H). The obtained mesylate exhibited good crystallinity, and its XRPD characterization pattern was shown in Figure 6. The main diffraction peak data were as follows:

| Peak position 2θ angle (°) | Relative peak intensity % |
|---|---|
| 4.887 | 55.7 |
| 6.038 | 15.3 |
| 9.733 | 12.7 |
| 10.514 | 14.1 |
| 11.471 | 70.2 |
| 12.306 | 15.4 |
| 14.492 | 76.5 |
| 15.055 | 35.4 |
| 16.808 | 33.2 |
| 18.487 | 47.2 |
| 18.871 | 100 |
| 21.557 | 27.5 |
| 22.023 | 19.5 |
| 22.316 | 17.2 |
| 22.767 | 39.4 |

(continued)

| Peak position 2θ angle (°) | Relative peak intensity % |
|---|---|
| 23.372 | 22.8 |
| 24.260 | 43.2 |
| 25.353 | 39.4 |
| 26.675 | 27.2 |
| 27.264 | 20.9 |

It could be inferred from the calculation of the free base content by HPLC, the determination of the water content, and the hydrogen ratio in $^1$H-NMR of the nuclear magnetic resonances (see the table below) that the base/acid/$H_2O$ of the mesylate was 1:3.5:1.

| Name | Theoretical ratio (base/ acid/ $H_2O$) | Theoretical water content (%) | Theoretical acid content (%) | Theoretical base content (%) | Measured water content (%) | Measured base content (%, HPLC) | Acid: base hydrogen atom number ratio ($^1$H-NMR) |
|---|---|---|---|---|---|---|---|
| Example 5 mesylate | 1:3.5:1 | 2.3% | 42.2% | 55.6% | 3.0% | 57.1% | 1:3.5 |

**Example 6: Preparation of an L-malate of the arylaminopurine derivative**

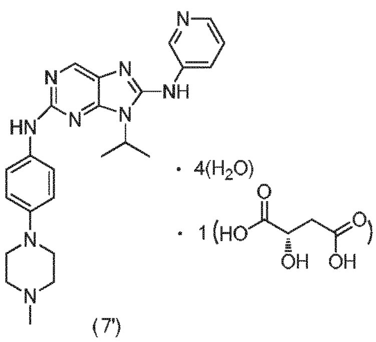

(7')

The arylaminopurine derivative (8g, 18mmol) from Preparation Example 1, 64 mL of purified water, and 32 mL of acetone were added to the reactor. The mixture was heated to 40±5°C under stirring, and L-malic acid (2.902g, 21.6mmol) was added to the reactor. After completing the addition, 168 mL of acetone was added, and the reaction was continued for 1 hour while keeping the temperature at 40±5°C. Then the reaction mixture was cooled down to 10±5°C under stirring and crystallized for 2 hours. Suction filtration was performed. The filter cake was washed with 30 mL of acetone to produce a yellow L-malate (8.63g). $^1$H-NMR(600MHz, $D_2O$) δ: 1.590(d, 6H), δ: 2.527(q, 1H), δ: 2.749(q, 1H), δ: 2.929(s, 3H), δ: 3.010(t, 2H), δ: 3.184(t, 2H), δ: 3.587(d, 4H), δ: 4.328(d, 1H), δ: 4.606(d, 1H), δ: 6.983(d, 2H), δ: 7.370(d, 2H), δ: 7.538(q, 1H), δ: 8.052(d, 2H), δ: 8.254(d, 1H), δ: 8.690(d, 1H). The obtained malate exhibited good crystallinity, and its XRPD characterization pattern was shown in Figure 7. The main diffraction peak data were as follows:

| Peak position 2θ angle (°) | Relative peak intensity % |
|---|---|
| 7.039 | 100 |
| 9.340 | 83.6 |
| 11.953 | 14.5 |

(continued)

| Peak position 2θ angle (°) | Relative peak intensity % |
|---|---|
| 12.945 | 19.3 |
| 13.976 | 11.3 |
| 16.648 | 15.0 |
| 17.646 | 31.3 |
| 18.518 | 22.7 |
| 19.651 | 30.9 |
| 22.995 | 10.7 |
| 24.198 | 13.9 |
| 25.190 | 20.7 |
| 25.937 | 73.4 |
| 27.535 | 22.5 |

It could be inferred from the calculation of the free base content by HPLC, the determination of the water content, and the hydrogen ratio in $^1$H-NMR of the nuclear magnetic resonances (see the table below) that the base/acid/$H_2O$ of the malate was 1:1:4.

| Name | Theoretical ratio (base/ acid/ $H_2O$) | Theoretical water content (%) | Theoretical acid content (%) | Theoretical base content (%) | Measured water content (%) | Measured base content (%, HPLC) | Acid:base hydrogen atom number ratio ($^1$H-NMR) |
|---|---|---|---|---|---|---|---|
| Example 6 L-malate | 1:1:4 | 11.1% | 20.6% | 68.3% | 11.0% | 69.1% | 1:1 |

**Example 7: Preparation of an L-tartrate of the arylaminopurine derivative**

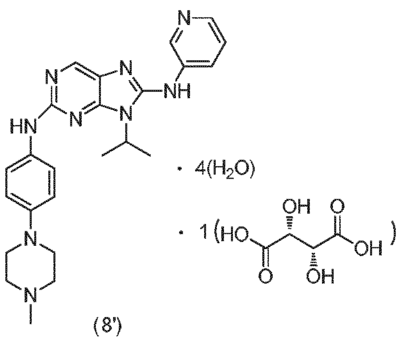

(8')

The arylaminopurine derivative (8g, 18mmol) from Preparation Example 1, 64 mL of purified water, and 32 mL of acetone were added to the reactor. The mixture was heated to 40±5°C under stirring, and L-tartaric acid (3.248g, 21.6mmol) was added to the reactor. After completing the addition, 168 mL of acetone was added, and the reaction was continued for 1 hour while keeping the temperature at 40±5°C. Then the reaction mixture was cooled down to 10±5°C under stirring and crystallized for 2 hours. Suction filtration was performed. The filter cake was washed with 30 mL of acetone to produce a yellow L-tartrate (10.06g). $^1$H-NMR(600MHz, $D_2O$) δ: 1.626(d, 6H), δ: 2.954(s, 3H), δ: 3.086(t, 2H), δ: 3.242(t, 2H), δ: 3.629(d, 2H), δ: 3.747(d, 2H), δ: 4.414(s, 2H), δ: 4.683(m, 1H), δ: 7.098(d, 2H), δ: 7.453(d, 2H),

δ: 7.705(m, 1H), δ: 8.109(s, 1H), δ: 8.255(d, 1H), δ: 8.349(d, 1H), δ: 8.857(s, 1H). The obtained tartrate exhibited good crystallinity, and its XRPD characterization pattern was shown in Figure 8. The main diffraction peak data were as follows:

| Peak position 2θ angle (°) | Relative peak intensity % |
|---|---|
| 6.895 | 61.5 |
| 9.058 | 100 |
| 12.884 | 27.6 |
| 13.810 | 25.9 |
| 16.470 | 29.0 |
| 17.773 | 40.2 |
| 19.419 | 42.8 |
| 20.087 | 21.6 |
| 25.503 | 95.3 |
| 26.920 | 26.6 |

It could be inferred from the calculation of the free base content by HPLC, the determination of the water content, and the hydrogen ratio in $^1H$-NMR of the nuclear magnetic resonances (see the table below) that the base/acid/$H_2O$ of the tartrate was 1:1:4.

| Name | Theoretical ratio (base/ acid/ $H_2O$) | Theoretical water content (%) | Theoretical acid content (%) | Theoretical base content (%) | Measured water content (%) | Measured base content (%, HPLC) | Acid:base hydrogen atom number ratio ($^1H$-NMR) |
|---|---|---|---|---|---|---|---|
| Example 7 L-tartrate | 1:1:4 | 10.8% | 22.6% | 66.7% | 11.0% | 68.0% | 1:1 |

**Example 8: Preparation of an L-tartrate of the arylaminopurine derivative**

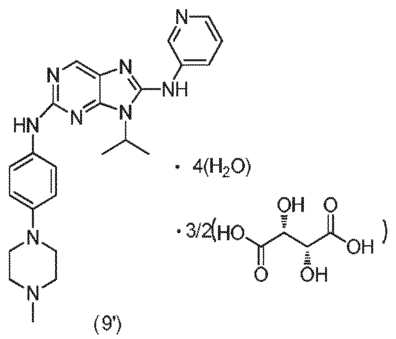

(9')

The arylaminopurine derivative (7g, 15.8mmol) from Preparation Example 1, 56 mL of purified water, and 28 mL of acetone were added to the reactor. The mixture was heated to 40±5°C under stirring, and L-tartaric acid (5.685g, 37.9mmol) was added to the reactor. After completing the addition, 147 mL of acetone was added, and the reaction was continued for 1 hour while keeping the temperature at 40±5°C. Then the reaction mixture was cooled down to 10±5°C under stirring and crystallized for 2 hours. Suction filtration was performed. The filter cake was washed with 30 mL of acetone to produce a pale yellow L-tartrate (11.3g). $^1H$-NMR(600MHz, $D_2O$) δ: 1.610(d, 6H), δ: 2.948(s, 3H), δ: 3.067(s, 2H), δ: 3.229(s, 2H), δ: 3.630(s, 2H), δ: 3.755(s, 2H), δ: 4.469(s, 3H), δ: 4.697(m, 1H), δ: 7.085(d, 2H), δ: 7.431(d, 2H),

δ: 7.798(m, 1H), δ: 8.094(s, 1H), δ: 8.366(m, 2H), δ: 8.978(s, 1H). The obtained tartrate exhibited good crystallinity, and its XRPD characterization pattern was shown in Figure 9. The main diffraction peak data were as follows:

| Peak position 2θ angle (°) | Relative peak intensity % |
|---|---|
| 8.531 | 64.5 |
| 9.754 | 30.4 |
| 10.144 | 28.7 |
| 11.267 | 21.1 |
| 13.722 | 18.5 |
| 14.831 | 46.5 |
| 15.447 | 26.4 |
| 16.345 | 32.4 |
| 17.081 | 57.1 |
| 17.648 | 23.4 |
| 18.837 | 87.3 |
| 20.461 | 33.8 |
| 22.316 | 33.4 |
| 24.578 | 80.1 |
| 26.075 | 100 |

It could be inferred from the calculation of the free base content by HPLC, the determination of the water content, and the hydrogen ratio in $^1$H-NMR of the nuclear magnetic resonances (see the table below) that the base/acid/$H_2O$ of the tartrate was 1:1.5:4.

| Name | Theoretical ratio (base/ acid/ $H_2O$) | Theoretical water content (%) | Theoretical acid content (%) | Theoretical base content (%) | Measured water content (%) | Measured base content (%, HPLC) | Acid:base hydrogen atom number ratio ($^1$H-NMR) |
|---|---|---|---|---|---|---|---|
| Example 8 L-tartrate | 1:1.5:4 | 9.7% | 30.4% | 59.9% | 14.4% | 61.0% | 1:1.5 |

**Example 9: Preparation of an L-tartrate of the arylaminopurine derivative**

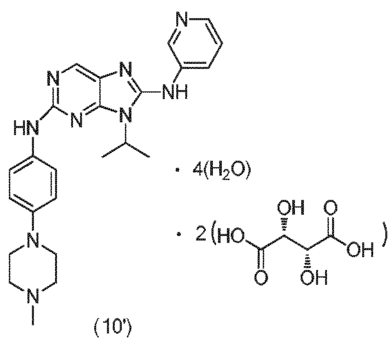

(10')

The preparation process of Example 8 was repeated except for changing the amount of L-tartaric acid to (8.29g, 55.2mmol), and a pale yellow L-tartrate (11.59g) was obtained. $^1$H-NMR(600MHz, $D_2O$) δ: 1.646(d, 6H), δ: 2.983(s, 3H), δ: 3.100(s, 2H), δ: 3.276(s, 2H), δ: 3.674(s, 2H), δ: 3.817(s, 2H), δ: 4.528(s, 4H), δ: 7.148(s, 2H), δ: 7.489(s, 2H), δ: 7.896(m, 1H), δ: 8.148(s, 1H), δ: 8.440(d, 1H), δ: 8.502(d, 1H), δ: 9.072(s, 1H). The obtained tartrate exhibited good crystallinity, and its XRPD characterization pattern was shown in Figure 10. The main diffraction peak data were as follows:

| Peak position 2θ angle (°) | Relative peak intensity % |
|---|---|
| 7.011 | 16.1 |
| 8.253 | 54.4 |
| 8.912 | 63.3 |
| 9.531 | 100 |
| 12.455 | 33.3 |
| 13.132 | 26.4 |
| 14.794 | 56.0 |
| 16.034 | 23.0 |
| 17.670 | 71.7 |
| 18.129 | 21.9 |
| 19.184 | 32.4 |
| 21.005 | 67.6 |
| 23.571 | 39.5 |
| 24.023 | 55.1 |
| 25.251 | 42.6 |
| 26.727 | 35.9 |

It could be inferred from the calculation of the free base content by HPLC, the determination of the water content, and the hydrogen ratio in $^1$H-NMR of the nuclear magnetic resonances (see the table below) that the base/acid/$H_2O$ of the tartrate was 1:2:4.

| Name | Theoretical ratio (base/ acid/ $H_2O$) | Theoretical water content (%) | Theoretical acid content (%) | Theoretical base content (%) | Measured water content (%) | Measured base content (%, HPLC) | Acid:base hydrogen atom number ratio ($^1$H-NMR) |
|---|---|---|---|---|---|---|---|
| Example 9 L-tartrate | 1:2:4 | 8.8% | 36.8% | 54.4% | 8.3% | 55.8% | 1:2 |

**Example 10: Preparation of an oxalate of the arylaminopurine derivative**

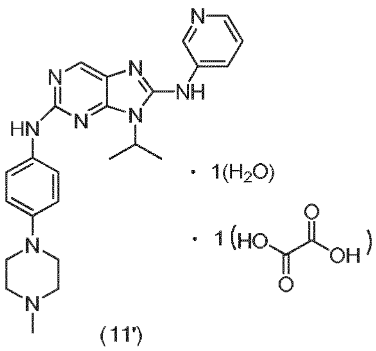

(11')

The arylaminopurine derivative (7g, 15.8mmol) from Preparation Example 1, 28 mL of purified water, and 28 mL of acetone were added to the reactor. The mixture was heated to 40±5°C under stirring, and oxalic acid dihydrate (2.388g, 18.9mmol) was added to the reactor. After completing the addition, 147 mL of acetone was added, and the reaction was continued for 1 hour while keeping the temperature at 40±5°C. Then the reaction mixture was cooled down to 10±5°C under stirring and crystallized for 2 hours. Suction filtration was performed. The filter cake was washed with 30 mL of acetone to produce a yellow oxalate (8.01g). $^1$H-NMR(600MHz, $D_2O$) δ: 1.637(d, 6H), δ: 2.974(s, 3H), δ: 3.158(m, 2H), δ: 3.276(m, 2H), δ: 3.663(d, 2H), δ: 3.848(d, 2H), δ: 4.790(m, 1H), δ: 7.192(d, 2H), δ: 7.505(d, 2H), δ: 7.972(m, 1H), δ: 8.139(s, 1H), δ: 8.491(d, 1H), δ: 8.601(d, 1H), δ: 9.127(s, 1H). The obtained oxalate exhibited good crystallinity, and its XRPD characterization pattern was shown in Figure 11. The main diffraction peak data were as follows:

| **Peak position 2θ angle (°)** | **Relative peak intensity %** |
|---|---|
| 8.063 | 59.5 |
| 8.353 | 91.6 |
| 8.983 | 29.0 |
| 14.103 | 35. 0 |
| 14.799 | 28.1 |
| 16.712 | 28.2 |
| 17.884 | 19.3 |
| 18.510 | 14.4 |
| 19.560 | 19.6 |
| 23.634 | 14.4 |
| 25.622 | 100 |

It could be inferred from the calculation of the free base content by HPLC, the determination of the water content, and the hydrogen ratio in $^1$H-NMR of the nuclear magnetic resonances (see the table below) that the base/acid/$H_2O$ of the oxalate was 1:1:1.

| Name | Theoretical ratio (base/acid/ $H_2O$) | Theoretical water content (%) | Theoretical acid content (%) | Theoretical base content (%) | Measured water content (%) | Measured base content (%, HPLC) | Acid:base hydrogen atom number ratio ($^1$H-NMR) |
|---|---|---|---|---|---|---|---|
| Example 10 oxalate | 1:1:1 | 3.3% | 16.3% | 80.4% | 5.1% | 80.9% | N/A |

**Example 11: Preparation of an oxalate of the arylaminopurine derivative**

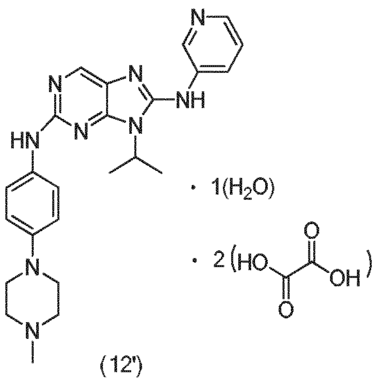

(12')

The preparation process of Example 10 was repeated except for changing the amount of oxalic acid dihydrate to (4.755g, 37.9mmol), and a yellow oxalate (8.01g) was obtained. $^1$H-NMR(600MHz, $D_2O$) δ: 1.621(d, 6H), δ: 2.963(s, 3H), δ: 3.126(m, 2H), δ: 3.257(m, 2H), δ: 3.650(d, 2H), δ: 3.833(d, 2H), δ: 4.757(m, 1H), δ: 7.167(d, 2H), δ: 7.474(d, 2H), δ: 8.013(m, 1H), δ: 8.127(s, 1H), δ: 8.496(d, 1H), δ: 8.665(d, 1H), δ: 9.191(s, 1H). The obtained oxalate exhibited good crystallinity, and its XRPD characterization pattern was shown in Figure 12. The main diffraction peak data were as follows:

| **Peak position 2θ angle (°)** | **Relative peak intensity %** |
|---|---|
| 7.108 | 100 |
| 8.272 | 14.7 |
| 12.195 | 49.8 |
| 14.202 | 28.6 |
| 16.442 | 35.7 |
| 17.690 | 31.3 |
| 18.599 | 25.6 |
| 19.047 | 36.5 |
| 24.385 | 56.4 |

It could be inferred from the calculation of the free base content by HPLC, the determination of the water content, and the hydrogen ratio in $^1$H-NMR of the nuclear magnetic resonances (see the table below) that the base/acid/$H_2O$ of the oxalate was 1:2:1.

| Name | Theoretical ratio (base/ acid/ $H_2O$) | Theoretical water content (%) | Theoretical acid content (%) | Theoretical base content (%) | Measured water content (%) | Measured base content (%, HPLC) | Acid:base hydrogen atom number ratio ($^1$H-NMR) |
|---|---|---|---|---|---|---|---|
| Example 11 oxalate | 1:2:1 | 2.8% | 28.1% | 69.2% | 2.9% | 70.1% | N/A |

**Example 12: Preparation of a succinate of the arylaminopurine derivative**

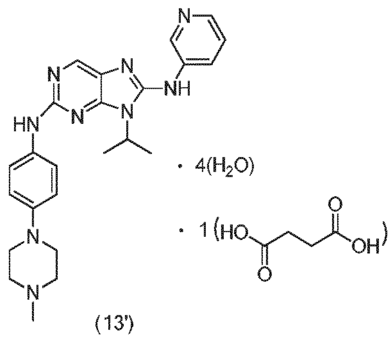

(13')

The arylaminopurine derivative (7g, 15.8mmol) from Preparation Example 1, 28 mL of purified water, and 28 mL of acetone were added to the reactor. The mixture was heated to 40±5°C under stirring, and succinic acid (2.236g, 18.9mmol) was added to the reactor. After completing the addition, 147 mL of acetone was added, and the reaction was continued for 1 hour while keeping the temperature at 40±5°C. Then the reaction mixture was cooled down to 10±5°C under stirring and crystallized for 2 hours. Suction filtration was performed. The filter cake was washed with 30 mL of acetone to produce a pale yellow succinate (7.51g). $^1$H-NMR(600MHz, $D_2O$) δ: 1.584(d, 6H), δ: 2.482(s, 4H), δ: 2.923(s, 3H), δ: 2.992(m, 2H), δ: 3.174(m, 2H), δ: 3.594(m, 4H), δ: 4.584(m, 1H), δ: 6.959(d, 2H), δ: 7.365(d, 2H), δ: 7.440(m, 1H), δ: 7.929(d, 1H), δ: 8.058(s, 1H), δ: 8.227(d, 1H), δ: 8.579(s, 1H). The obtained succinate exhibited good crystallinity, and its XRPD characterization pattern was shown in Figure 13. The main diffraction peak data were as follows:

| Peak position 2θ angle (°) | Relative peak intensity % |
|---|---|
| 7.024 | 80.2 |
| 9.128 | 55.0 |
| 11.323 | 46.7 |
| 13.065 | 23.9 |
| 13.849 | 36.0 |
| 14.399 | 46.2 |
| 15.969 | 18.2 |
| 16.769 | 40.5 |
| 17.744 | 33.6 |
| 18.476 | 52.3 |
| 20.351 | 53.8 |
| 21.017 | 48.7 |
| 22.437 | 100 |
| 24.204 | 35.9 |
| 25.889 | 46.9 |
| 27.115 | 50.9 |

It could be inferred from the calculation of the free base content by HPLC, the determination of the water content, and the hydrogen ratio in $^1$H-NMR of the nuclear magnetic resonances (see the table below) that the base/acid/$H_2O$ of the succinate was 1:1:4.

| Name | Theoretical ratio (base/ acid/ $H_2O$) | Theoretical water content (%) | Theoretical acid content (%) | Theoretical base content (%) | Measured water content (%) | Measured base content (%, HPLC) | Acid: base hydrogen atom number ratio ($^1$H-NMR) |
|---|---|---|---|---|---|---|---|
| Example 12 succinate | 1:1:4 | 11.4% | 18.6% | 70.0% | 12.6% | 69.3% | 1:1 |

**Example 13: Preparation of a succinate of the arylaminopurine derivative**

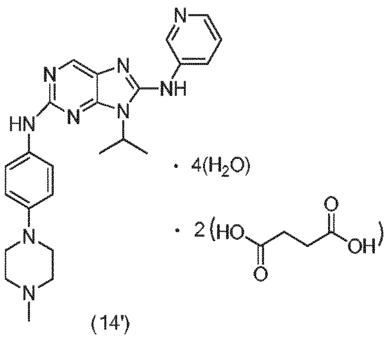

(14')

The preparation process of Example 12 was repeated except for changing the amount of succinic acid to (4.473g, 37.9mmol), and a pale yellow succinate (8.14g) was obtained. $^1$H-NMR(600MHz, $D_2O$) δ: 1.643(d, 6H), δ: 2.548(s, 9H), δ: 2.961(s, 3H), δ: 3.080(m, 2H), δ: 3.246(m, 2H), δ: 3.639(d, 2H), δ: 3.762(d, 2H), δ: 4.695(m, 1H), δ: 7.114(d, 2H), δ: 7.497(d, 2H), δ: 7.638(m, 1H), δ: 8.138(s, 1H), δ: 8.159(d, 1H), δ: 8.347(d, 1H), δ: 8.769(s, 1H). The obtained succinate exhibited good crystallinity, and its XRPD characterization pattern was shown in Figure 14. The main diffraction peak data were as follows:

| Peak position 2θ angle (°) | Relative peak intensity % |
|---|---|
| 6.965 | 100 |
| 9.193 | 47.5 |
| 11.919 | 15.0 |
| 16.657 | 19.6 |
| 17.626 | 32.2 |
| 18.410 | 30.6 |
| 19.661 | 31.0 |
| 20.273 | 10.7 |
| 22.953 | 11.9 |
| 24.149 | 11.9 |
| 25.171 | 19.1 |
| 25.816 | 58.1 |
| 27.263 | 28.5 |

It could be inferred from the calculation of the free base content by HPLC, the determination of the water content, and the hydrogen ratio in $^1$H-NMR of the nuclear magnetic resonances (see the table below) that the base/acid/$H_2O$ of the succinate was 1:2:4.

| Name | Theoretical ratio (base/ acid/ $H_2O$) | Theoretical water content (%) | Theoretical acid content (%) | Theoretical base content (%) | Measured water content (%) | Measured base content (%, HPLC) | Acid:base hydrogen atom number ratio ($^1$H-NMR) |
|---|---|---|---|---|---|---|---|
| Example 13 succinate | 1:2:4 | 9.6% | 31.4% | 59.0% | 12.7% | 58.9% | 1:2 |

**Example 14: Preparation of an acetate of the arylaminopurine derivative**

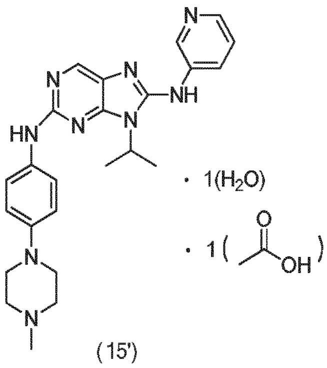

(15')

The arylaminopurine derivative (7g, 15.8mmol) from Preparation Example 1, 28 mL of purified water, and 28 mL of acetone were added to the reactor. The mixture was heated to 40±5°C under stirring, and acetic acid (1.13g, 18.9mmol) was added to the reactor. After completing the addition, 147 mL of acetone was added, and the reaction was continued for 1 hour while keeping the temperature at 40±5°C. Then the reaction mixture was cooled down to 10±5°C under stirring and crystallized for 2 hours. Suction filtration was performed. The filter cake was washed with 30 mL of acetone to produce an off-white acetate (7.51g). $^1$H-NMR(600MHz, DMSO) δ: 1.676(d, 6H), δ: 2.216(s, 3H), δ: 2.442(m, 2H), δ: 2.497(m, 2H), δ: 3.038(m, 4H), δ: 4.846(m, 1H), δ: 6.868(d, 2H), δ: 7.350(q, 1H), δ: 7.622(d, 2H), δ: 8.188(q, 1H), δ: 8.324(q, 1H), δ: 8.379(s, 1H), δ: 8.931(d, 1H), δ: 9.029(s, 1H), δ: 9.204(s, 1H). The obtained acetate exhibited good crystallinity, and its XRPD characterization pattern was shown in Figure 15. The main diffraction peak data were as follows:

| Peak position 2θ angle (°) | Relative peak intensity % |
|---|---|
| 6.319 | 18.9 |
| 8.867 | 21.5 |
| 10.861 | 62.7 |
| 11.498 | 19.6 |
| 12.164 | 32.7 |
| 12.622 | 83.6 |
| 15.148 | 66.2 |
| 17.754 | 91.8 |
| 19.221 | 81.2 |
| 19.645 | 75.1 |
| 20.988 | 55.0 |
| 21.767 | 57.8 |

(continued)

| Peak position 2θ angle (°) | Relative peak intensity % |
|---|---|
| 22.268 | 6 2.3 |
| 24.595 | 100 |
| 25.405 | 57.7 |

It could be inferred from the calculation of the free base content by HPLC, the determination of the water content, and the hydrogen ratio in $^1$H-NMR of the nuclear magnetic resonances (see the table below) that the base/acid/$H_2O$ of the acetate was 1:1:1.

| Name | Theoretical ratio (base/ acid/ $H_2O$) | Theoretical water content (%) | Theoreti cal acid content (%) | Theoretical base content (%) | Measured water content (%) | Measured base content (%, HPLC) | Acid:base hydrogen atom number ratio ($^1$H-NMR) |
|---|---|---|---|---|---|---|---|
| Example 14 acetate | 1:1:1 | 3.5% | 11.5% | 85.0% | 3.7% | 86.2% | 1:1 |

**Example 15: Preparation of an acetate of the arylaminopurine derivative**

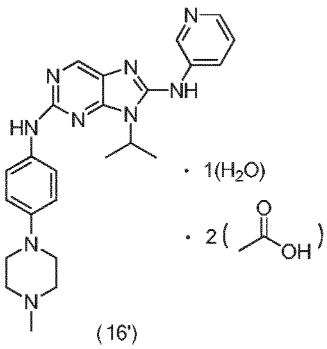

(16')

The arylaminopurine derivative (10g, 22.5mmol) from Preparation Example 1, 10 mL of purified water, and 40 mL of acetone were added to the reactor. The mixture was heated to 40±5°C under stirring, and acetic acid (4.74g, 78.9mmol) was added to the reactor. After completing the addition, 210 mL of acetone was added, and the reaction was continued for 1 hour while keeping the temperature at 40±5°C. Then the reaction mixture was cooled down to 10±5°C under stirring and crystallized for 2 hours. Suction filtration was performed. The filter cake was washed with 40 mL of acetone to produce an off-white acetate (9.04g). $^1$H-NMR(600MHz, $D_2O$) δ: 1.542(d, 6H), δ: 1.951(s, 6H), δ: 2.902(s, 1H), δ: 2.934(m, 4H), δ: 3.126(m, 2H), δ: 3.553(m, 4H), δ: 4.541(m, 1H), δ: 6.885(m, 2H), δ: 7.284(m, 2H), δ: 7.417(m, 1H), δ: 7.899(m, 1H), δ: 7.997(s, 1H), δ: 8.181(s, 1H), δ: 8.552(s, 1H). The obtained acetate exhibited good crystallinity, and its XRPD characterization pattern was shown in Figure 16. The main diffraction peak data were as follows:

| Peak position 2θ angle (°) | Relative peak intensity % |
|---|---|
| 6.174 | 100 |
| 8.109 | 29.6 |
| 9.097 | 33.4 |
| 12.231 | 92.9 |

(continued)

| Peak position 2θ angle (°) | Relative peak intensity % |
|---|---|
| 15.024 | 16.9 |
| 16.074 | 29.9 |
| 17.496 | 63.6 |
| 18.193 | 31.4 |
| 20.676 | 35.7 |
| 21.453 | 38.7 |
| 23.399 | 41.6 |
| 24.766 | 48.4 |
| 28.820 | 21.1 |

It could be inferred from the calculation of the free base content by HPLC, the determination of the water content, and the hydrogen ratio in $^1$H-NMR of the nuclear magnetic resonances (see the table below) that the base/acid/$H_2O$ of the acetate was 1:2:1.

| Name | Theoretical ratio (base/ acid/ $H_2O$) | Theoretical water content (%) | Theoretical acid content (%) | Theoretical base content (%) | Measured water content (%) | Measured base content (%, HPLC) | Acid:base hydrogen atom number ratio ($^1$H-NMR) |
|---|---|---|---|---|---|---|---|
| Example 15 acetate | 1:2:1 | 3.1% | 20.7% | 76.3% | 3.5% | 77.1% | 1:2 |

**Example 16: Preparation of a sulfate of the arylaminopurine derivative**

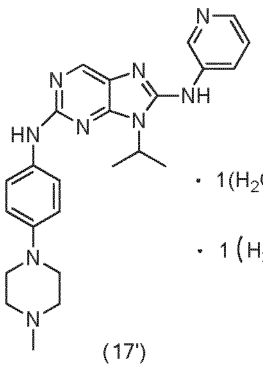

· 1($H_2O$)

· 1 ($H_2SO_4$)

(17')

The arylaminopurine derivative (7g, 15.8mmol) from Preparation Example 1, 7 mL of purified water, and 28 mL of acetone were added to the reactor. The mixture was heated to 40±5°C under stirring, and sulfuric acid (1.86g, 18.9mmol) was added to the reactor. After completing the addition, 147 mL of acetone was added, and the reaction was continued for 1 hour while keeping the temperature at 40±5°C. Then the reaction mixture was cooled down to 10±5°C under stirring and crystallized for 2 hours. Suction filtration was performed. The filter cake was washed with 30 mL of acetone to produce a pale yellow sulfate (7.5g). $^1$H-NMR(600MHz, $D_2O$) δ: 1.533(d, 6H), δ: 2.894(s, 3H), δ: 3.009(m, 2H), δ: 3.086(m, 2H), δ: 3.547(d, 2H), δ: 3.634(d, 2H), δ: 4.699(m, 1H), δ: 6.936(d, 2H), δ: 7.291(d, 2H), δ: 7.929(m, 1H), δ: 8.114(s, 1H), δ: 8.387(d, 1H), δ: 8.640(m, 1H), δ: 9.217(d, 1H). The obtained sulfate exhibited good crystallinity, and its XRPD characterization pattern was shown in Figure 17. The main diffraction peak data were as follows:

| Peak position 2θ angle (°) | Relative peak intensity % |
|---|---|
| 4.825 | 59.1 |
| 7.010 | 89.3 |
| 8.553 | 46.5 |
| 9.183 | 64.5 |
| 9.528 | 96.8 |
| 11.644 | 33.4 |
| 12.785 | 43.3 |
| 13.556 | 82.2 |
| 15.743 | 72.1 |
| 17.576 | 45.9 |
| 18.612 | 100 |
| 20.504 | 61.7 |
| 21.565 | 77.2 |
| 23.753 | 42.9 |
| 25.697 | 98.8 |

It could be inferred from the calculation of the free base content by HPLC, the determination of the water content, and the hydrogen ratio in $^1$H-NMR of the nuclear magnetic resonances (see the table below) that the base/acid/$H_2O$ of the sulfate was 1:1:1.

| Name | Theoretical ratio (base/ acid/ $H_2O$) | Theoretical water content (%) | Theoretical acid content (%) | Theoretical base content (%) | Measured water content (%) | Measured base content (%, HPLC) | Acid:base hydrogen atom number ratio ($^1$H-NMR) |
|---|---|---|---|---|---|---|---|
| Example 16 sulfate | 1:1:1 | 3.2% | 17.5% | 79.3% | 3.4% | 74.1% | N/A |

**Example 17: Preparation of a sulfate of the arylaminopurine derivative**

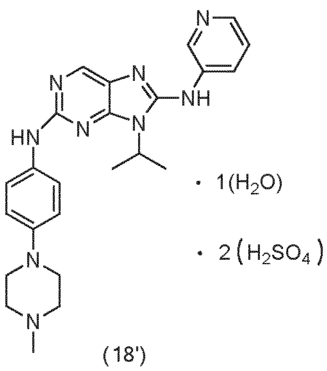

(18')

The preparation process of Example 16 was repeated except for changing the amount of succinic acid to (3.71g, 37.9mmol), and a pale yellow succinate (10.0g) was obtained. $^1$H-NMR(600MHz, $D_2O$) δ: 1.600(d, 6H), δ: 2.957(s, 3H),

δ: 3.243(m, 4H), δ: 3.644(d, 2H), δ: 3.829(d, 2H), δ: 4.757(m, 1H), δ: 7.208(d, 2H), δ: 7.294(d, 2H), δ: 8.014(m, 1H), δ: 8.155(s, 1H), δ: 8.485(d, 1H), δ: 8.685(m, 1H), δ: 9.217(d, 1H). The obtained sulfate exhibited good crystallinity, and its XRPD characterization pattern was shown in Figure 18. The main diffraction peak data were as follows:

| **Peak position 2θ angle (°)** | **Relative peak intensity %** |
|---|---|
| 8.622 | 33.2 |
| 9.588 | 78.1 |
| 15.681 | 44.6 |
| 16.519 | 14.5 |
| 17.129 | 31.1 |
| 19.269 | 50.0 |
| 20.033 | 49.8 |
| 21.862 | 29.5 |
| 23.467 | 21.6 |
| 24.352 | 16.5 |
| 26.649 | 100 |

It could be inferred from the calculation of the free base content by HPLC, the determination of the water content, and the hydrogen ratio in $^1$H-NMR of the nuclear magnetic resonances (see the table below) that the base/acid/$H_2O$ of the sulfate was 1:2:1.

| Name | Theoretical ratio (base/ acid/ $H_2O$) | Theoretical water content (%) | Theoretical acid content (%) | Theoretical base content (%) | Measured water content (%) | Measured base content (%, HPLC) | Acid:base hydrogen atom number ratio ($^1$H-NMR) |
|---|---|---|---|---|---|---|---|
| Example 17 sulfate | 1:2:1 | 2.7% | 29.8% | 67.5% | 3.5% | 68.0% | N/A |

Test example 1: DSC and TGA tests

The salts obtained in Examples 1 and 3-17 and the compound represented by Formula 1 were subjected to the DSC and TGA tests in media, and the test results were shown in the following table:

| **Example** | **Salt** | **DSC** | **TGA** |
|---|---|---|---|
| Example 1 | Hydrochloride | Easy to lose the water of crystallization and the acid, no obvious melting point (see Figure 19) | Starting to lose the water of crystallization at about 40°C; starting to lose the acid at about 140°C (see Figure 19) |
| Example 3 | Mesylate | Easy to lose the water of crystallization and the acid, no obvious melting point (see Figure 20) | Starting to lose the water of crystallization at about 40°C; starting to lose the acid at about 120°C (see Figure 21) |
| Example 4 | Mesylate | Easy to lose the water of crystallization and the acid, no obvious melting point (see Figure 22) | Starting to lose the water of crystallization at about 40°C; starting to lose the acid at about 220°C (see Figure 23) |
| Example 5 | Mesylate | Easy to lose the water of crystallization and the acid, no obvious melting point (see Figure 24) | Starting to lose the water of crystallization at about 40°C; starting to lose the acid at about 190°C (see Figure 25) |

(continued)

| Example | Salt | DSC | TGA |
|---|---|---|---|
| Example 6 | L-Malate | Easy to lose the water of crystallization and the acid, no obvious melting point (see Figure 26) | Starting to lose the water of crystallization at about 40°C; starting to lose the acid at about 170°C (see Figure 26) |
| Example 7 | L-Tartrate | Easy to lose the water of crystallization and the acid, no obvious melting point (see Figure 27) | Starting to lose the water of crystallization at about 40°C; starting to lose the acid at about 180°C (see Figure 27) |
| Example 8 | L-Tartrate | Easy to lose the water of crystallization and the acid, no obvious melting point (see Figure 28) | Starting to lose the water of crystallization at about 40°C; starting to lose the acid at about 180°C (see Figure 28) |
| Example 9 | L-Tartrate | Easy to lose the water of crystallization and the acid, no obvious melting point (see Figure 29) | Starting to lose the water of crystallization at about 40°C; starting to lose the acid at about 170°C (see Figure 29) |
| Example 10 | Oxalate | Easy to lose the water of crystallization and the acid, no obvious melting point (see Figure 30) | Starting to lose the water of crystallization at about 40°C; starting to lose the acid at about 170°C (see Figure 30) |
| Example 11 | Oxalate | Easy to lose the water of crystallization and the acid, no obvious melting point (see Figure 31) | Starting to lose the water of crystallization at about 40°C; starting to lose the acid at about 220°C (see Figure 31) |
| Example 12 | Succinate | Easy to lose the water of crystallization and the acid, no obvious melting point (see Figure 32) | Starting to lose the water of crystallization at about 40°C; starting to lose the acid at about 140°C (see Figure 33) |
| Example 13 | Succinate | Easy to lose the water of crystallization and the acid, no obvious melting point (see Figure 34) | Starting to lose the water of crystallization at about 40°C; starting to lose the acid at about 140°C (see Figure 35) |
| Example 14 | Acetate | Easy to lose the water of crystallization and the acid, no obvious melting point (see Figure 36) | Starting to lose the water of crystallization at about 40°C; starting to lose the acid at about 80°C (see Figure 37) |
| Example 15 | Acetate | Easy to lose the water of crystallization and the acid, no obvious melting point (see Figure 38) | Starting to lose the water of crystallization at about 40°C; starting to lose the acid at about 80°C (see Figure 39) |
| Example 16 | Sulfate | Easy to lose the water of crystallization and the acid, no obvious melting point (see Figure 40) | Starting to lose the water of crystallization at about 40°C; starting to lose the acid at about 240°C (See Figure 40) |
| Example 17 | Sulfate | Easy to lose the water of crystallization and the acid, no obvious melting point (see Figure 41) | Starting to lose the water of crystallization at about 40°C; starting to lose the acid at about 210°C (see Figure 41) |
| The compound represented by Formula 1 | | Starting to melt at about 150°C and decompose at about 155°C (see Figure 42) | Starting to decompose at about 155°C (see Figure 42) |

Test Example 2: Solubility Test

The salts obtained in Examples 1 and 3-17 and the compound represented by Formula 1 were subjected to the solubility test in media, and the test results were shown in the following table:

| Example | Salt | Solubility (25°C, mg/mL) | | | |
|---|---|---|---|---|---|
| | | Water medium | Water medium | Water medium | Water medium |
| | | | pH=1.2 | pH=4.5 | pH=6.8 |
| Example 1 | Hydrochloride | 40.41 | 33.89 | 47.27 | 50.62 |
| Example 3 | Mesylate | 212.73 | 360.00 | 207.53 | 153.30 |
| Example 4 | Mesylate | >1000 | >1000 | >1000 | >1000 |
| Example 5 | Mesylate | >1000 | >1000 | >1000 | >1000 |
| Example 6 | L-Malate | 9.68 | 54.70 | 14.59 | 12.05 |
| Example 7 | L-Tartrate | 6.91 | 43.07 | 6.51 | 12.24 |
| Example 8 | L-Tartrate | 9.74 | 36.41 | 14.36 | 10.46 |
| Example 9 | L-Tartrate | 10.50 | 44.51 | 15.17 | 11.48 |
| Example 10 | Oxalate | 2.49 | 66.52 | 13.98 | 6.36 |
| Example 11 | Oxalate | 27.78 | 87.38 | 16.13 | 8.61 |
| Example 12 | Succinate | 3.09 | 42.39 | 2.70 | 0.14 |
| Example 13 | Succinate | 5.06 | 59.71 | 8.42 | 1.12 |
| Example 14 | Acetate | 0.04 | 41.47 | 0.72 | 0.03 |
| Example 15 | Acetate | 63.57 | 96.58 | 45.67 | 27.84 |
| Example 16 | Sulfate | 76.59 | 129.50 | 90.03 | 90.09 |
| Example 17 | Sulfate | 16.51 | 33.29 | 21.64 | 31.39 |
| The compound represented by Formula 1 | | 0.05 | 12.60 | 0.57 | 0.04 |

Test Example 3: Accelerated Stability Test

Appropriate amounts of the salt samples obtained from Examples 1 and 3-17 were placed at a temperature of 25±2°C under 0%±5%RH in an open environment for 10 days and at a temperature of 40±2°C under 75%±5%RH in an open environment for 10 days respectively to perform the accelerated tests, and the results were as follows:

| **Example** | **Salt** | **Placed at 25±2°C under 0%±5%RH in an open evironment for 10 days** | | **Placed at 40±2°C under 75%±5%RH in an open evironment for 10 days** | |
|---|---|---|---|---|---|
| | | **Appearance** | **Crystal Form** | **Appearance** | **Crystal Form** |
| Example 1 | Hydrochloride | Pale yellow or yellow solid | Keeping consistent before and after being placed | Pale yellow or yellow solid | Keeping consistent before and after being placed |
| Example 3 | Mesylate | Pale yellow or yellow solid | Keeping consistent before and after being placed | Pale yellow or yellow solid | Keeping consistent before and after being placed |
| Example 4 | Mesylate | Liquid | N/a | Liquid | N/A |
| Example 5 | Mesylate | Liquid | N/a Liquid | Liquid | N/A |
| Example 6 | L-Malate | Yellow solid | Keeping consistent before and after being placed | Yellow solid | Keeping consistent before and after being placed |

(continued)

| Example | Salt | Placed at 25±2°C under 0%±5%RH in an open environment for 10 days | | Placed at 40±2°C under 75%±5%RH in an open environment for 10 days | |
|---|---|---|---|---|---|
| | | Appearance | Crystal Form | Appearance | Crystal Form |
| Example 7 | L-Tartrate | Yellow solid | Keeping consistent before and after being placed | Yellow solid | Keeping consistent before and after being placed |
| Example 8 | L-Tartrate | Pale yellow solid | Keeping consistent before and after being placed | Pale yellow solid | Keeping consistent before and after being placed |
| Example 9 | L-Tartrate | Pale yellow solid | Keeping consistent before and after being placed | Pale yellow solid | Keeping consistent before and after being placed |
| Example 10 | Oxalate | Yellow solid | Keeping consistent before and after being placed | Yellow solid | Keeping consistent before and after being placed |
| Example 11 | Oxalate | Yellow solid | Keeping consistent before and after being placed | Yellow solid | Keeping consistent before and after being placed |
| Example 12 | Succinate | Pale yellow solid | Keeping consistent before and after being placed | Pale yellow solid | Keeping consistent before and after being placed |
| Example 13 | Succinate | Pale yellow solid | Keeping consistent before and after being placed | Pale yellow solid | Keeping consistent before and after being placed |
| Example 14 | Acetate | Off-white solid | Keeping consistent before and after being placed | Off-white solid | Keeping consistent before and after being placed |
| Example 15 | Acetate | Off-white solid | Keeping consistent before and after being placed | Off-white solid | Keeping consistent before and after being placed |
| Example 16 | Sulfate | Pale yellow solid | Keeping consistent before and after being placed | Pale yellow solid | Keeping consistent before and after being placed |
| Example 17 | Sulfate | Pale yellow solid | Keeping consistent before and after being placed | Pale yellow solid | Keeping consistent before and after being placed |

Test Example 4: Hygroscopicity Test

Appropriate amounts of the salt samples obtained from Examples 1 and 3-17 were subjected to the hygroscopicity test at a temperature of 25±1°C under a relative humidity of 80%±2%, and the results were as follows:

| Example | Salt | Result of hygroscopicity test (DVS, 80%RH) | |
|---|---|---|---|
| | | Weight gain due to hygroscopicity | Hygroscopicity |
| Example 1 | Hydrochloride | 0.7% | Slightly hygroscopic |
| Example 3 | Mesylate | 5.86% | Hygroscopic |
| Example 4 | Mesylate | 6.94% | Hygroscopic |

(continued)

| Example | Salt | Result of hygroscopicity test (DVS, 80%RH) | |
|---|---|---|---|
| | | Weight gain due to hygroscopicity | Hygroscopicity |
| Example 5 | Mesylate | 19.62% | Very hygroscopic |
| Example 6 | L-Malate | 1.02% | Slightly hygroscopic |
| Example 7 | L-Tartrate | 1.19% | Slightly hygroscopic |
| Example 8 | L-Tartrate | 1.43% | Slightly hygroscopic |
| Example 9 | L-Tartrate | 1.43% | Slightly hygroscopic |
| Example 10 | Oxalate | 0.54% | Slightly hygroscopic |
| Example 11 | Oxalate | 0.68% | Slightly hygroscopic |
| Example 12 | Succinate | 0.11% | Not or nearly not hygroscopic |
| Example 13 | Succinate | 0.05% | Not or nearly not hygroscopic |
| Example 14 | Acetate | 1.59% | Slightly hygroscopic |
| Example 15 | Acetate | 2.59% | Hygroscopic |
| Example 16 | Sulfate | 7.67% | Hygroscopic |
| Example 17 | Sulfate | 1.34% | Slightly hygroscopic |
| The compound represented by Formula 1 | | 0.45% | Slightly hygroscopic |

Test Example 5: Long-Term Stability Test

An appropriate amount of the salt sample obtained from Example 1 was taken, a medicinal low-density polyethylene sack was used as the internal packaging, and a polyester/aluminum/polyethylene composite bag for medicine packaging was used as the external packaging. Samples were taken respectively at the end of the 3rd, 6th, 9th, 12th, and 18th months after being stored at a temperature of 25±2°C under a relative humidity of 60%±5%. The appearances were compared, followed by measuring other investigation indexes. The results were compared with those measured in the 0th month. The test results were shown in the following table:

| Time | Character | Moisture (%) | Acidity | Related substances (%) | Content (%) |
|---|---|---|---|---|---|
| 0 Month | Yellow crystalline powder | 13.9 | 3.1 | 0.33 | 100.9 |
| 3 Month | Yellow crystalline powder | 13.9 | 3.3 | 0.34 | 100.4 |
| 6 Month | Yellow crystalline powder | 14.3 | 3.3 | 0.34 | 100.8 |
| 9 Month | Yellow crystalline powder | 14.3 | 3.3 | 0.31 | 101.7 |
| 12 Month | Yellow crystalline powder | 13.9 | 3.3 | 0.34 | 99.6 |
| 18 Month | Yellow crystalline powder | 14.2 | 3.3 | 0.26 | 100.2 |

Test Example 6: Biological Activity Test

The salt sample obtained from Example 1 was tested according to the kinase inhibitory activity test described in the biological assessment of the patent application WO2011/147066. The test results showed that the sample could inhibit the activities of FLT3, EGFR, Abl, Fyn, Hck, Lck, Lyn, Ret, Yes, VEGFR2, ALK, BTK, c-KIT, c-SRC, FGFR1, KDR, MET and PDGFRα kinases, and the test results for some kinases were shown in the following table.

| Kinase | $IC_{50}$ (nM) |
|---|---|
| FLT3(h) | 26 |
| FLT3-ITD(h) | 3-10 |

(continued)

| Kinase | $IC_{50}$ (nM) |
|---|---|
| EGFR(h) | 42 |
| Abl(h) | 25 |
| Fyn(h) | 34 |
| Hck(h) | 93 |
| Lck(h) | 37 |
| Lyn(h) | 7 |
| Ret(h) | 10 |
| Yes | 4 |
| c-SRC(h) | 176 |
| FGFR1(h) | 247 |
| KDR(h) | 323 |

The salt sample obtained from Example 1 was tested (specifically for FLT3-ITD acute myeloid leukemia, non-small cell lung cancer with EGFR activating mutations, or Ph-positive chronic myeloid leukemia) according to the in-vivo anti-tumor test described in the biological assessment of the patent application WO2011/147066. The test result showed that, in the MV4-11 (FLT3-ITD mutation) subcutaneous tumor model test (with reference to the model established in Assay 4 of WO2011/147066), the sample (once daily oral administration for 21 days) could completely inhibit the tumor growth at the administration dose of 5 mg/kg, and could cause the complete regression of the tumor at the administration doses of 10 mg/kg and 20 mg/kg. In the non-small cell lung cancer model (with reference to the model established in Assay 3 of WO2011/147066), the sample could dose-dependently inhibit the growth of human non-small cell lung cancer HCC827: the tumor shrinkage (compared with the initial tumor) was caused in three dose groups of 7.5 mg/kg, 15 mg/kg and 30 mg/kg (once daily oral administration for 30 days), wherein the 30 mg/kg group could cause the nearly complete regression of the tumor. In the K562 (BCR-Abl gene rearrangement) subcutaneous tumor model test (a model established similarly to the MV4-11 subcutaneous tumor model), the sample (once daily oral administration for 18 days) could effectively inhibit the tumor growth at the administration dose of 70 mg/kg, and the tumor inhibition rate reached 71.3%.

The present disclosure provides the following technical solutions, but the present invention is not limited thereto, and the protection scope of the present invention is determined according to the scope defined by the claims:

[Technical Solution 1]. A salt of the arylaminopurine derivative, wherein said salt is represented by Formula 2:

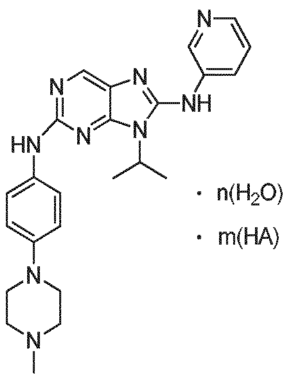

(2)

wherein,

HA is an acid;

$H_2O$ is the water of crystallization;

m is an integer or half-integer from 1 to 4;

n is an integer or half-integer from 0 to 5.

[Technical Solution 2]. The salt of the arylaminopurine derivative according to technical solution 1, wherein the acid is selected from a group consisting of hydrochloric acid, methanesulfonic acid, L- malic acid, L-tartaric acid, oxalic acid, succinic acid, acetic acid, or sulfuric acid; preferably hydrochloric acid, L-malic acid, L- tartaric acid, oxalic acid, succinic acid, acetic acid, or sulfuric acid; more preferably hydrochloric acid, L- malic acid, L- tartaric acid, oxalic acid, succinic acid or acetic acid; further preferably hydrochloric acid.

[Technical Solution 3]. The salt of the arylaminopurine derivative according to technical solution 1, wherein the salt is a hydrochloride represented by Formula 3:

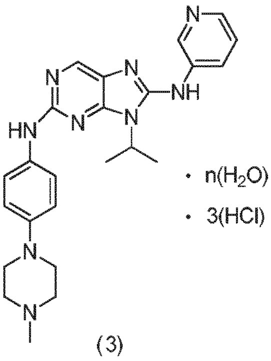

(3) ,

n is 0, 0.5, 1, 1.5, 2, 2.5, 3, 3.5, 4, 4.5, or 5;

preferably, the salt is a hydrochloride represented by Formula 3':

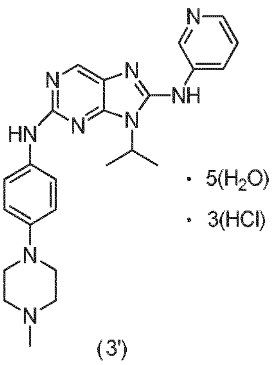

(3') ;

preferably,

the hydrochloride represented by Formula 3 or Formula 3' has an X-ray powder diffraction pattern comprising peaks at 2θ values of 8.5±0.2°, 11.8±0.2°, 19.6±0.2°, 25.2±0.2°, 27.2±0.2° as measured with CuKα radiation;

or, the hydrochloride represented by Formula 3 or Formula 3' has an X-ray powder diffraction pattern comprising peaks at 2θ values of 8.5±0.2°, 11.8±0.2°, 12.6±0.2°, 19.6±0.2°, 20.0±0.2°, 23.7±0.2°, 25.2±0.2°, 27.2±0.2° as measured with CuKα radiation;

or, the hydrochloride represented by Formula 3 or Formula 3' has an X-ray powder diffraction pattern comprising peaks at 2θ values of 7.3±0.2°, 8.5±0.2°, 9.0±0.2°, 11.8±0.2°, 12.6±0.2°, 14.3±0.2°, 18.1±0.2°, 19.6±0.2°, 20.0±0.2°, 21.1±0.2°, 21.9±0.2°, 23.7±0.2°, 25.2±0.2°, 26.1±0.2°, 27.2±0.2° as measured with CuKα radiation;

or, the hydrochloride represented by Formula 3 or Formula 3' has an X-ray powder diffraction pattern comprising peaks at 2θ values of 7.3±0.2°, 8.5±0.2°, 9.1±0.2°, 11.8±0.2°, 12.6±0.2°, 14.3±0.2°, 18.1±0.2°, 19.6±0.2°, 20.0±0.2°, 21.1±0.2°, 21.9±0.2°, 23.7±0.2°, 25.2±0.2°, 26.1±0.2°, 27.2±0.2° as measured with CuKα radiation;

or, the hydrochloride represented by Formula 3 or Formula 3' has an X-ray powder diffraction pattern substantially as shown in Figure 1 or Figure 3, as measured with CuKα radiation;

or, the single crystal of the hydrochloride represented by Formula 3 or Formula 3', as measured with CuKα radiation, belongs to the triclinic system, space group $P\bar{1}$, and has the unit cell parameters: {a=7.04142(7)Å, b=12.15291(7)Å, c=18.13188(10)Å, α=93.2215(5)°, β=95.3039(6)°, γ=91.9554(6)°, V=1541.32(2)Å$^3$}.

[Technical Solution 4]. The salt of the arylaminopurine derivative according to technical solution 1, wherein the salt is a mesylate represented by Formula 4, Formula 5, or Formula 6:

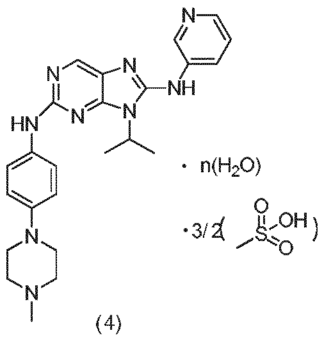
(4) ,
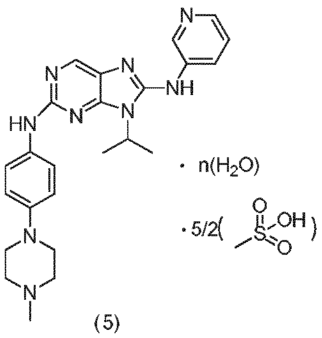
(5) ,
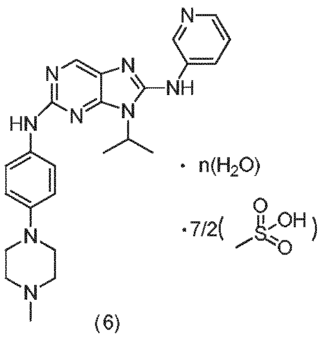
(6) ,

n is 0, 0.5, 1, 1.5, 2, 2.5, 3, 3.5, 4, 4.5, or 5;

preferably, the salt is a mesylate represented by Formula 4', Formula 5', or Formula 6':

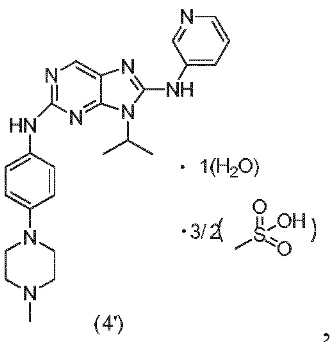
(4') ,
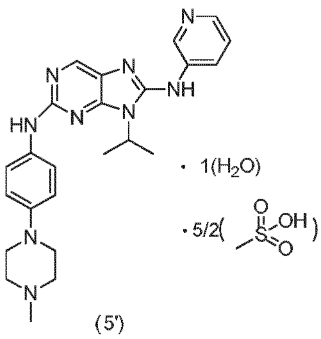
(5') ,
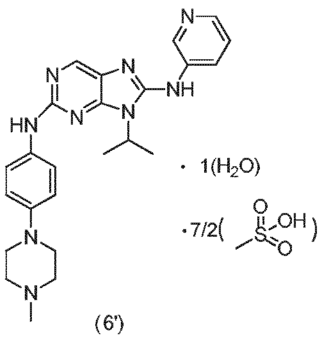
(6') ,

preferably,

the mesylate represented by Formula 4 or Formula 4' has an X-ray powder diffraction pattern comprising peaks at 2θ values of 6.8±0.2°, 15.1±0.2°, 16.3±0.2°, 21.0±0.2°, 25.0±0.2° as measured with CuKα radiation;
or, the mesylate represented by Formula 4 or Formula 4' has an X-ray powder diffraction pattern comprising peaks at 2θ values of 6.8±0.2°, 8.6±0.2°, 10.7±0.2°, 12.6±0.2°, 13.1±0.2°, 13.4±0.2°, 15.1±0.2°, 16.3±0.2°, 17.7±0.2°, 19.0±0.2°, 19.9±0.2°, 21.0±0.2°, 25.0±0.2° as measured with CuKα radiation;
or, the mesylate represented by Formula 4 or Formula 4' has an X-ray powder diffraction pattern substantially as shown in Figure 4, as measured with CuKα radiation;
or preferably,

the mesylate represented by Formula 5 or Formula 5' has an X-ray powder diffraction pattern comprising peaks at 2θ values of 6.1±0.2°, 6.4±0.2°, 17.4±0.2°, 18.9±0.2°, 19.3±0.2°, 24.4±0.2°, 26.4±0.2° as measured with CuKα radiation;
or, the mesylate represented by Formula 5 or Formula 5' has an X-ray powder diffraction pattern comprising peaks at 2θ values of 6.1±0.2°, 6.4±0.2°, 17.5±0.2°, 18.9±0.2°, 19.3±0.2°, 24.4±0.2°, 26.4±0.2° as measured with CuKα radiation;
or, the mesylate represented by Formula 5 or Formula 5' has an X-ray powder diffraction pattern comprising peaks at 2θ values of 6.1±0.2°, 6.4±0.2°, 11.7±0.2°, 12.4±0.2°, 16.0±0.2°, 16.6±0.2°, 16.9±0.2°, 17.4±0.2°, 18.0±0.2°, 18.9±0.2°, 19.3±0.2°, 19.9±0.2°, 20.2±0.2°, 23.4±0.2°, 24.4±0.2°, 26.4±0.2°, 27.3±0.2° as measured with CuKα radiation;
or, the mesylate represented by Formula 5 or Formula 5' has an X-ray powder diffraction pattern comprising peaks at 2θ values of 6.1±0.2°, 6.4±0.2°, 11.7±0.2°, 12.4±0.2°, 16.0±0.2°, 16.6±0.2°, 16.9±0.2°, 17.5±0.2°, 18.0±0.2°, 18.9±0.2°, 19.3±0.2°, 19.9±0.2°, 20.2±0.2°, 23.4±0.2°, 24.4±0.2°, 26.4±0.2°, 27.3±0.2° as measured with CuKα radiation;
or, the mesylate represented by Formula 5 or Formula 5' has an X-ray powder diffraction pattern substantially as shown in Figure 5, as measured with CuKα radiation;

or preferably,

the mesylate represented by Formula 6 or Formula 6' has an X-ray powder diffraction pattern comprising peaks at 2θ values of 4.9±0.2°, 11.5±0.2°, 14.5±0.2°, 18.5±0.2°, 18.9±0.2° as measured with CuKα radiation;
or, the mesylate represented by Formula 6 or Formula 6' has an X-ray powder diffraction pattern comprising peaks at 2θ values of 4.9:f:0.2°, 6.0±0.2°, 9.7±0.2°, 10.5±0.2°, 11.5±0.2°, 12.3±0.2°, 14.5±0.2°, 15.1±0.2°, 16.8±0.2°, 18.5±0.2°, 18.9±0.2°, 21.6±0.2°, 22.0±0.2°, 22.3±0.2°, 22.8±0.2°, 23.4±0.2°, 24.3±0.2°, 25.4±0.2°, 26.7±0.2°, 27.3±0.2° as measured with CuKα radiation;
or, the mesylate represented by Formula 6 or Formula 6' has an X-ray powder diffraction pattern substantially as shown in Figure 6, as measured with CuKα radiation.

[Technical Solution 5]. The salt of the arylaminopurine derivative according to technical solution 1, wherein the salt is an L-malate represented by Formula 7:

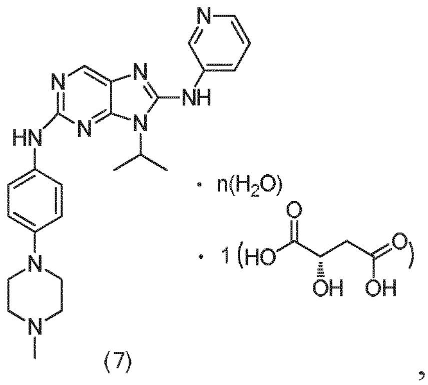
(7)
,

n is 0, 0.5, 1, 1.5, 2, 2.5, 3, 3.5, 4, 4.5, or 5;
preferably, the salt is an L-malate represented by Formula 7':

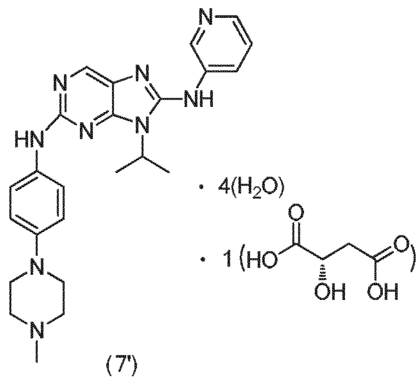
(7')
;

preferably,

the L-malate represented by Formula 7 or Formula 7' has an X-ray powder diffraction pattern comprising peaks at 2θ values of 7.0±0.2°, 9.3±0.2°, 17.6±0.2°, 19.7±0.2°, 25.9±0.2° as measured with CuKα radiation;
or, the L-malate represented by Formula 7 or Formula 7' has an X-ray powder diffraction pattern comprising peaks at 2θ values of 7.0±0.2°, 9.3±0.2°, 12.0±0.2°, 12.9±0.2°, 14.0±0.2°, 16.6±0.2°, 17.6±0.2°, 18.5±0.2°, 19.7±0.2°, 24.2±0.2°, 25.2±0.2°, 25.9±0.2°, 27.5±0.2° as measured with CuKα radiation;
or, the L-malate represented by Formula 7 or Formula 7' has an X-ray powder diffraction pattern comprising peaks at 2θ values of 7.0±0.2°, 9.3±0.2°, 12.0±0.2°, 12.9±0.2°, 14.0±0.2°, 16.6±0.2°, 17.6±0.2°, 18.5±0.2°, 19.7±0.2°, 23.0±0.2°, 24.2±0.2°, 25.2±0.2°, 25.9±0.2°, 27.5±0.2° as measured with CuKα radiation;
or, the L-malate represented by Formula 7 or Formula 7' has an X-ray powder diffraction pattern substantially as shown in Figure 7, as measured with CuKα radiation.

[Technical Solution 6]. The salt of the arylaminopurine derivative according to technical solution 1, wherein the salt is an L-tartrate represented by Formula 8, Formula 9, or Formula 10:

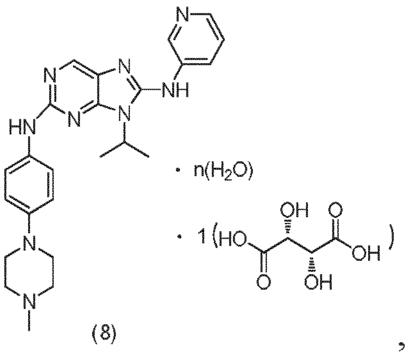

(8) ,

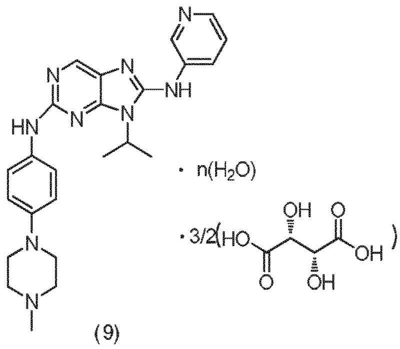

(9) ,

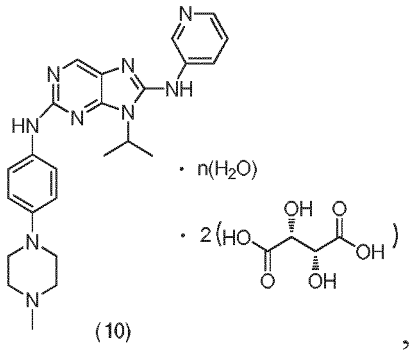

(10) ,

n is 0, 0.5, 1, 1.5, 2, 2.5, 3, 3.5, 4, 4.5, or 5;

preferably, the salt is an L-tartrate represented by Formula 8', Formula 9', or Formula 10':

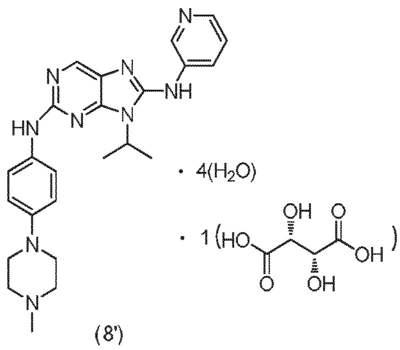

(8') ,

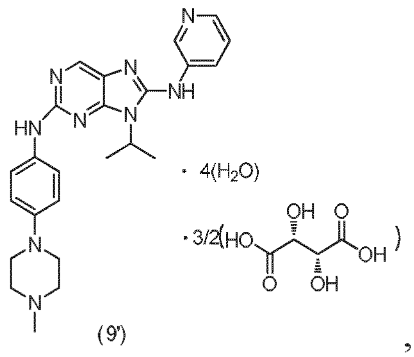

(9') ,

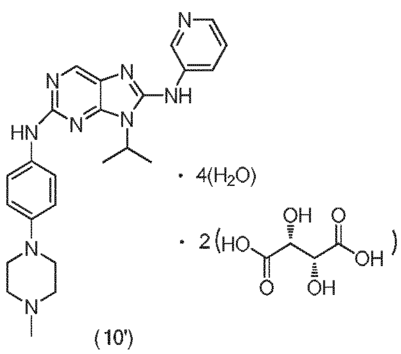

(10') ,

preferably,

the L-tartrate represented by Formula 8 or Formula 8' has an X-ray powder diffraction pattern comprising peaks at 2θ values of 6.9:f:0.2°, 9.1±0.2°, 17.8±0.2°, 19.4±0.2°, 25.5±0.2° as measured with CuKα radiation;
or, the L-tartrate represented by Formula 8 or Formula 8' has an X-ray powder diffraction pattern comprising peaks at 2θ values of 6.9±0.2°, 9.1±0.2°, 12.9±0.2°, 13.8±0.2°, 16.5±0.2°, 17.8±0.2°, 19.4±0.2°, 20.1±0.2°, 25.5±0.2°, 26.9±0.2° as measured with CuKα radiation;
or, the L-tartrate represented by Formula 8 or Formula 8' has an X-ray powder diffraction pattern substantially as shown in Figure 8, as measured with CuKα radiation;
or preferably,

the L-tartrate represented by Formula 9 or Formula 9' has an X-ray powder diffraction pattern comprising peaks at 2θ values of 8.5±0.2°, 14.8±0.2°, 17.1±0.2°, 18.8±0.2°, 24.6±0.2°, 26.1±0.2° as measured with CuKα radiation;
or, the L-tartrate represented by Formula 9 or Formula 9' has an X-ray powder diffraction pattern comprising peaks at 2θ values of 8.5±0.2°, 9.8±0.2°, 10.1±0.2°, 11.3±0.2°, 13.7±0.2°, 14.8±0.2°, 15.4±0.2°, 16.3±0.2°, 17.1±0.2°, 17.6±0.2°, 18.8±0.2°, 20.5±0.2°, 22.3±0.2°, 24.6±0.2°, 26.1±0.2° as measured with CuKα radiation;
or, the L-tartrate represented by Formula 9 or Formula 9' has an X-ray powder diffraction pattern substantially as shown in Figure 9, as measured with CuKα radiation;

or preferably,

the L-tartrate represented by Formula 10 or Formula 10' has an X-ray powder diffraction pattern comprising peaks at 2θ values of 8.3±0.2°, 8.9±0.2°, 9.5±0.2°, 14.8±0.2°, 17.7±0.2°, 21.0±0.2°, 24.0±0.2° as measured with CuKα radiation;
or, the L-tartrate represented by Formula 10 or Formula 10' has an X-ray powder diffraction pattern comprising peaks at 2θ values of 7.0±0.2°, 8.3±0.2°, 8.9±0.2°, 9.5±0.2°, 12.5±0.2°, 13.1±0.2°, 14.8±0.2°, 16.0±0.2°, 17.7±0.2°, 18.1±0.2°, 19.2±0.2°, 21.0±0.2°, 23.6±0.2°, 24.0±0.2°, 25.3±0.2°, 26.7±0.2° as measured with CuKα radiation;
or, the L-tartrate represented by Formula 10 or Formula 10' has an X-ray powder diffraction pattern substantially as shown in Figure 10, as measured with CuKα radiation.

[Technical Solution 7]. The salt of the arylaminopurine derivative according to technical solution 1, wherein the salt is an oxalate represented by Formula 11, or Formula 12:

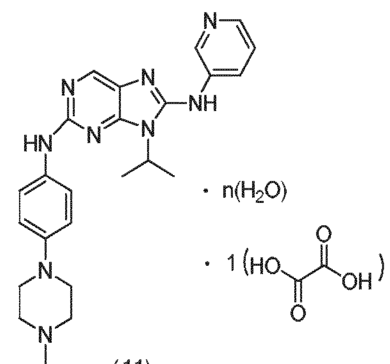
(11) ,

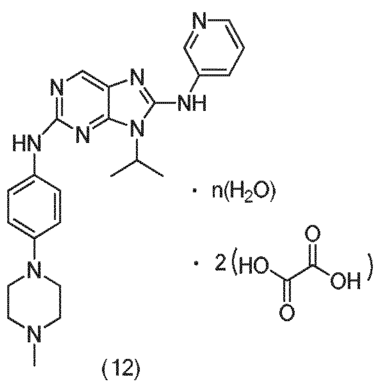
(12) ,

n is 0, 0.5, 1, 1.5, 2, 2.5, 3, 3.5, 4, 4.5, or 5;
preferably, the salt is an oxalate represented by Formula 11', or Formula 12':

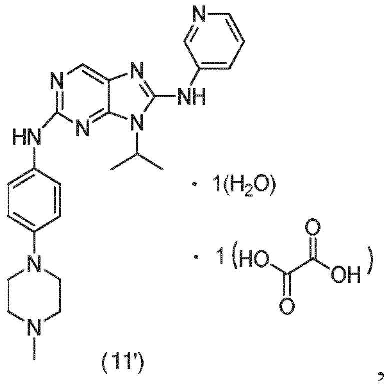

(11'),

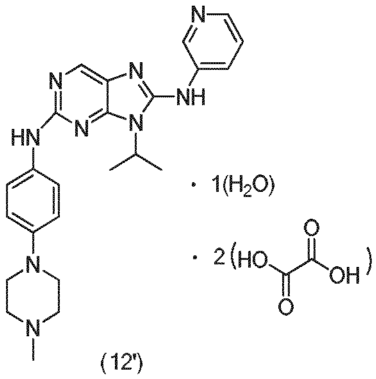

(12'),

preferably,

the oxalate represented by Formula 11 or Formula 11' has an X-ray powder diffraction pattern comprising peaks at 2θ values of 8.1±0.2°, 8.4±0.2°, 9.0±0.2°, 14.1±0.2°, 16.7±0.2°, 25.6±0.2° as measured with CuKα radiation;
or, the oxalate represented by Formula 11 or Formula 11' has an X-ray powder diffraction pattern comprising peaks at 2θ values of 8.1±0.2°, 8.4±0.2°, 9.0±0.2°, 14.1±0.2°, 14.8±0.2°, 16.7±0.2°, 17.9±0.2°, 18.5±0.2°, 19.6±0.2°, 23.6±0.2°, 25.6±0.2° as measured with CuKα radiation;
or, the oxalate represented by Formula 11 or Formula 11' has an X-ray powder diffraction pattern substantially as shown in Figure 11, as measured with CuKα radiation;

or preferably,

the oxalate represented by Formula 12 or Formula 12' has an X-ray powder diffraction pattern comprising peaks at 2θ values of 7.1±0.2°, 12.2±0.2°, 14.2±0.2°, 16.4±0.2°, 17.7±0.2°, 19.0±0.2°, 24.4±0.2° as measured with CuKα radiation;
or, the oxalate represented by Formula 12 or Formula 12' has an X-ray powder diffraction pattern comprising peaks at 2θ values of 7.1±0.2°, 8.3±0.2°, 12.2±0.2°, 14.2±0.2°, 16.4±0.2°, 17.7±0.2°, 18.6±0.2°, 19.0±0.2°, 24.4±0.2° as measured with CuKα radiation;
or, the oxalate represented by Formula 12 or Formula 12' has an X-ray powder diffraction pattern substantially as shown in Figure 12, as measured with CuKα radiation.

[Technical Solution 8]. The salt of the arylaminopurine derivative according to technical solution 1, wherein the salt is a succinate represented by Formula 13, or Formula 14:

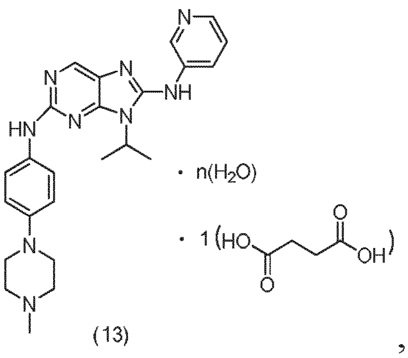

(13),

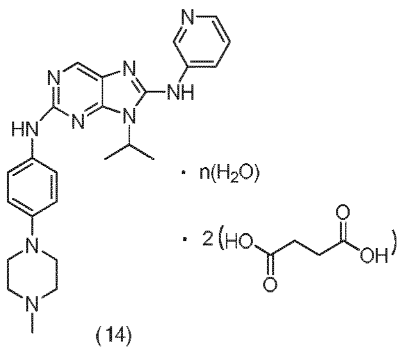

(14),

n is 0, 0.5, 1, 1.5, 2, 2.5, 3, 3.5, 4, 4.5, or 5;
preferably, the salt is a succinate represented by Formula 13', or Formula 14':

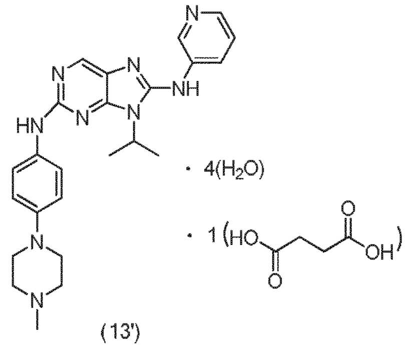

(13'),

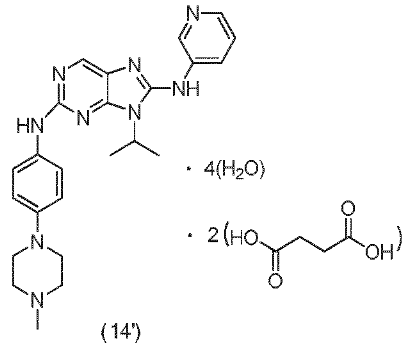

(14'),

preferably,

the succinate represented by Formula 13 or Formula 13' has an X-ray powder diffraction pattern comprising peaks at 2θ values of 7.0±0.2°, 9.1±0.2°, 11.3±0.2°, 16.8±0.2°, 20.4±0.2°, 21.0±0.2°, 22.4±0.2° as measured with CuKα radiation;

or, the succinate represented by Formula 13 or Formula 13' has an X-ray powder diffraction pattern comprising peaks at 2θ values of 7.0±0.2°, 9.1±0.2°, 18.5±0.2°, 20.4±0.2°, 21.0±0.2°, 22.4±0.2°, 27.1±0.2° as measured with CuKα radiation;

or, the succinate represented by Formula 13 or Formula 13' has an X-ray powder diffraction pattern comprising peaks at 2θ values of 7.0±0.2°, 9.1±0.2°, 11.3±0.2°, 13.1±0.2°, 13.8±0.2°, 14.4±0.2°, 16.0±0.2°, 16.8±0.2°, 17.7±0.2°, 18.5±0.2°, 20.4±0.2°, 21.0±0.2°, 22.4±0.2°, 24.2±0.2°, 25.9±0.2°, 27.1±0.2° as measured with CuKα radiation;

or, the succinate represented by Formula 13 or Formula 13' has an X-ray powder diffraction pattern substantially as shown in Figure 13, as measured with CuKα radiation;

or preferably,

the succinate represented by Formula 14 or Formula 14' has an X-ray powder diffraction pattern comprising peaks at 2θ values of 7.0±0.2°, 9.2±0.2°, 17.6±0.2°, 18.4±0.2°, 19.7±0.2°, 25.8±0.2°, 27.3±0.2° as measured with CuKα radiation;

or, the succinate represented by Formula 14 or Formula 14' has an X-ray powder diffraction pattern comprising peaks at 2θ values of 7.0±0.2°, 9.2±0.2°, 11.9±0.2°, 16.7±0.2°, 17.6±0.2°, 18.4±0.2°, 19.7±0.2°, 23.0±0.2°, 24.1±0.2°, 25.2±0.2°, 25.8±0.2°, 27.3±0.2° as measured with CuKα radiation;

or, the succinate represented by Formula 14 or Formula 14' has an X-ray powder diffraction pattern comprising peaks at 2θ values of 7.0±0.2°, 9.2±0.2°, 11.9±0.2°, 16.7±0.2°, 17.6±0.2°, 18.4±0.2°, 19.7±0.2°, 20.3±0.2°, 23.0±0.2°, 24.1±0.2°, 25.2±0.2°, 25.8±0.2°, 27.3±0.2° as measured with CuKα radiation;

or, the succinate represented by Formula 14 or Formula 14' has an X-ray powder diffraction pattern substantially as shown in Figure 14, as measured with CuKα radiation.

[Technical Solution 9]. The salt of the arylaminopurine derivative according to technical solution 1, wherein the salt is an acetate represented by Formula 15, or Formula 16:

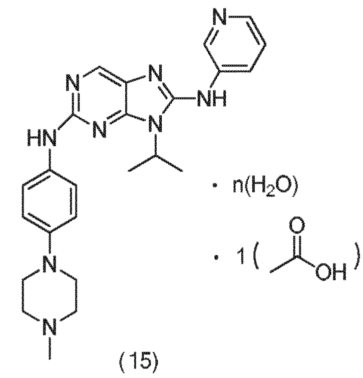

(15),

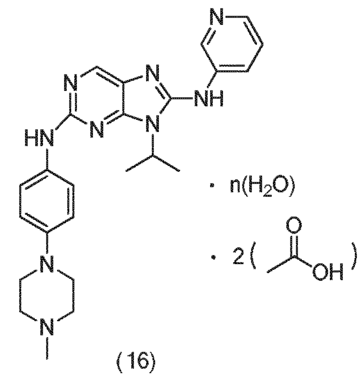

(16),

n is 0, 0.5, 1, 1.5, 2, 2.5, 3, 3.5, 4, 4.5, or 5;
preferably, the salt is an acetate represented by Formula 15', or Formula 16':

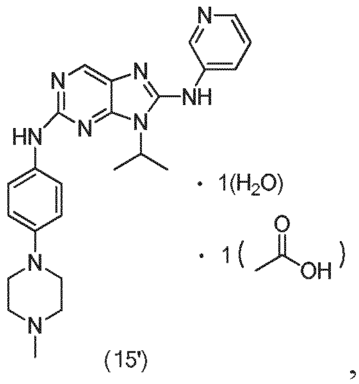
(15'),
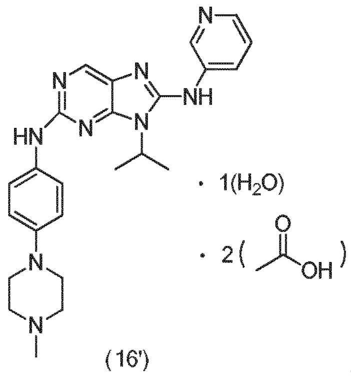
(16'),

preferably,

the acetate represented by Formula 15 or Formula 15' has an X-ray powder diffraction pattern comprising peaks at 2θ values of 10.9±0.2°, 12.6±0.2°, 15.1±0.2°, 17.8±0.2°, 19.2±0.2°, 19.6±0.2°, 21.0±0.2°, 21.8±0.2°, 22.3±0.2°, 24.6±0.2°, 25.4±0.2° as measured with CuKα radiation;
or, the acetate represented by Formula 15 or Formula 15' has an X-ray powder diffraction pattern comprising peaks at 2θ values of 6.3±0.2°, 8.9±0.2°, 10.9±0.2°, 11.5±0.2°, 12.2±0.2°, 12.6±0.2°, 15.1±0.2°, 17.8±0.2°, 19.2±0.2°, 19.6±0.2°, 21.0±0.2°, 21.8±0.2°, 22.3±0.2°, 24.6±0.2°, 25.4±0.2° as measured with CuKα radiation;
or, the acetate represented by Formula 15 or Formula 15' has an X-ray powder diffraction pattern substantially as shown in Figure 15, as measured with CuKα radiation;

or preferably,

the acetate represented by Formula 16 or Formula 16' has an X-ray powder diffraction pattern comprising peaks at 2θ values of 6.2±0.2°, 12.2±0.2°, 16.1±0.2°, 17.5±0.2°, 23.4±0.2°, 24.8±0.2° or at 2θ values of 6.2±0.2°, 12.2±0.2°, 17.5±0.2°, 21.5±0.2°, 23.4±0.2°, 24.8±0.2° as measured with CuKα radiation;
or, the acetate represented by Formula 16 or Formula 16' has an X-ray powder diffraction pattern comprising peaks at 2θ values of 6.2±0.2°, 8.1±0.2°, 9.1±0.2°, 12.2±0.2°, 15.0±0.2°, 16.1±0.2°, 17.5±0.2°, 18.2±0.2°, 20.7±0.2°, 21.5±0.2°, 23.4±0.2°, 24.8±0.2°, 28.8±0.2° as measured with CuKα radiation;
or, the acetate represented by Formula 16 or Formula 16' has an X-ray powder diffraction pattern substantially as shown in Figure 16, as measured with CuKα radiation.

[Technical Solution 10]. The salt of the arylaminopurine derivative according to technical solution 1, wherein the salt is a sulfate represented by Formula 17, or Formula 18:

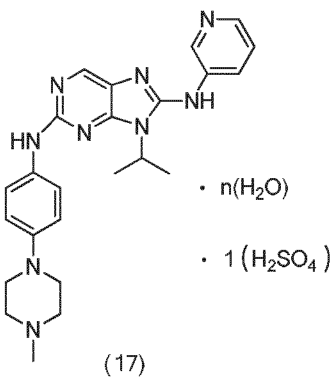
(17),
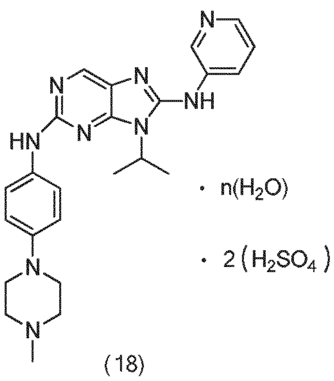
(18),

n is 0, 0.5, 1, 1.5, 2, 2.5, 3, 3.5, 4, 4.5, or 5;

preferably, the salt is a sulfate represented by Formula 17', or Formula 18':

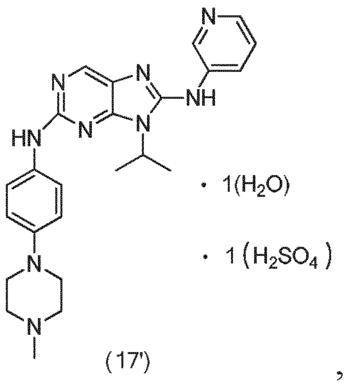
(17'),

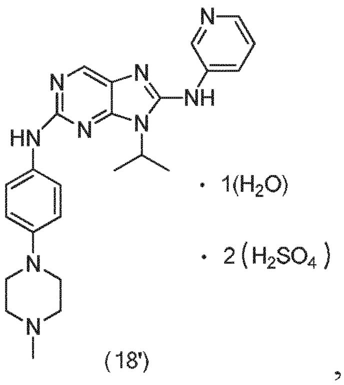
(18'),

preferably, the sulfate represented by Formula 17 or Formula 17' has an X-ray powder diffraction pattern comprising peaks at 2θ values of 4.8±0.2°, 7.0±0.2°, 9.5±0.2°, 13.6±0.2°, 15.7±0.2°, 18.6±0.2°, 21.6±0.2°, 25.7±0.2°as measured with CuKα radiation;
or, the sulfate represented by Formula 17 or Formula 17' has an X-ray powder diffraction pattern comprising peaks at 2θ values of 4.8±0.2°, 7.0±0.2°, 9.2±0.2°, 9.5±0.2°, 13.6±0.2°, 15.7±0.2°, 18.6±0.2°, 21.6±0.2°, 25.7±0.2° as measured with CuKα radiation;
or, the sulfate represented by Formula 17 or Formula 17' has an X-ray powder diffraction pattern comprising peaks at 2θ values of 4.8±0.2°, 7.0±0.2°, 8.6±0.2°, 9.2±0.2°, 9.5±0.2°, 11.6±0.2°, 12.8±0.2°, 13.6±0.2°, 15.7±0.2°, 17.6±0.2°, 18.6±0.2°, 20.5±0.2°, 21.6±0.2°, 23.8±0.2°, 25.7±0.2°as measured with CuKα radiation;
or, the sulfate represented by Formula 17 or Formula 17' has an X-ray powder diffraction pattern substantially as shown in Figure 17, as measured with CuKα radiation;
or preferably,

the sulfate represented by Formula 18 or Formula 18' has an X-ray powder diffraction pattern comprising peaks at 2θ values of 8.6+0.2°, 9.6±0.2°, 15.7±0.2°, 19.3±0.2°, 20.0±0.2°, 21.9±0.2°, 26.6±0.2° as measured with CuKα radiation;
or, the sulfate represented by Formula 18 or Formula 18' has an X-ray powder diffraction pattern comprising peaks at 2θ values of 8.6±0.2°, 9.6±0.2°, 15.7±0.2°, 17.1±0.2°, 19.3±0.2°, 20.0±0.2°, 26.6±0.2° as measured with CuKα radiation;
or, the sulfate represented by Formula 18 or Formula 18' has an X-ray powder diffraction pattern comprising peaks at 2θ values of 8.6±0.2°, 9.6+0.2°, 15.7+0.2°, 16.5+0.2°, 17.1±0.2°, 19.3±0.2°, 20.0+0.2°, 21.9±0.2°, 23.5±0.2°, 24.4±0.2°, 26.6±0.2° as measured with CuKα radiation;
or, the sulfate represented by Formula 18 or Formula 18' has an X-ray powder diffraction pattern substantially as shown in Figure 18, as measured with CuKα radiation.

[Technical Solution 11]. A pharmaceutical composition, comprising the salt represented by Formula 2 of the arylaminopurine derivative according to any of technical solutions 1-10.

[Technical Solution 12]. Use of the salt represented by Formula 2 of the arylaminopurine derivative according to any of technical solutions 1-10 or the pharmaceutical composition according to technical solution 11 in manufacture of a medicament as the protein kinase inhibitor, wherein the kinase is selected from FLT3, EGFR, Abl, Fyn, Hck, Lck, Lyn, Ret, Yes, VEGFR2, ALK, BTK, c-KIT, c-SRC, FGFR1, KDR, MET or PDGFRα;
preferably, the medicament as the protein kinase inhibitor is an antitumor drug, the tumor is selected from non-small cell lung cancer, acute myeloid leukemia, chronic myelocytic leukemia, chronic myeloid leukemia, squamous cell carcinoma, mammary cancer, colorectal cancer, liver cancer, stomach cancer, and malignant melanoma, more preferably leukemia or lung cancer, further more preferably acute myeloid leukemia or non-small cell lung cancer, further preferably FLT3 mutation-positive acute myeloid leukemia (such as FLT3-ITD acute myeloid leukemia), Ph-positive chronic myeloid leukemia or non-small cell lung cancer with EGFR activating mutations.

[Technical Solution 13]. A method for preparing the salt represented by Formula 2 of the arylaminopurine derivative according to technical solution 1, which comprises a reaction of an arylaminopurine derivative represented by Formula 1 and an acid is performed in the presence of water and an organic solvent to obtain the salt represented by Formula 2 of the arylaminopurine derivative:

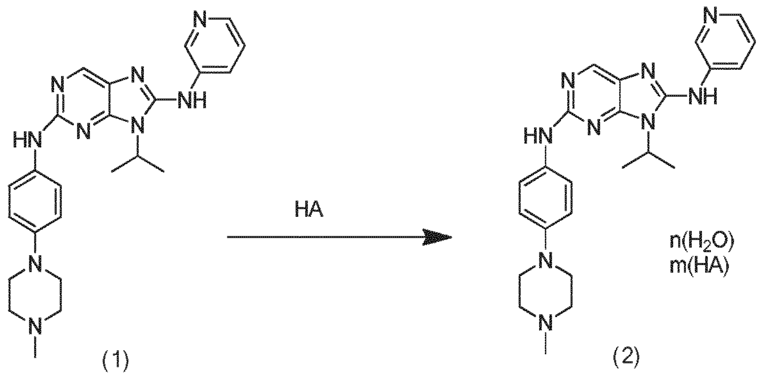

(1) (2)

,

wherein,

HA is an acid;
$H_2O$ is the water of crystallization;
m is an integer or half-integer from 1 to 4;
n is an integer or half-integer from 0 to 5.

[Technical Solution 14]. The method for preparing the salt of the arylaminopurine derivative according to technical solution 13, wherein the molar ratio of the arylaminopurine derivative represented by Formula 1 to the acid is 1:1 to 1:4, preferably 1:1.2 to 1:3.5;

the reaction temperature is 0-70°C, preferably 35-45°C;
the reaction is performed in the presence of the combination of water and one or more organic solvents selected from alcohols, ethers, esters, ketones, nitriles, and alkanes, preferably in the presence of $C_1$-$C_3$ lower alcohol and water, in the presence of a ketone and water, in the presence of a nitrile and water, or the presence of ether and water, and more preferably in the presence of methanol-water, ethanol-water, isopropanol-water, tetrahydrofuran-water, dioxane-water, acetone-water or acetonitrile-water; and the ratio of the use amounts by volume of the organic solvent to water is 1:10 to 10:1, for example, 1:1 to 10:1 or 1:10 to 1:1.

## Claims

**1.** A salt of the arylaminopurine derivative, wherein said salt is represented by Formula 2:

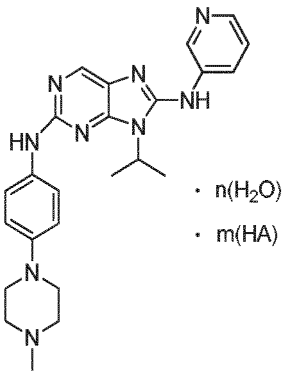

(2)

wherein,

HA is an acid;
$H_2O$ is the water of crystallization;
m is an integer or half-integer from 1 to 4;
n is an integer or half-integer from 0 to 5.

**2.** The salt of the arylaminopurine derivative according to claim 1, wherein the acid is selected from a group consisting of hydrochloric acid, methanesulfonic acid, L-malic acid, L-tartaric acid, oxalic acid, succinic acid, acetic acid, or sulfuric acid; preferably hydrochloric acid, L-malic acid, L-tartaric acid, oxalic acid, succinic acid, acetic acid, or sulfuric acid; more preferably hydrochloric acid, L-malic acid, L-tartaric acid, oxalic acid, succinic acid or acetic acid; further preferably hydrochloric acid.

**3.** The salt of the arylaminopurine derivative according to claim 1, wherein the salt is a hydrochloride represented by Formula 3:

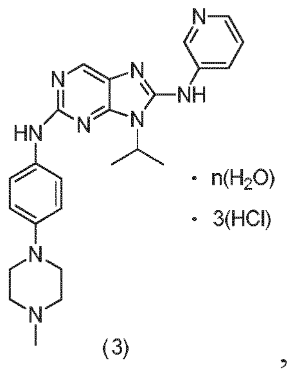

(3) ,

n is 0, 0.5, 1, 1.5, 2, 2.5, 3, 3.5, 4, 4.5, or 5;
preferably, the salt is a hydrochloride represented by Formula 3':

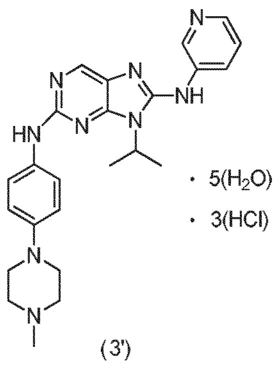

(3') ;

more preferably, the hydrochloride represented by Formula 3 or Formula 3' has an X-ray powder diffraction pattern comprising peaks at 2θ values of 8.5+0.2°, 11.8+0.2°, 19.6±0.2°, 25.2±0.2°, 27.2±0.2° as measured with CuKα radiation; Further more preferably, the hydrochloride represented by Formula 3 or Formula 3' has an X-ray powder diffraction pattern substantially as shown in Figure 1 or Figure 3, as measured with CuKα radiation;
or more preferably, the single crystal of the hydrochloride represented by Formula 3 or Formula 3', as measured with CuKα radiation, belongs to the triclinic system, space group $P\bar{1}$, and has the unit cell parameters: {a=7.04142(7)Å, b=12.15291(7)Å, c=18.13188(10)Å, α=93.2215(5)°, β=95.3039(6)°, γ=91.9554(6)°, V=1541.32(2)$Å^3$}.

**4.** The salt of the arylaminopurine derivative according to claim 1, wherein the salt is a mesylate represented by Formula 4, Formula 5, or Formula 6:

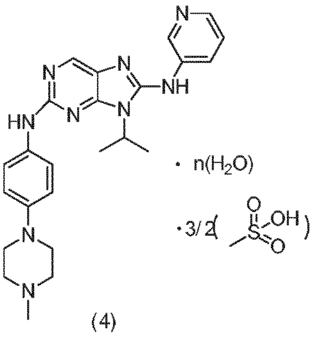

(4)

,

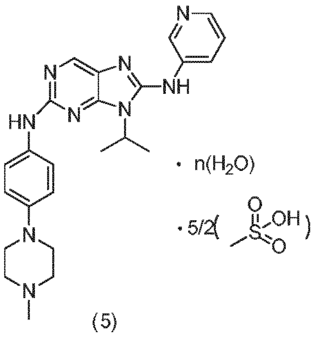

(5)

,

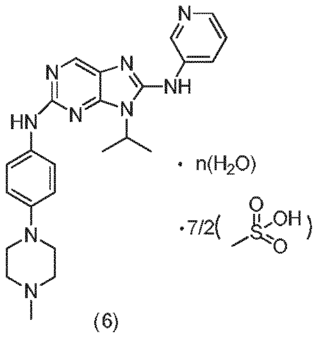

(6)

,

n is 0, 0.5, 1, 1.5, 2, 2.5, 3, 3.5, 4, 4.5, or 5;
preferably, the salt is a mesylate represented by Formula 4', Formula 5', or Formula 6':

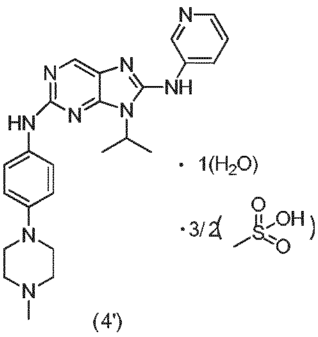

(4')

,

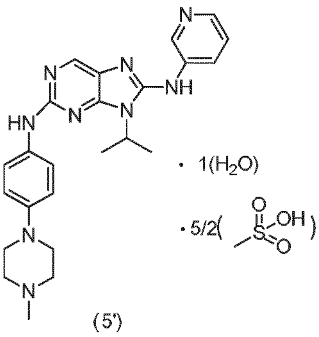

(5')

,

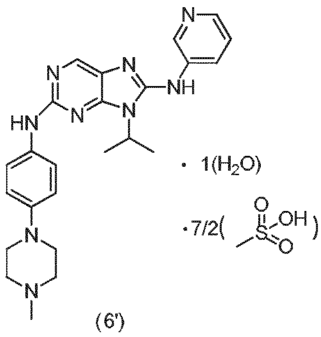

(6')

,

more preferably, the mesylate represented by Formula 4 or Formula 4' has an X-ray powder diffraction pattern comprising peaks at 2θ values of 6.8±0.2°, 15.1±0.2°, 16.3±0.2°, 21.0±0.2°, 25.0±0.2° as measured with CuKα radiation; further more preferably, the mesylate represented by Formula 4 or Formula 4' has an X-ray powder diffraction pattern substantially as shown in Figure 4, as measured with CuKα radiation;
or more preferably, the mesylate represented by Formula 5 or Formula 5' has an X-ray powder diffraction pattern comprising peaks at 2θ values of 6.1±0.2°, 6.4+0.2°, 17.5+0.2°, 18.9±0.2°, 19.3+0.2°, 24.4±0.2°, 26.4+0.2° as measured with CuKα radiation; further more preferably, the mesylate represented by Formula 5 or Formula 5' has an X-ray powder diffraction pattern substantially as shown in Figure 5, as measured with CuKα radiation;
or more preferably, the mesylate represented by Formula 6 or Formula 6' has an X-ray powder diffraction pattern comprising peaks at 2θ values of 4.9±0.2°, 11.5±0.2°, 14.5±0.2°, 18.5±0.2°, 18.9±0.2° as measured with CuKα radiation; further more preferably, the mesylate represented by Formula 6 or Formula 6' has an X-ray powder diffraction pattern substantially as shown in Figure 6, as measured with CuKα radiation.

**5.** The salt of the arylaminopurine derivative according to claim 1, wherein the salt is an L-malate represented by Formula 7:

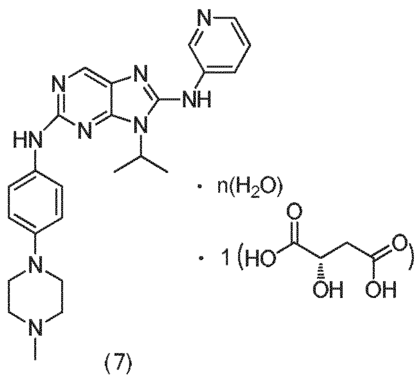

(7)

,

n is 0, 0.5, 1, 1.5, 2, 2.5, 3, 3.5, 4, 4.5, or 5;
preferably, the salt is an L-malate represented by Formula 7':

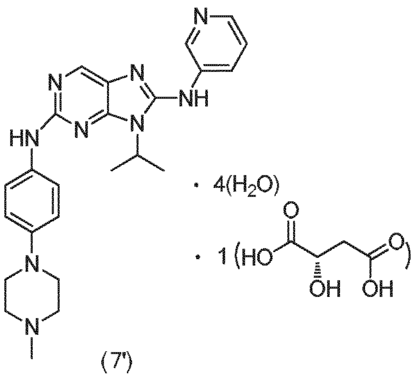

(7') ;

more preferably, the L-malate represented by Formula 7 or Formula 7' has an X-ray powder diffraction pattern comprising peaks at 2θ values of 7.0±0.2°, 9.3±0.2°, 17.6±0.2°, 19.7±0.2°, 25.9±0.2° as measured with CuKα radiation; further more preferably, the L-malate represented by Formula 7 or Formula 7' has an X-ray powder diffraction pattern substantially as shown in Figure 7, as measured with CuKα radiation.

**6.** The salt of the arylaminopurine derivative according to claim 1, wherein the salt is an L-tartrate represented by Formula 8, Formula 9, or Formula 10:

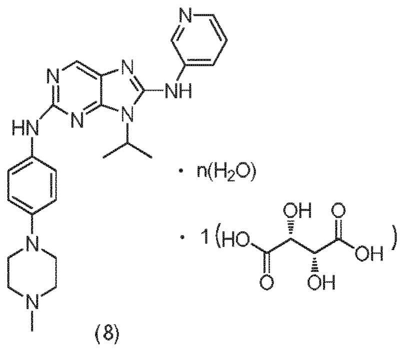

(8) ,

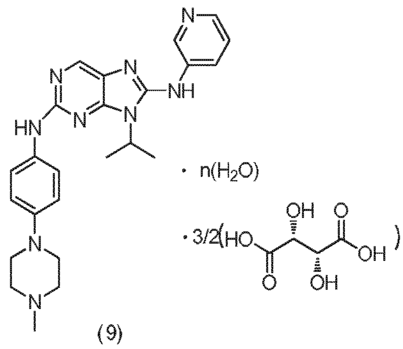

(9) ,

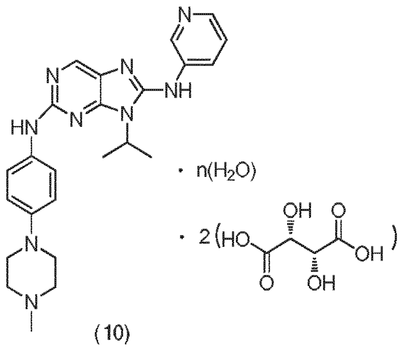

(10) ,

n is 0, 0.5, 1, 1.5, 2, 2.5, 3, 3.5, 4, 4.5, or 5;
preferably, the salt is an L-tartrate represented by Formula 8', Formula 9', or Formula 10':

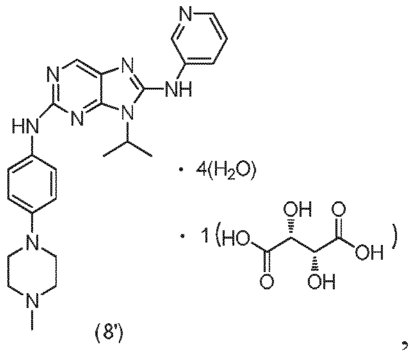

(8'),

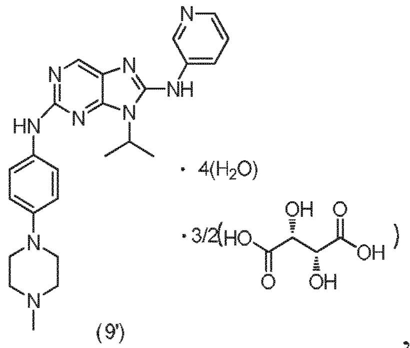

(9'),

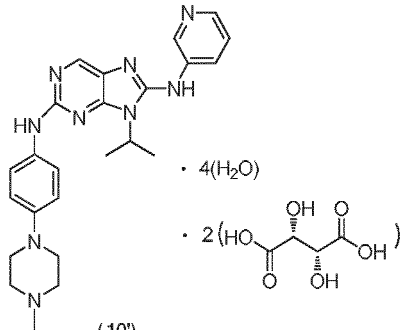

(10'),

more preferably, the L-tartrate represented by Formula 8 or Formula 8' has an X-ray powder diffraction pattern comprising peaks at 2θ values of 6.9±0.2°, 9.1±0.2°, 17.8±0.2°, 19.4±0.2°, 25.5±0.2° as measured with CuKα radiation; further more preferably, the L-tartrate represented by Formula 8 or Formula 8' has an X-ray powder diffraction pattern substantially as shown in Figure 8, as measured with CuKα radiation;
or more preferably, the L-tartrate represented by Formula 9 or Formula 9' has an X-ray powder diffraction pattern comprising peaks at 2θ values of 8.5+0.2°, 14.8±0.2°, 17.1±0.2°, 18.8±0.2°, 24.6±0.2°, 26.1±0.2° as measured with CuKα radiation; further more preferably, the L-tartrate represented by Formula 9 or Formula 9' has an X-ray powder diffraction pattern substantially as shown in Figure 9, as measured with CuKα radiation;
or more preferably, the L-tartrate represented by Formula 10 or Formula 10' has an X-ray powder diffraction pattern comprising peaks at 2θ values of 8.3±0.2°, 8.9±0.2°, 9.5±0.2°, 14.8±0.2°, 17.7±0.2°, 21.0±0.2°, 24.0±0.2° as measured with CuKα radiation; further more preferably, the L-tartrate represented by Formula 10 or Formula 10' has an X-ray powder diffraction pattern substantially as shown in Figure 10, as measured with CuKα radiation.

**7.** The salt of the arylaminopurine derivative according to claim 1, wherein the salt is an oxalate represented by Formula 11, or Formula 12:

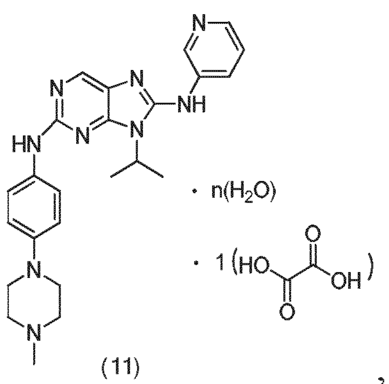

(11),

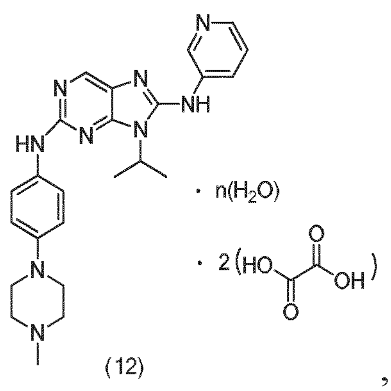

(12),

n is 0, 0.5, 1, 1.5, 2, 2.5, 3, 3.5, 4, 4.5, or 5;

preferably, the salt is an oxalate represented by Formula 11', or Formula 12':

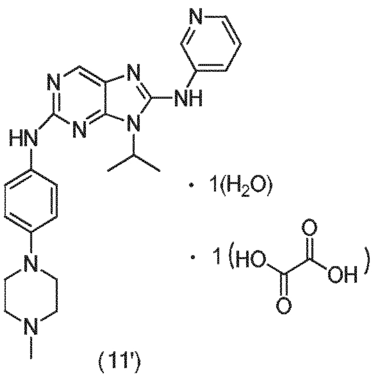

(11') ,

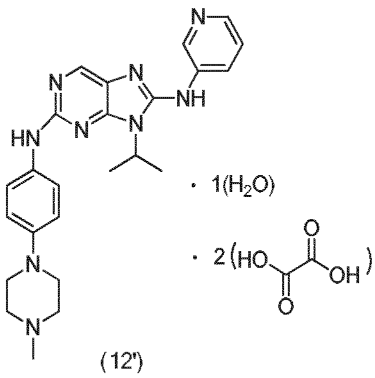

(12') ,

more preferably, the oxalate represented by Formula 11 or Formula 11' has an X-ray powder diffraction pattern comprising peaks at 2θ values of 8.1±0.2°, 8.4±0.2°, 9.0±0.2°, 14.1±0.2°, 16.7±0.2°, 25.6±0.2° as measured with CuKα radiation; further more preferably, the oxalate represented by Formula 11 or Formula 11' has an X-ray powder diffraction pattern substantially as shown in Figure 11, as measured with CuKα radiation;
or more preferably, the oxalate represented by Formula 12 or Formula 12' has an X-ray powder diffraction pattern comprising peaks at 2θ values of 7.1±0.2°, 12.2±0.2°, 14.2±0.2°, 16.4±0.2°, 17.7±0.2°, 19.0±0.2°, 24.4±0.2° as measured with CuKα radiation; further more preferably, the oxalate represented by Formula 12 or Formula 12' has an X-ray powder diffraction pattern substantially as shown in Figure 12, as measured with CuKα radiation.

**8.** The salt of the arylaminopurine derivative according to claim 1, wherein the salt is a succinate represented by Formula 13, or Formula 14:

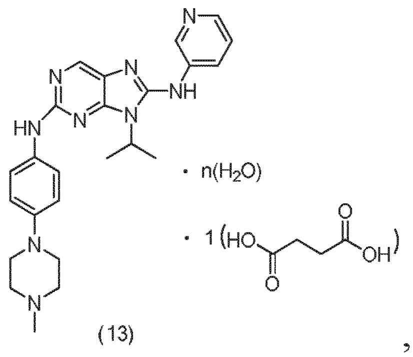

(13) ,

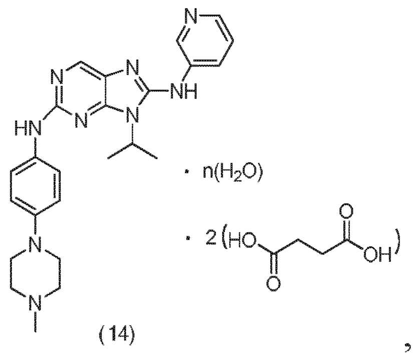

(14) ,

n is 0, 0.5, 1, 1.5, 2, 2.5, 3, 3.5, 4, 4.5, or 5;
preferably, the salt is a succinate represented by Formula 13', or Formula 14':

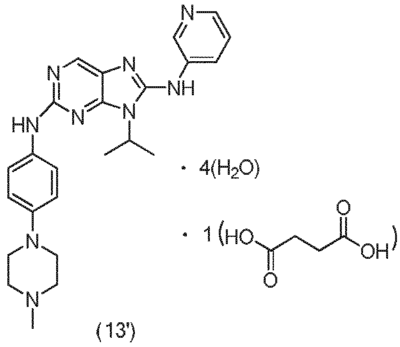

(13') ,

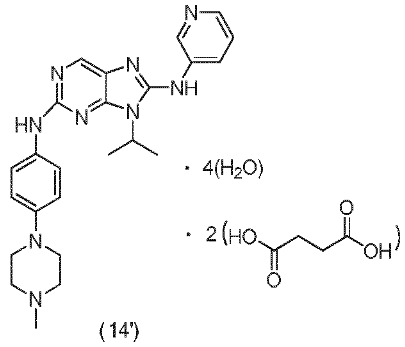

(14') ,

more preferably, the succinate represented by Formula 13 or Formula 13' has an X-ray powder diffraction pattern comprising peaks at 2θ values of 7.0+0.2°, 9.1+0.2°, 18.5+0.2°, 20.4±0.2°, 21.0+0.2°, 22.4±0.2°, 27.1±0.2° as measured with CuKα radiation; further more preferably, the succinate represented by Formula 13 or Formula 13' has an X-ray powder diffraction pattern substantially as shown in Figure 13, as measured with CuKα radiation;

or more preferably, the succinate represented by Formula 14 or Formula 14' has an X-ray powder diffraction pattern comprising peaks at 2θ values of 7.0+0.2°, 9.2+0.2°, 17.6+0.2°, 18.4+0.2°, 19.7+0.2°, 25.8±0.2°, 27.3±0.2° as measured with CuKα radiation; further more preferably, the succinate represented by Formula 14 or Formula 14' has an X-ray powder diffraction pattern substantially as shown in Figure 14, as measured with CuKα radiation.

**9.** The salt of the arylaminopurine derivative according to claim 1, wherein the salt is an acetate represented by Formula 15, or Formula 16:

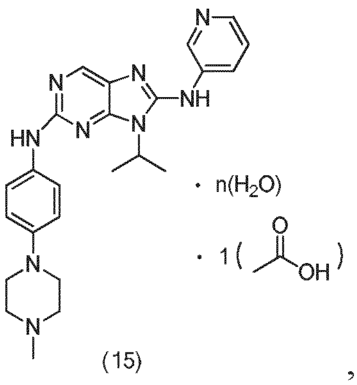
(15) ,

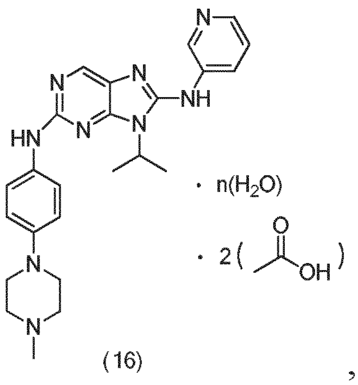
(16) ,

n is 0, 0.5, 1, 1.5, 2, 2.5, 3, 3.5, 4, 4.5, or 5;

preferably, the salt is an acetate represented by Formula 15', or Formula 16':

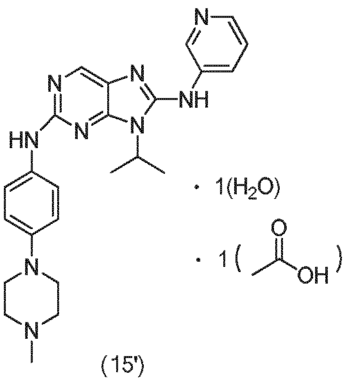
(15') ,

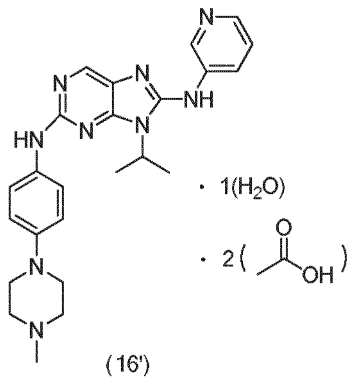
(16') ,

more preferably, the acetate represented by Formula 15 or Formula 15' has an X-ray powder diffraction pattern comprising peaks at 2θ values of 10.9±0.2°, 12.6±0.2°, 15.1±0.2°, 17.8±0.2°, 19.2±0.2°, 19.6±0.2°, 21.0±0.2°, 21.8±0.2°, 22.3±0.2°, 24.6±0.2°, 25.4±0.2° as measured with CuKα radiation; further more preferably, the acetate represented by Formula 15 or Formula 15' has an X-ray powder diffraction pattern substantially as shown in Figure 15, as measured with CuKα radiation;

or more preferably, the acetate represented by Formula 16 or Formula 16' has an X-ray powder diffraction pattern comprising peaks at 2θ values of 6.2±0.2°, 12.2±0.2°, 17.5±0.2°, 21.5±0.2°, 23.4±0.2°, 24.8±0.2° as measured with CuKα radiation; further more preferably, the acetate represented by Formula 16 or Formula 16' has an X-ray powder diffraction pattern substantially as shown in Figure 16, as measured with CuKα radiation.

**10.** The salt of the arylaminopurine derivative according to claim 1, wherein the salt is a sulfate represented by Formula 17, or Formula 18:

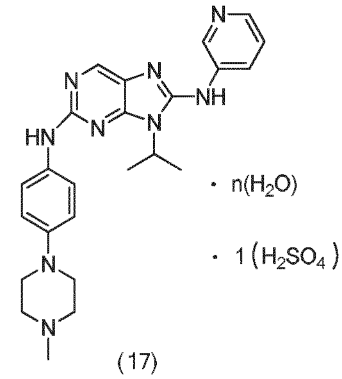
(17) ,
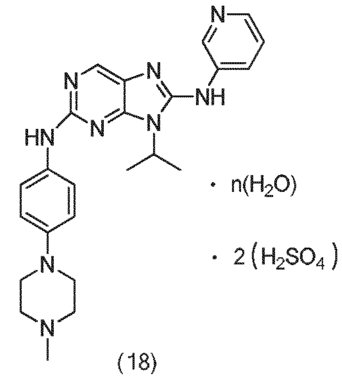
(18) ,

n is 0, 0.5, 1, 1.5, 2, 2.5, 3, 3.5, 4, 4.5, or 5;
preferably, the salt is a sulfate represented by Formula 17', or Formula 18':

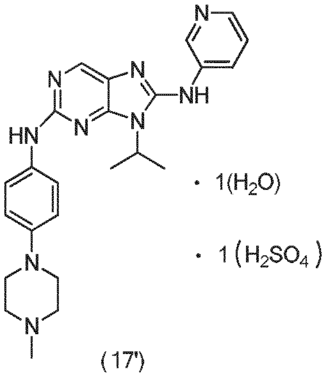
(17') ,
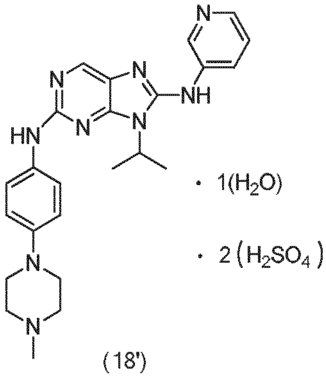
(18') ,

more preferably, the sulfate represented by Formula 17 or Formula 17' has an X-ray powder diffraction pattern comprising peaks at 2θ values of 4.8±0.2°, 7.0+0.2°, 8.6±0.2°, 9.2+0.2°, 9.5+0.2°, 11.6±0.2°, 12.8+0.2°, 13.6+0.2°, 15.7+0.2°, 17.6+0.2°, 18.6+0.2°, 20.5±0.2°, 21.6±0.2°, 23.8±0.2°, 25.7±0.2°as measured with CuKα radiation; further more preferably, the sulfate represented by Formula 17 or Formula 17' has an X-ray powder diffraction pattern substantially as shown in Figure 17, as measured with CuKα radiation;
or more preferably, the sulfate represented by Formula 18 or Formula 18' has an X-ray powder diffraction pattern comprising peaks at 2θ values of 8.6±0.2°, 9.6±0.2°, 15.7±0.2°, 17.1±0.2°, 19.3±0.2°, 20.0±0.2°, 26.6±0.2° as measured with CuKα radiation; further more preferably, the sulfate represented by Formula 18 or Formula 18' has an X-ray powder diffraction pattern substantially as shown in Figure 18, as measured with CuKα radiation.

**11.** A pharmaceutical composition, comprising the salt represented by Formula 2 of the arylaminopurine derivative according to any of claims 1-10.

**12.** Use of the salt represented by Formula 2 of the arylaminopurine derivative according to any of claims 1-10 or the pharmaceutical composition according to claim 11 in manufacture of a medicament as the protein kinase inhibitor, wherein the kinase is selected from FLT3, EGFR, Abl, Fyn, Hck, Lck, Lyn, Ret, Yes, VEGFR2, ALK, BTK, c-KIT, c-SRC, FGFR1, KDR, MET and PDGFRα;
preferably, the medicament as the protein kinase inhibitor is an antitumor drug, the tumor is selected from non-small cell lung cancer, acute myeloid leukemia, chronic myelocytic leukemia, chronic myeloid leukemia, squamous cell carcinoma, mammary cancer, colorectal cancer, liver cancer, stomach cancer, and malignant melanoma, more preferably leukemia or lung cancer, further more preferably acute myeloid leukemia or non-small cell lung cancer, further preferably FLT3 mutation-positive acute myeloid leukemia (such as FLT3-ITD acute myeloid leukemia), Ph-positive chronic myeloid leukemia, or non-small cell lung cancer with EGFR activating mutations.

**13.** A method for preparing the salt represented by Formula 2 of the arylaminopurine derivative according to claim 1, which comprises a reaction of an arylaminopurine derivative represented by Formula 1 and an acid is performed in the presence of water and an organic solvent to obtain the salt represented by Formula 2 of the arylaminopurine

derivative:

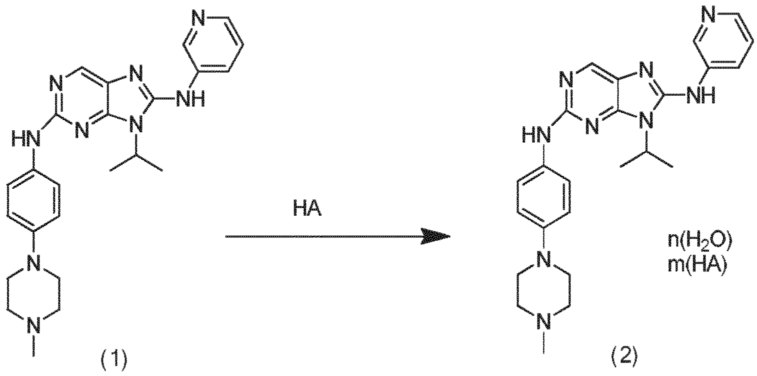

(1)

(2)

,

wherein,

HA is an acid;
$H_2O$ is the water of crystallization;
m is an integer or half-integer from 1 to 4;
n is an integer or half-integer from 0 to 5.

**14.** The method for preparing the salt of the arylaminopurine derivative according to claim 13, wherein the molar ratio of the arylaminopurine derivative represented by Formula 1 to the acid is 1:1 to 1:4, preferably 1:1.2 to 1:3.5;

the reaction temperature is 0-70°C, preferably 35-45°C;
the reaction is performed in the presence of the combination of water and one or more organic solvents selected from alcohols, ethers, esters, ketones, nitriles, and alkanes, preferably in the presence of $C_1$-$C_3$ lower alcohol and water, in the presence of a ketone and water, in the presence of a nitrile and water, or the presence of ether and water, and more preferably in the presence of methanol-water, ethanol-water, isopropanol-water, tetrahydrofuran-water, dioxane-water, acetone-water or acetonitrile-water; and the ratio of the use amounts by volume of the organic solvent to water is 1:10 to 10:1, for example, 1:1 to 10:1 or 1:10 to 1:1.

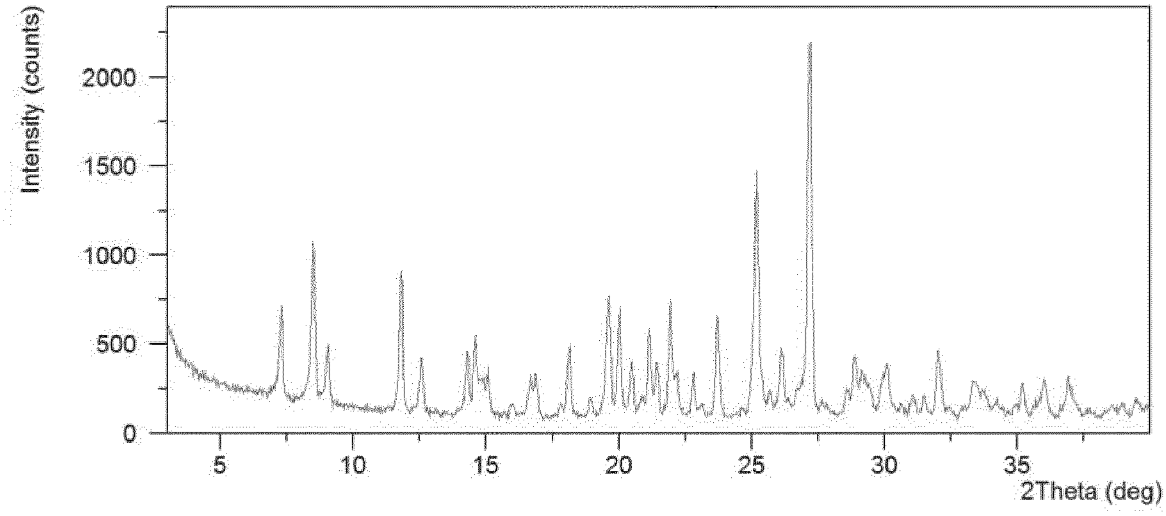


Figure 1

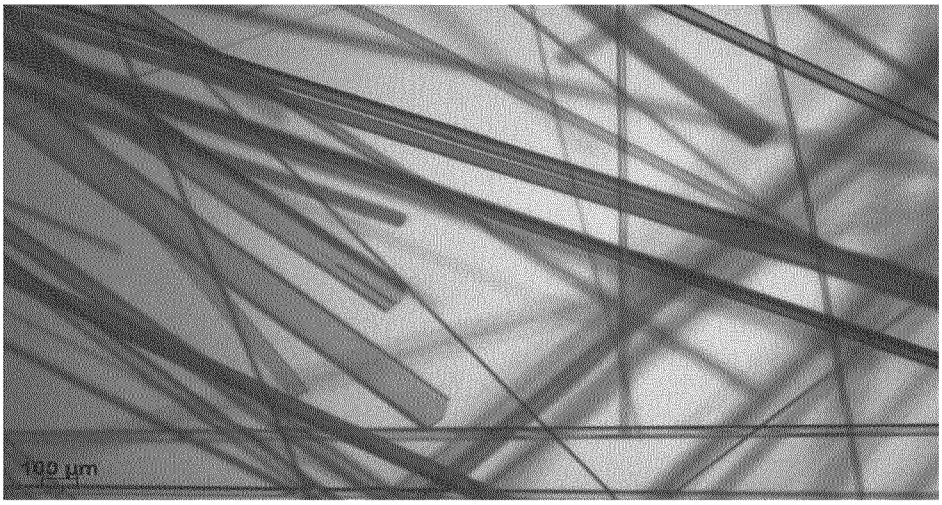


Figure 2

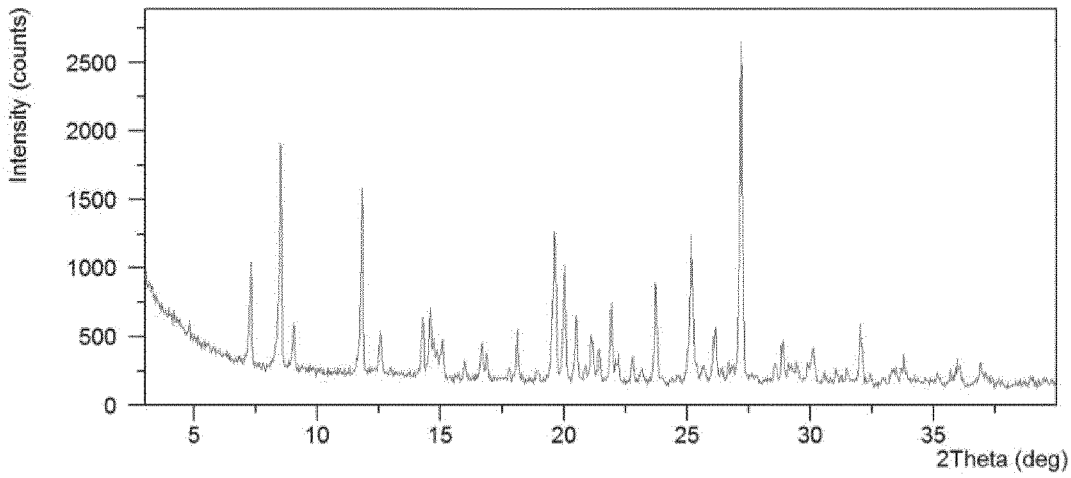


Figure 3

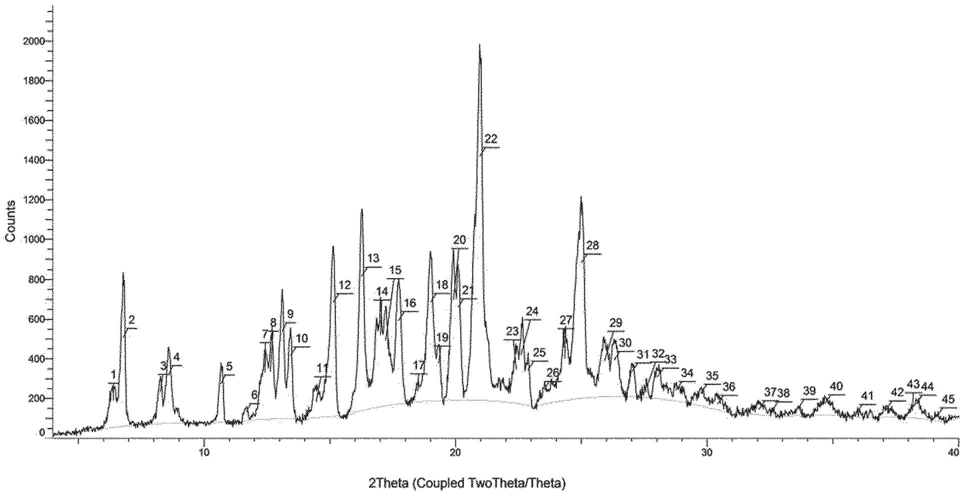


**Figure 4**

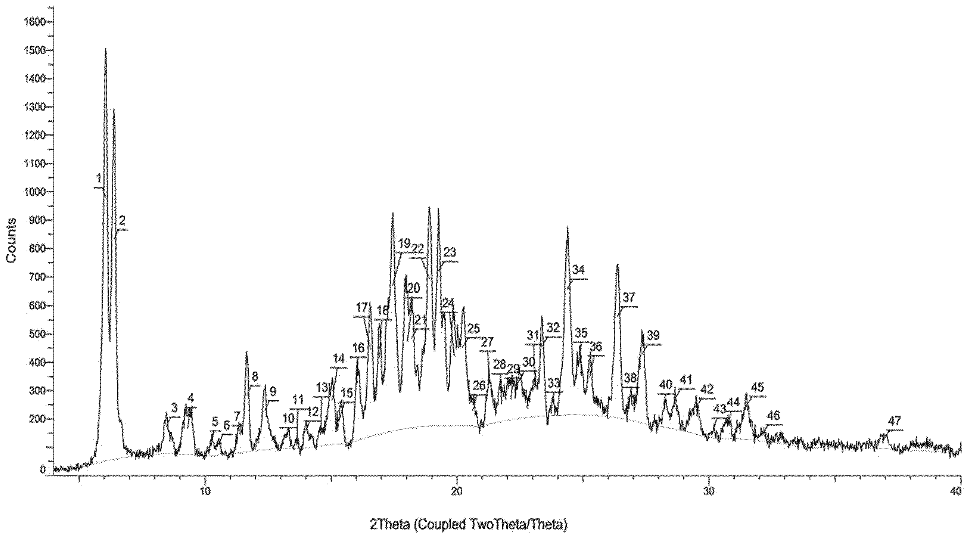


**Figure 5**

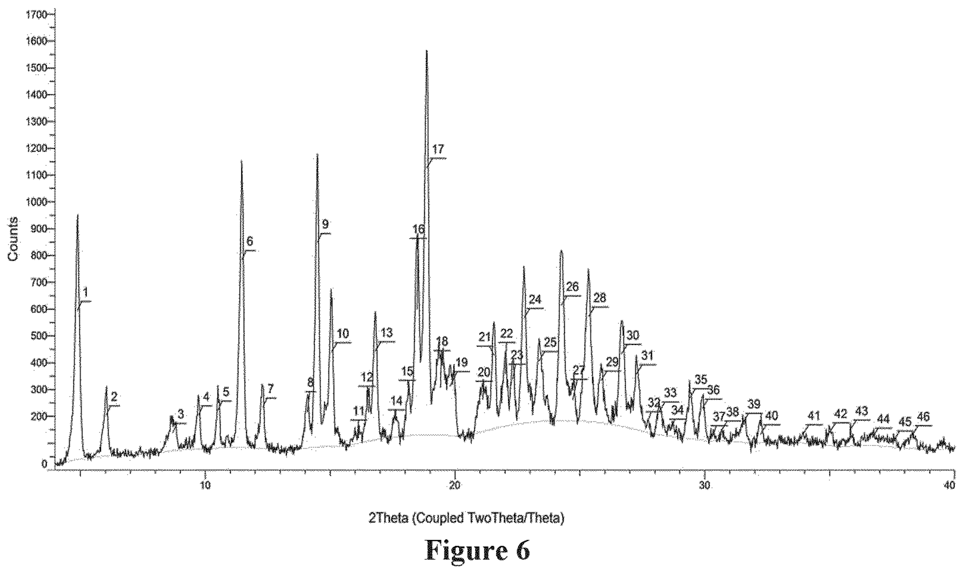


**Figure 6**

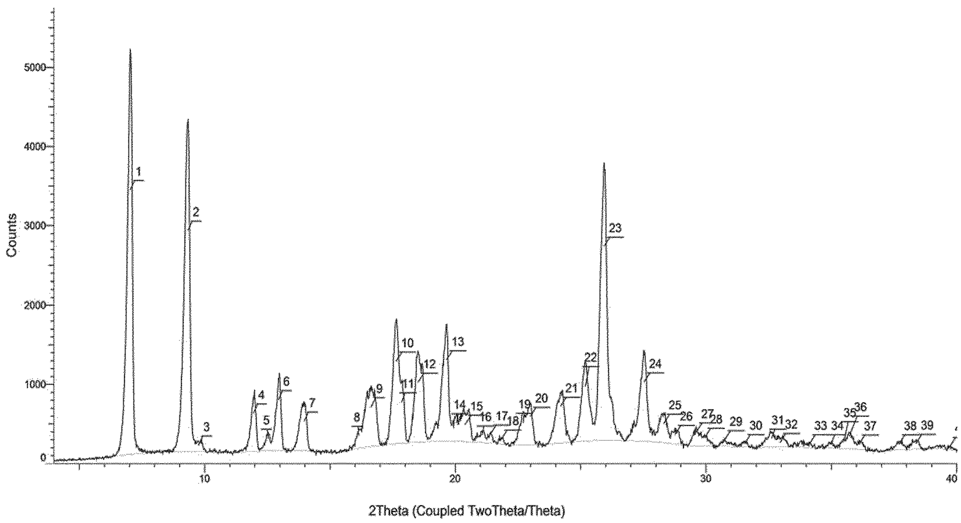


Figure 7

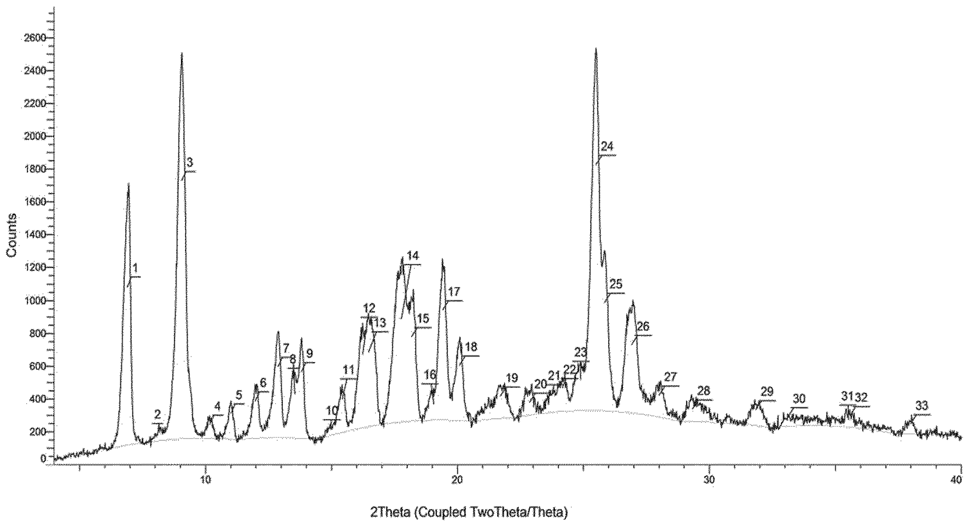


Figure 8

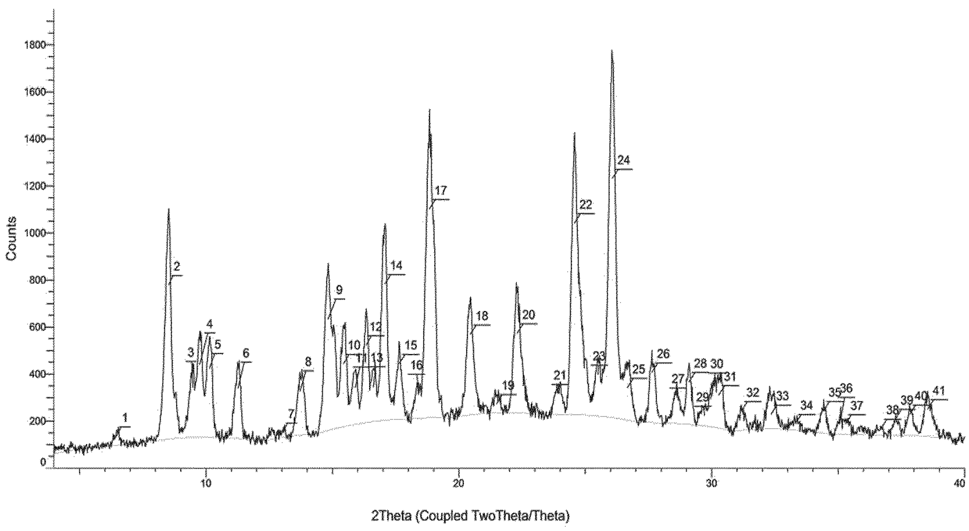


Figure 9

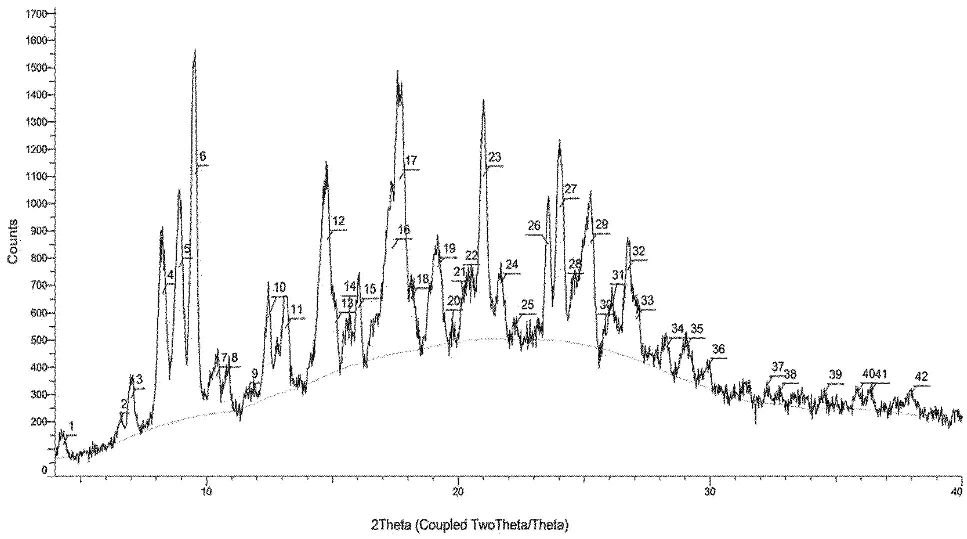


**Figure 10**

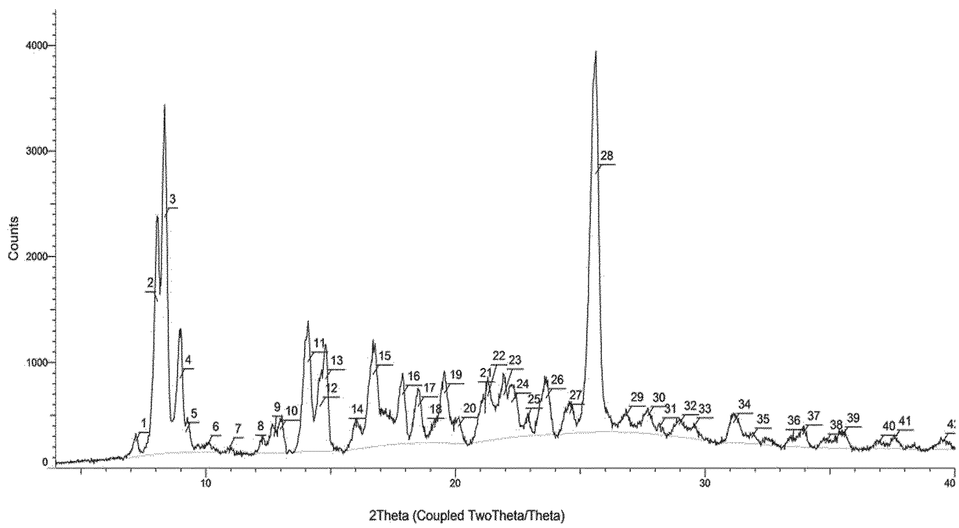


**Figure 11**

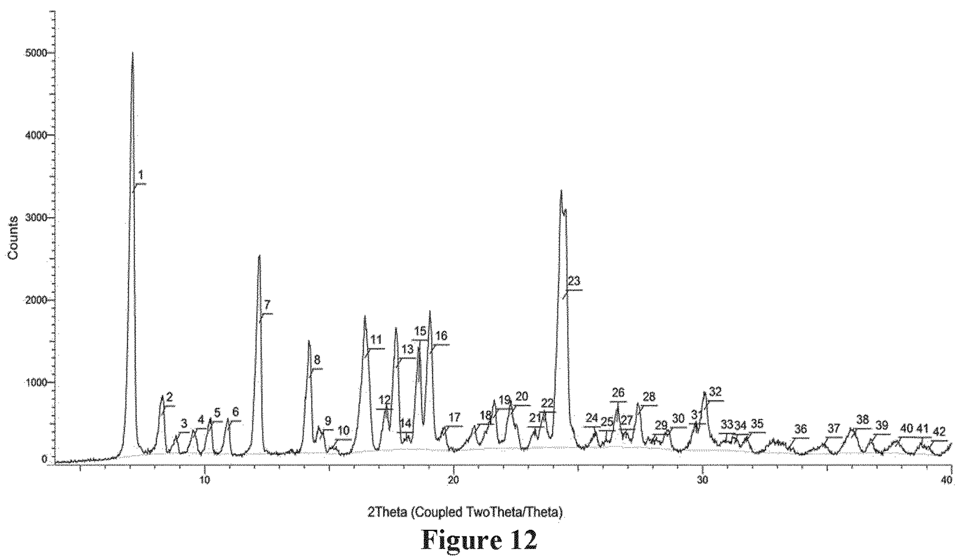


**Figure 12**

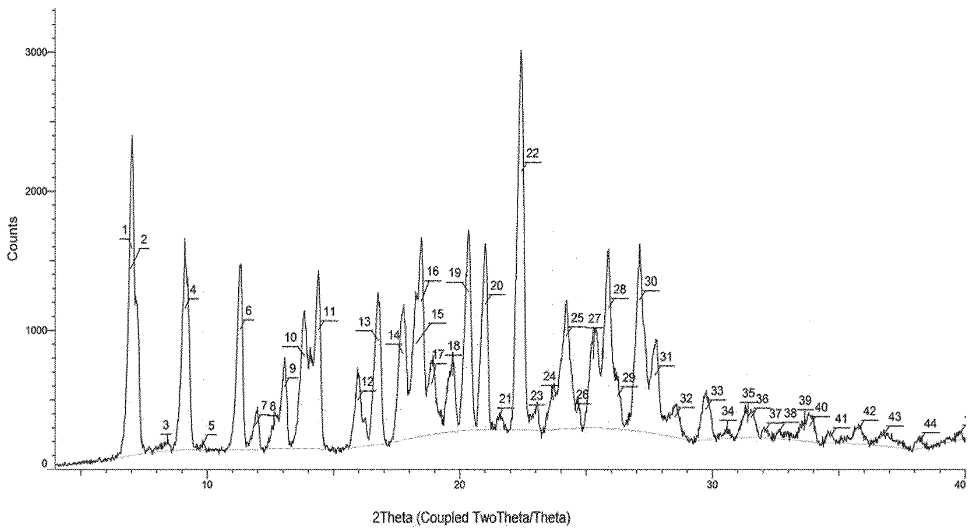


Figure 13

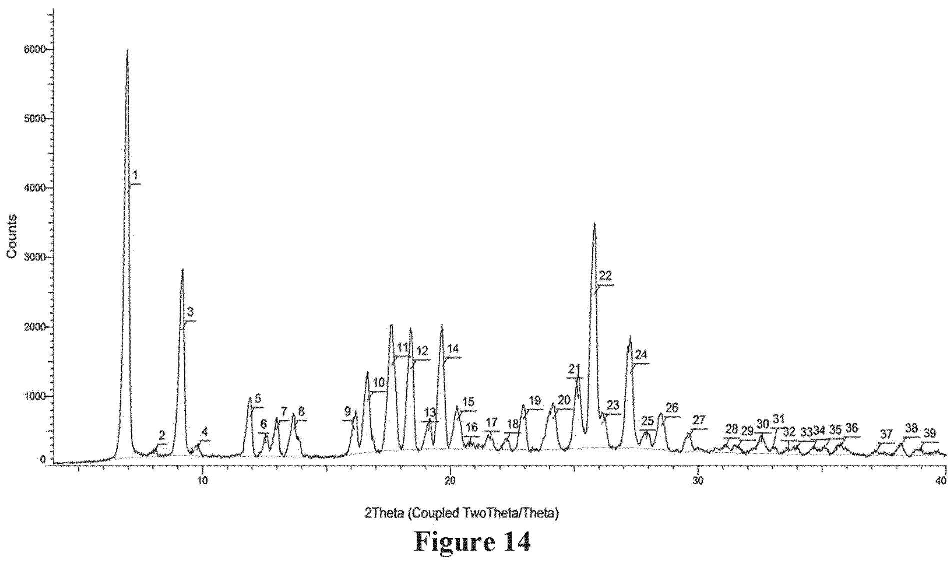


Figure 14

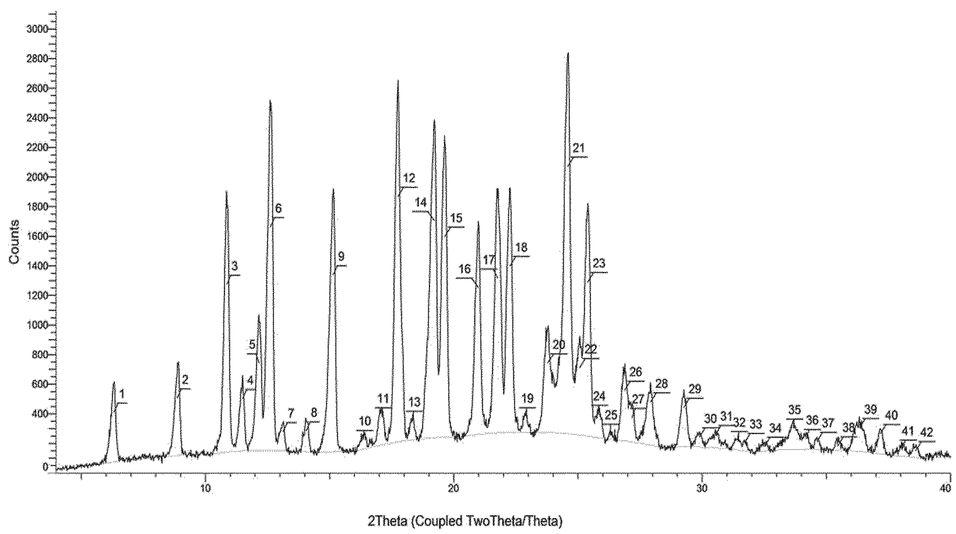


Figure 15

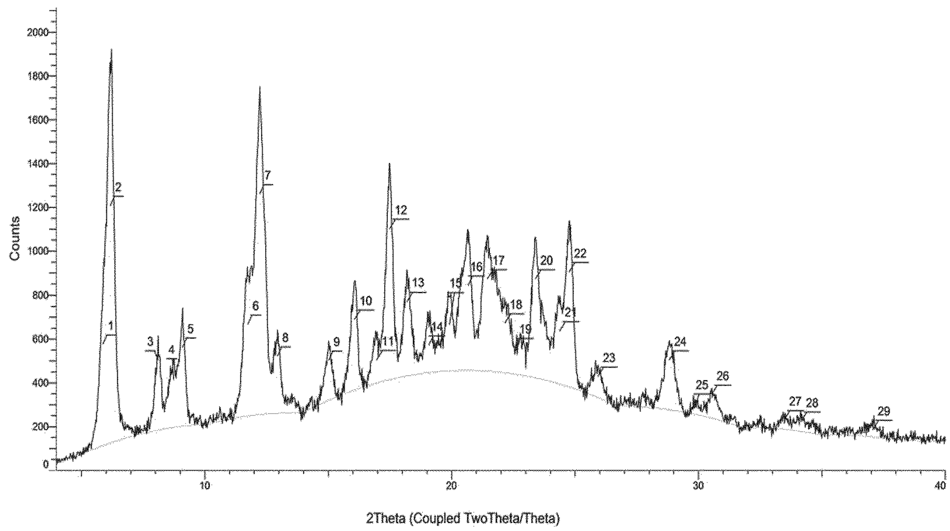


Figure 16

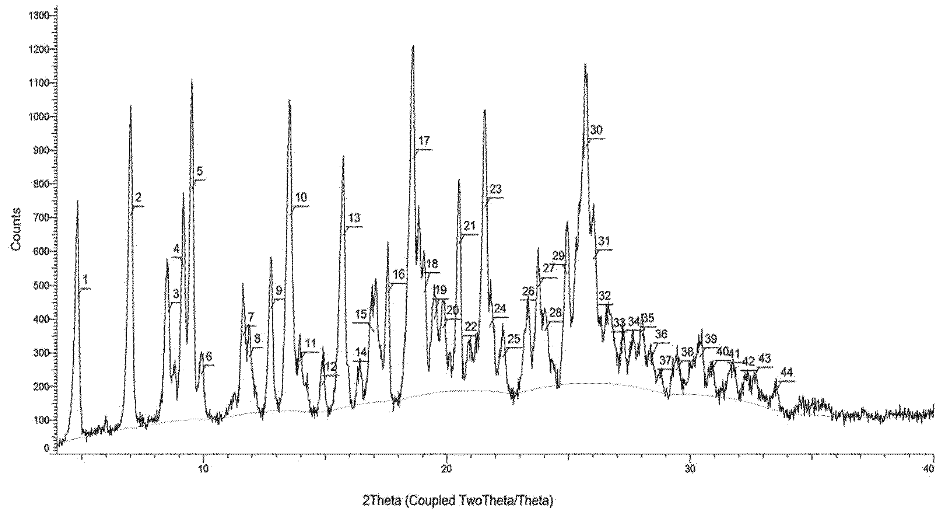


Figure 17

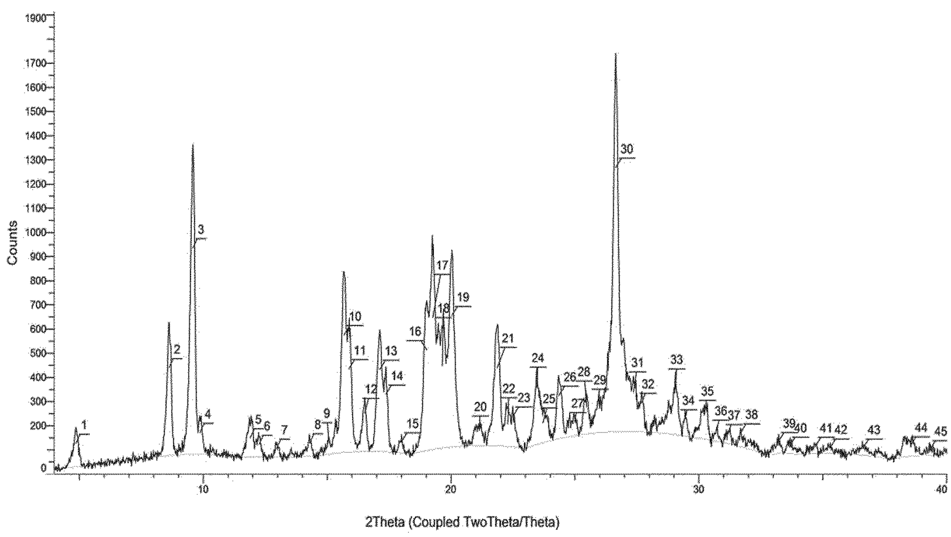


Figure 18

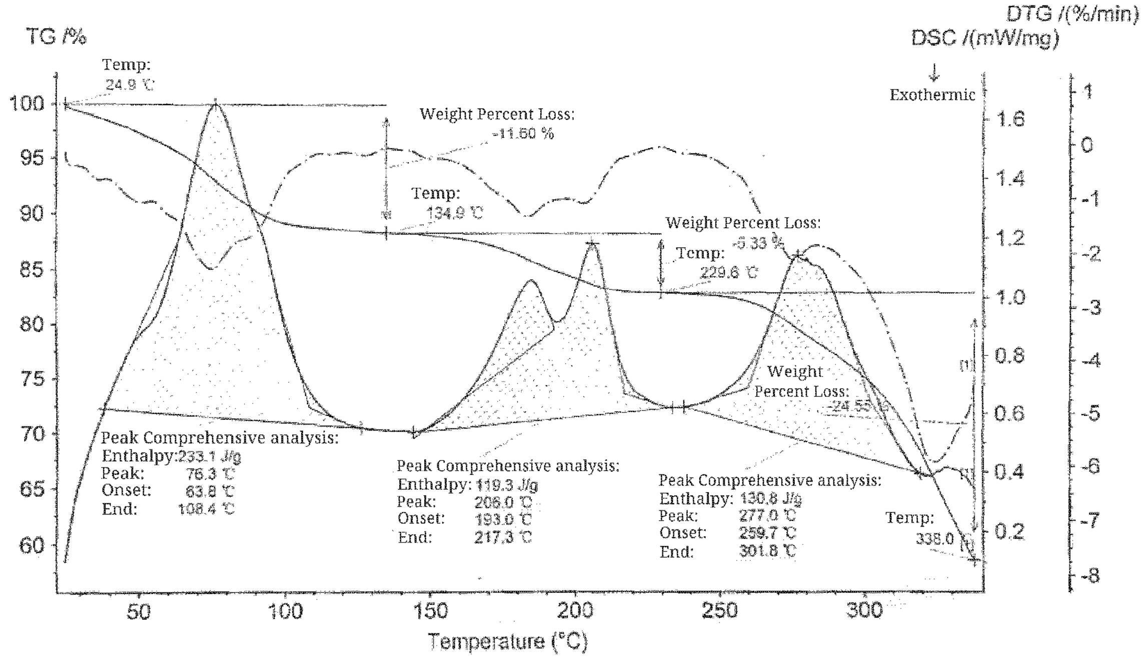


**Figure 19**

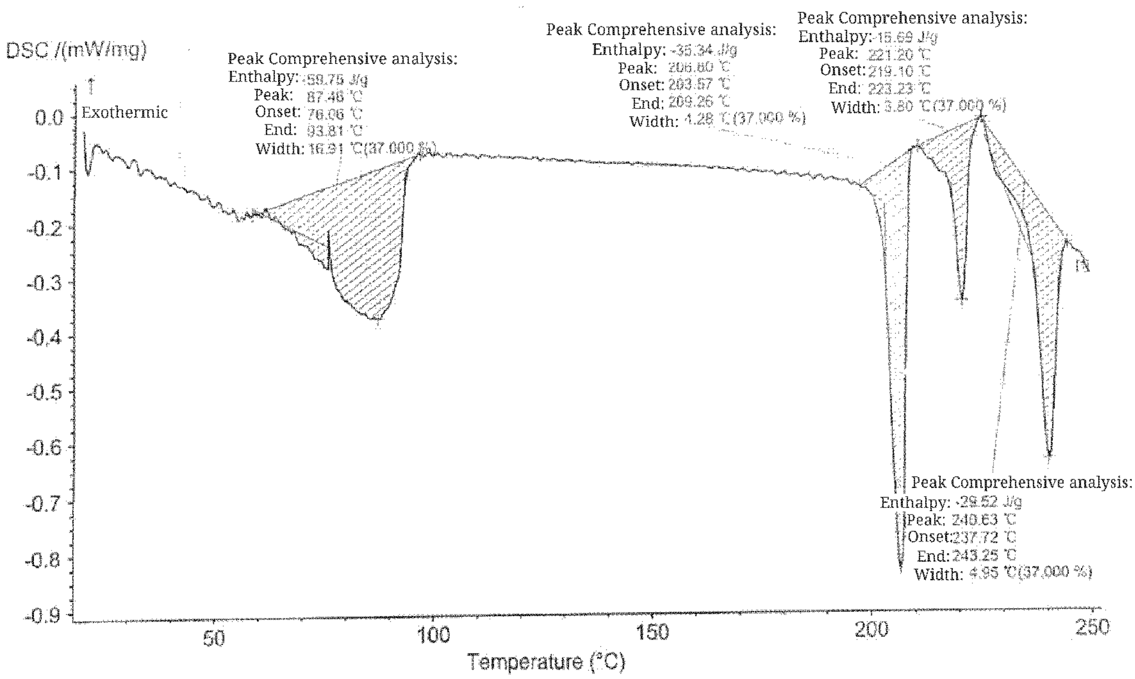


**Figure 20**

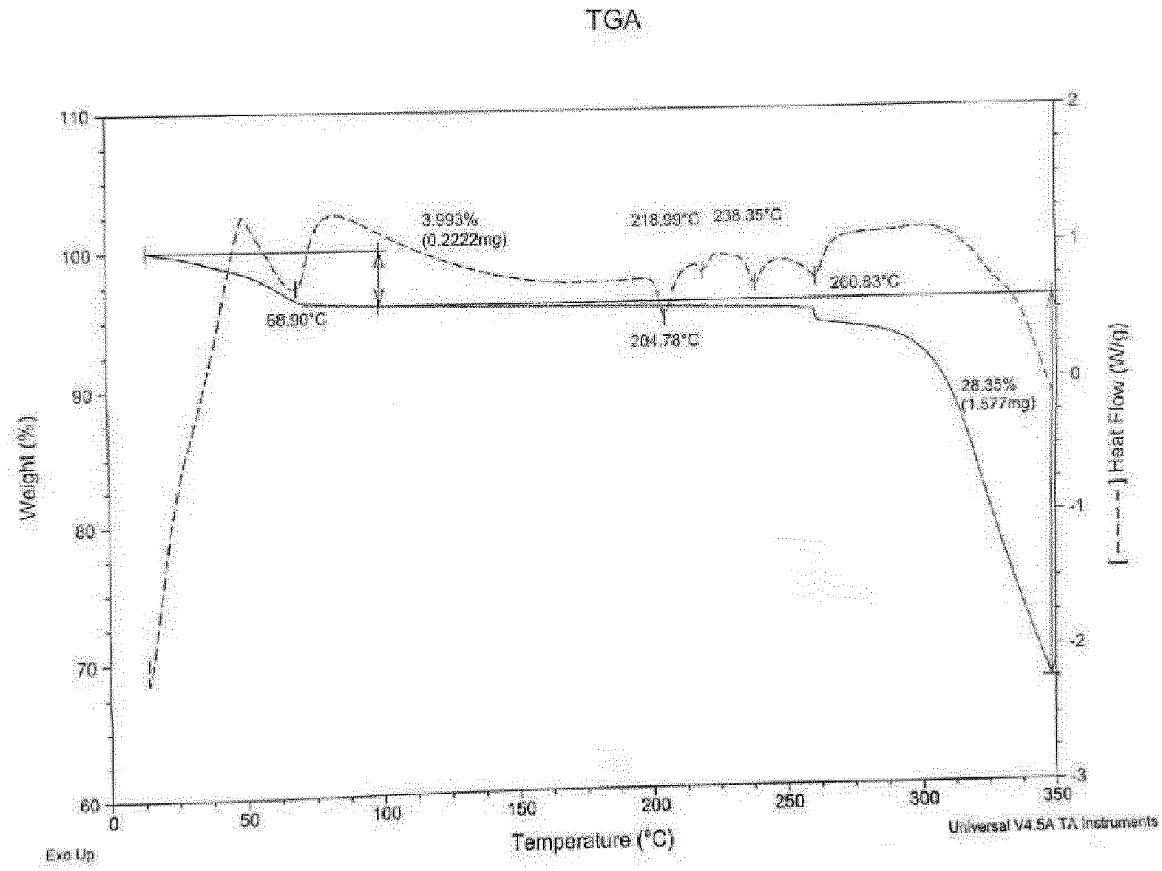


**Figure 21**

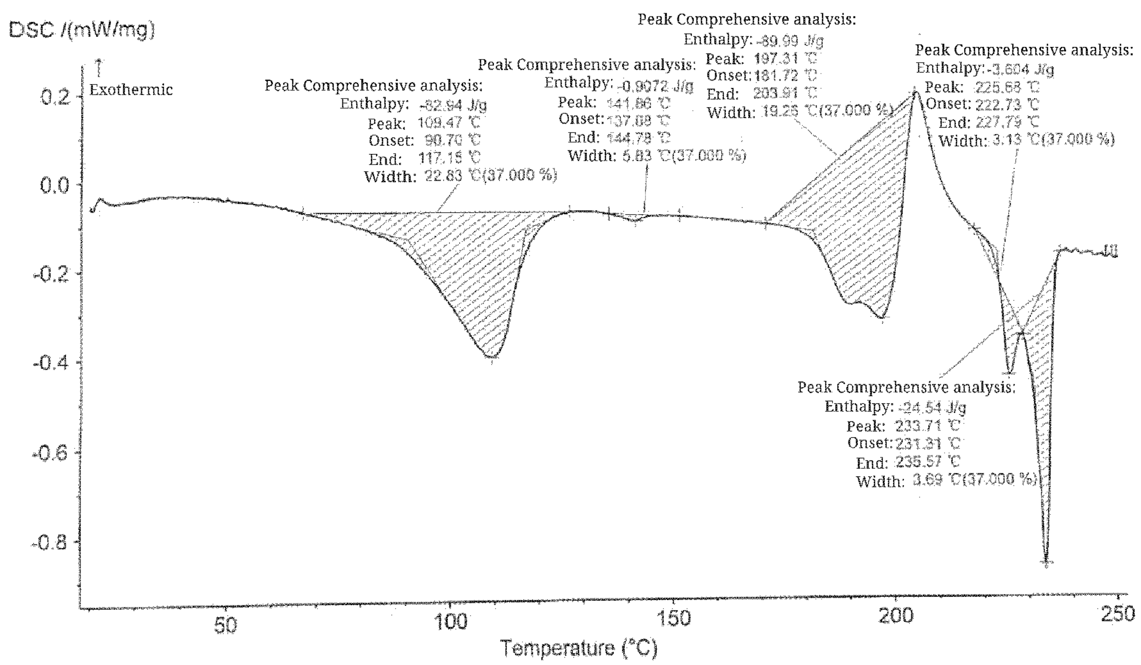


**Figure 22**

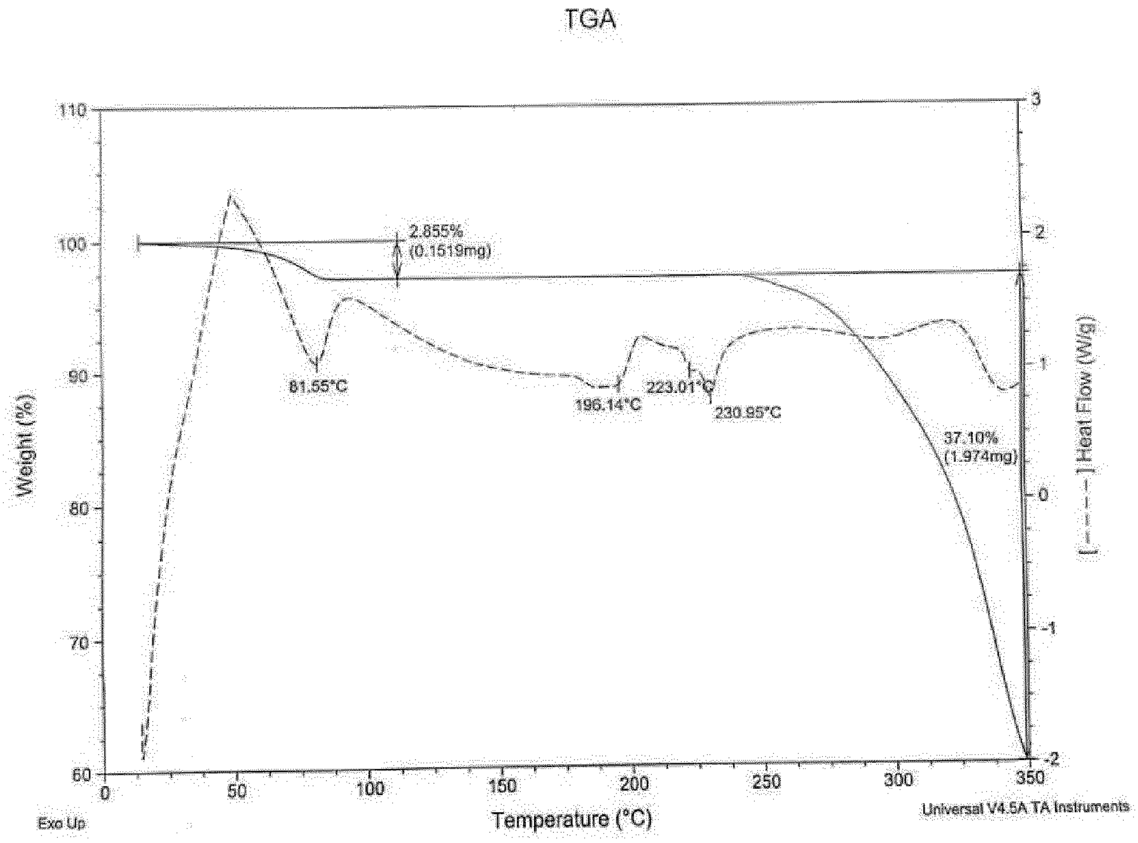


Figure 23

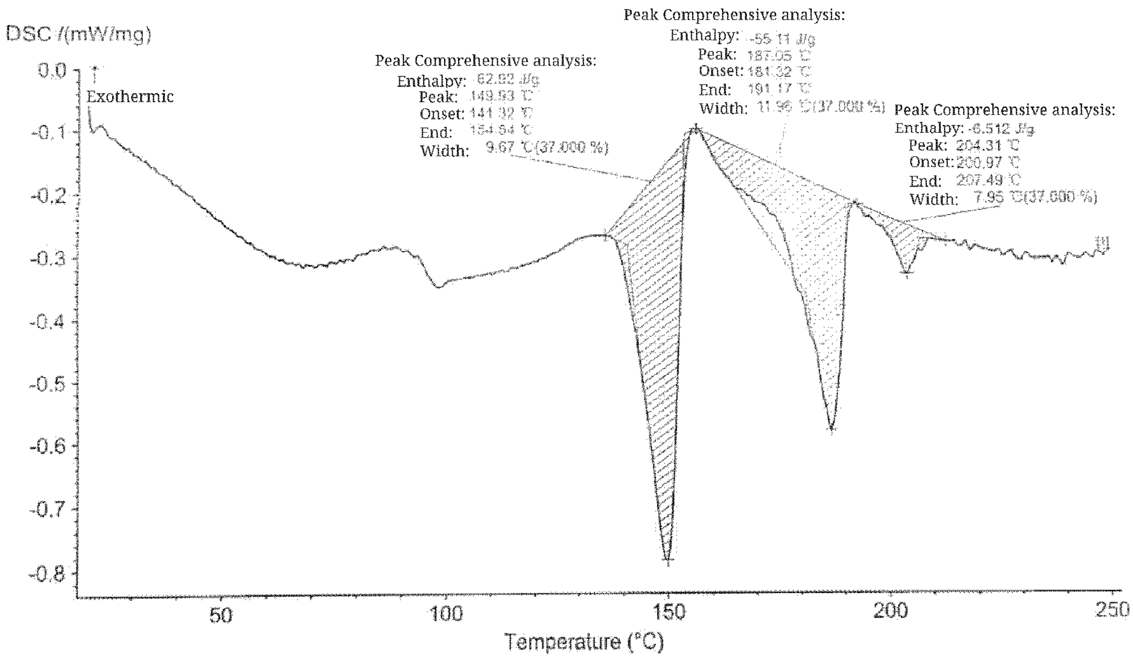


Figure 24

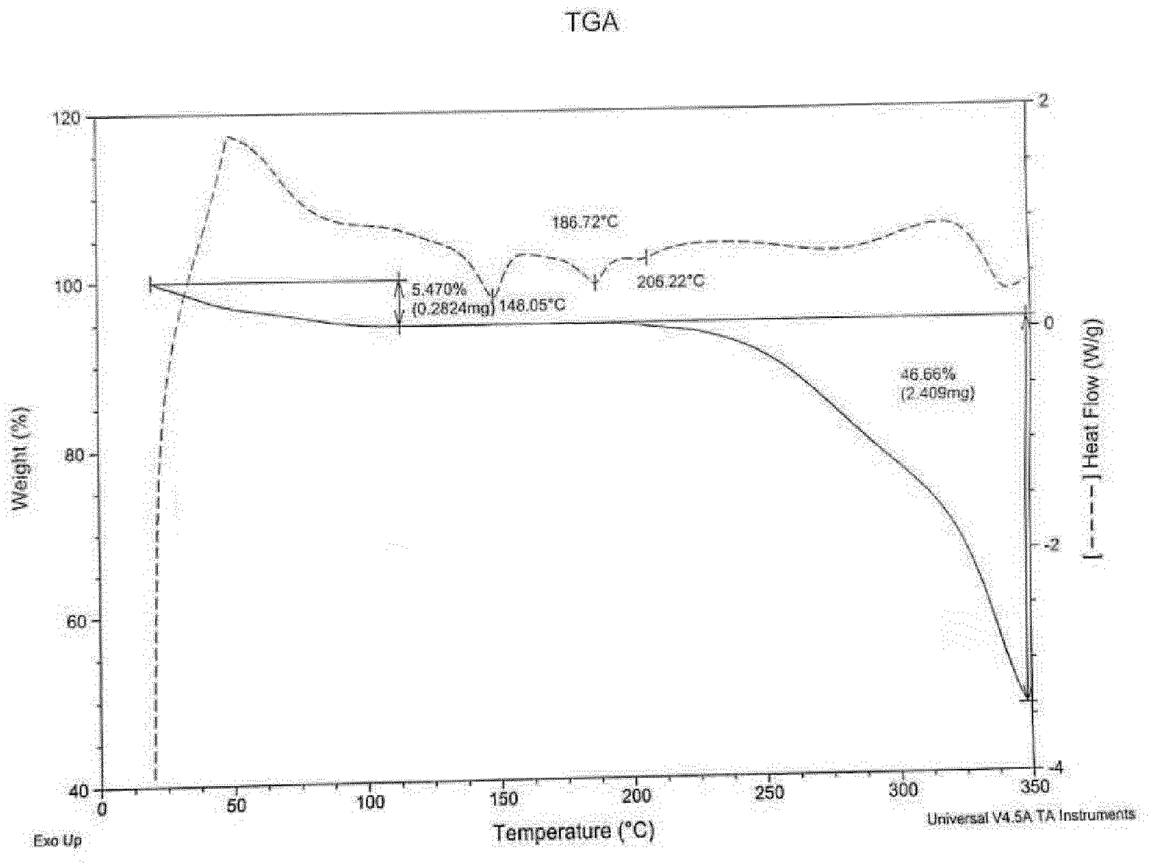


**Figure 25**

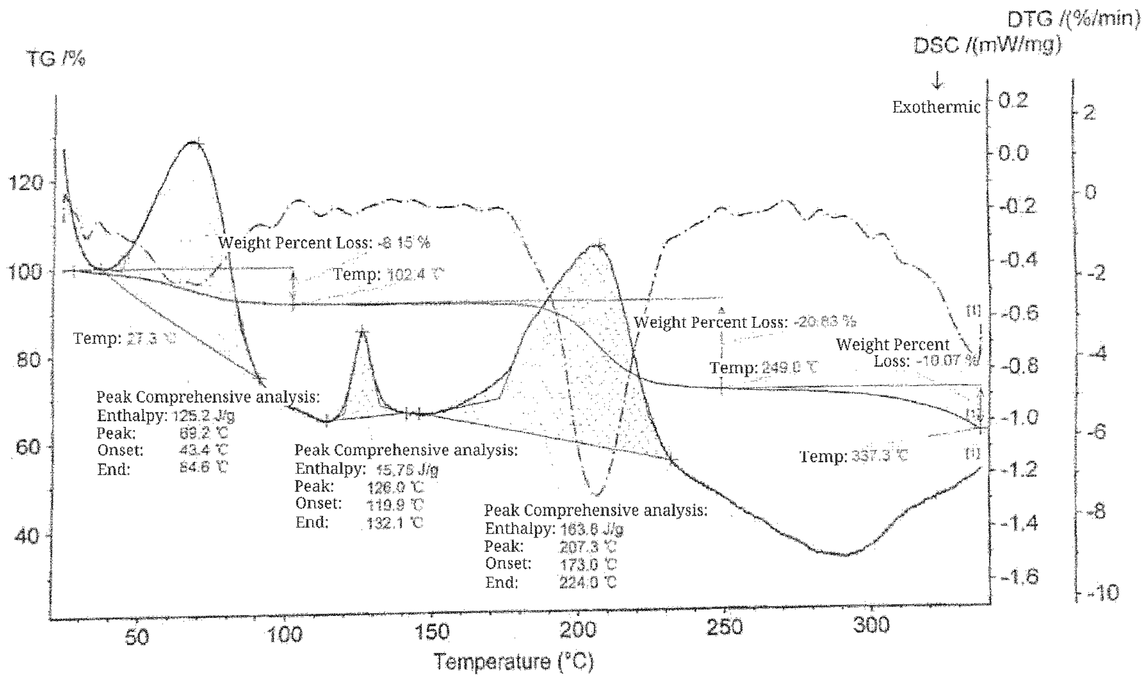


**Figure 26**

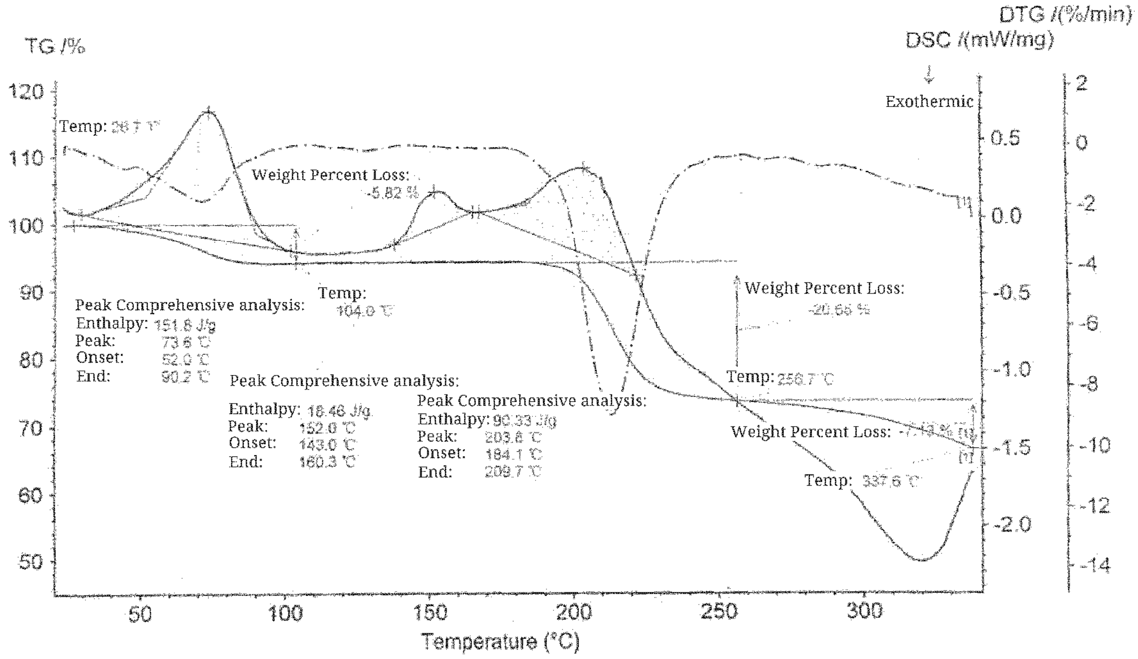


**Figure 27**

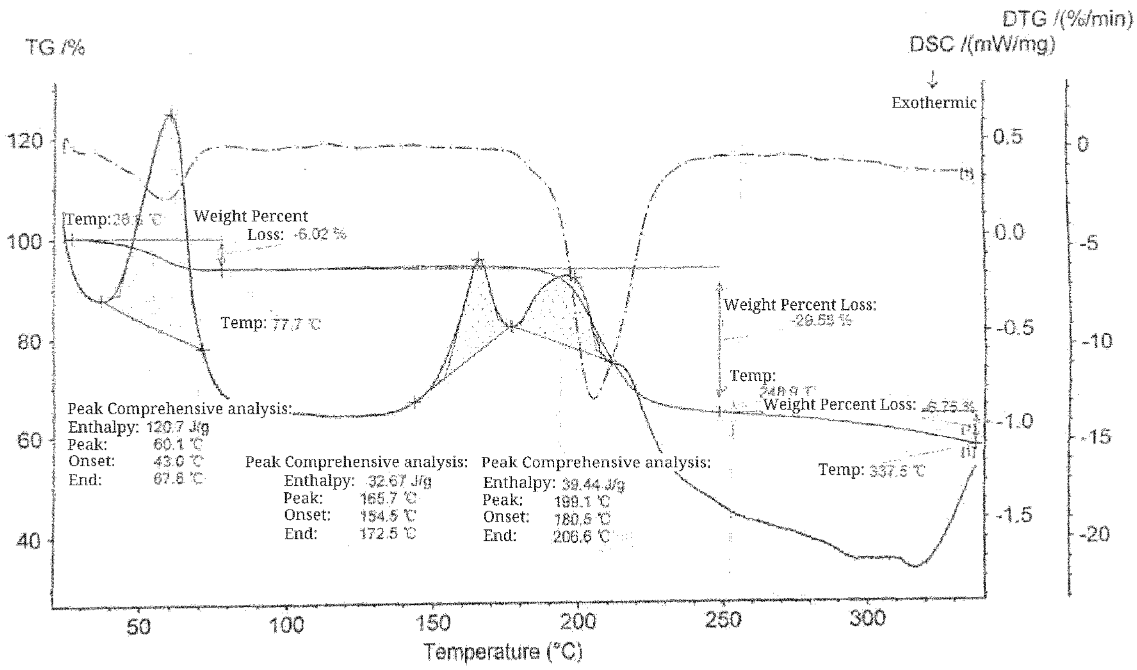


**Figure 28**

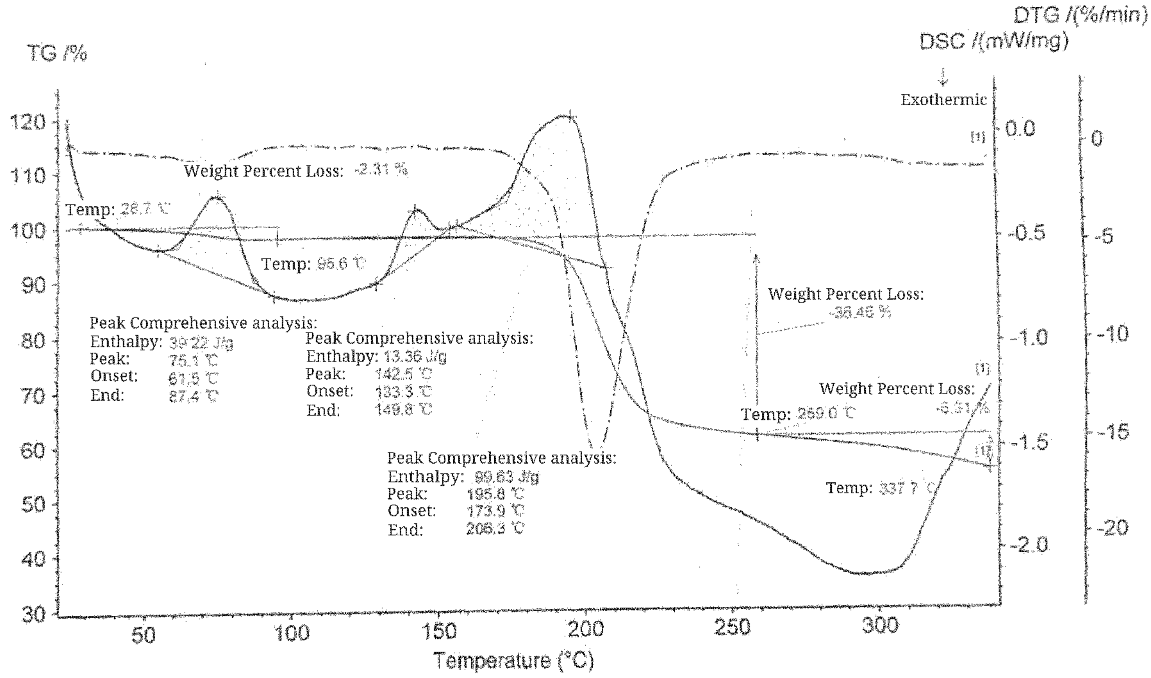


**Figure 29**

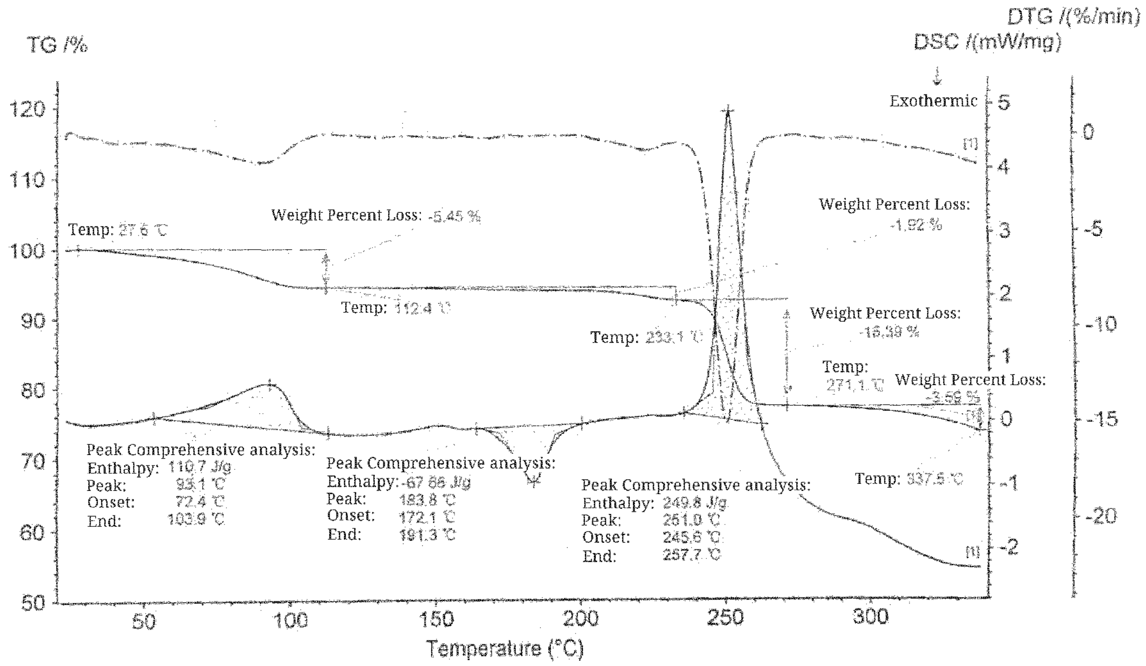


**Figure 30**

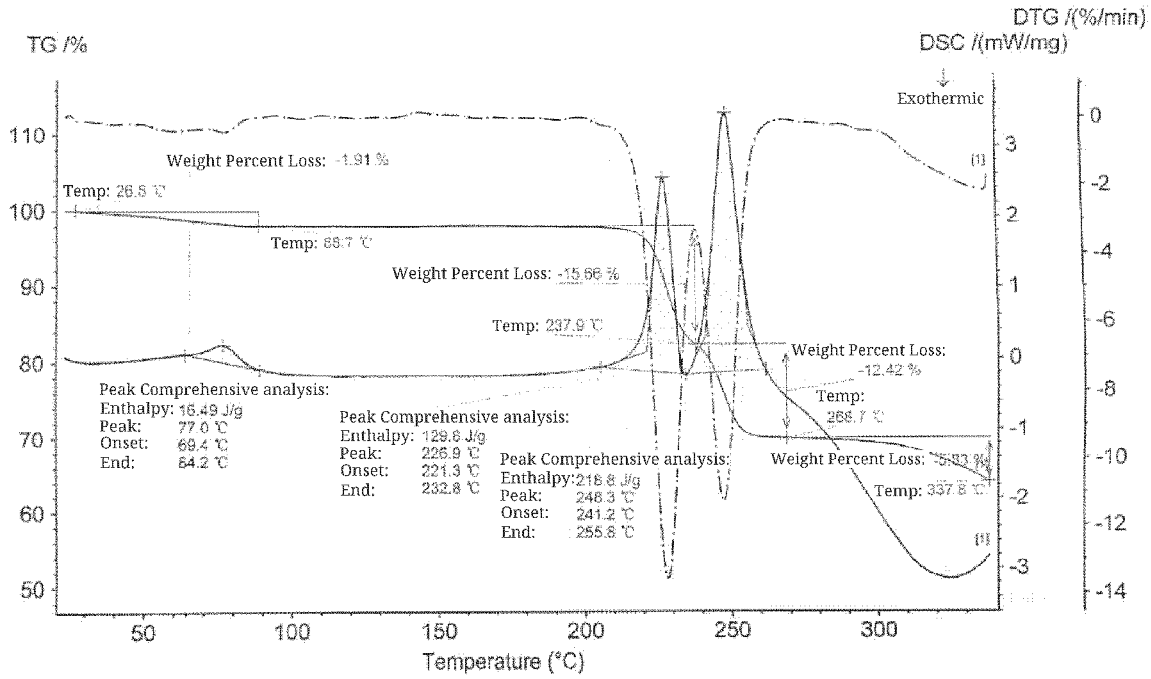


**Figure 31**

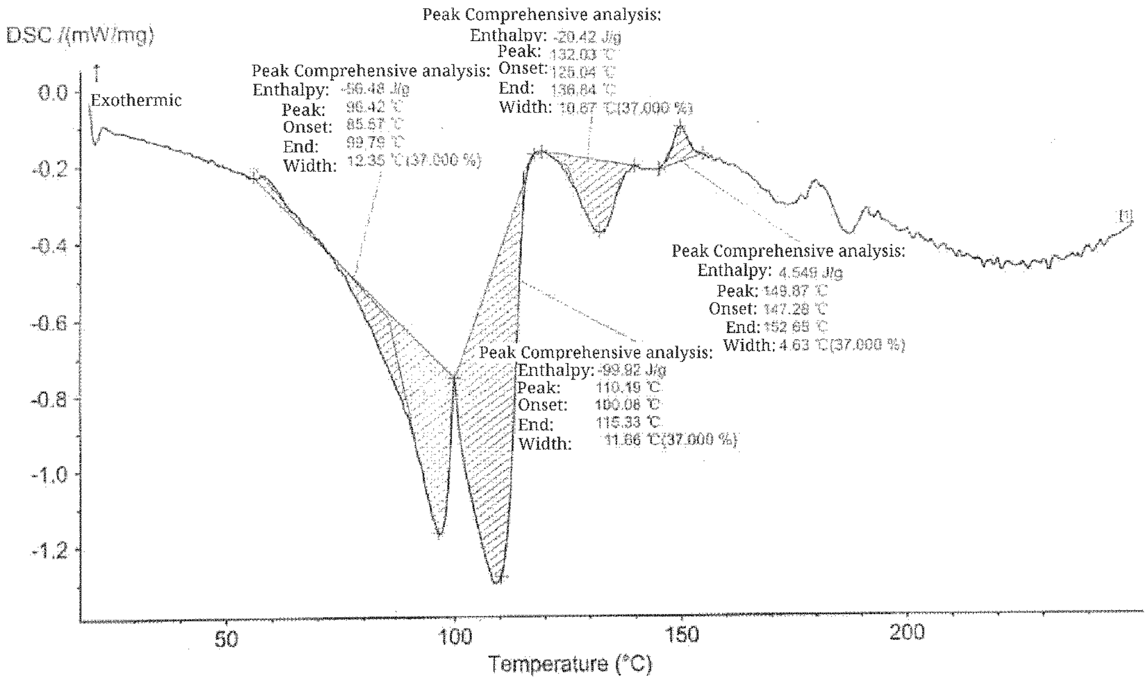


**Figure 32**

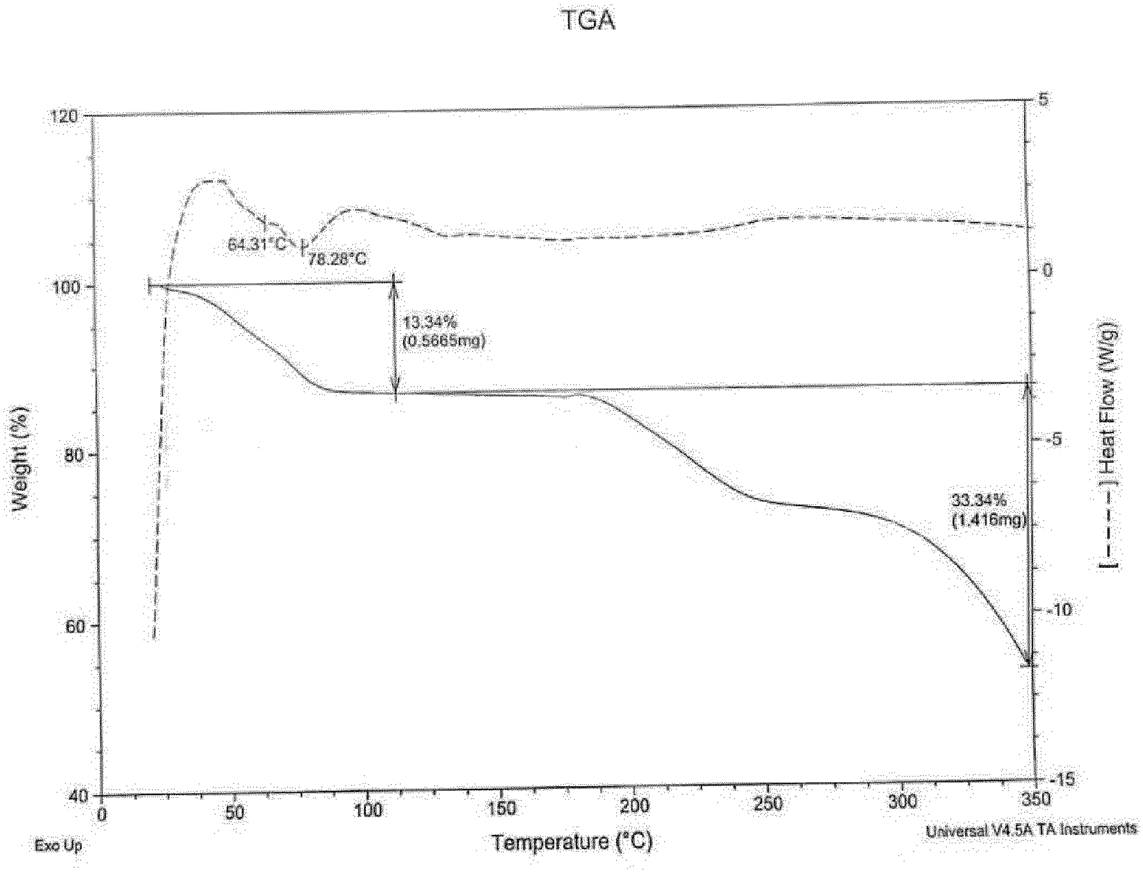


**Figure 33**

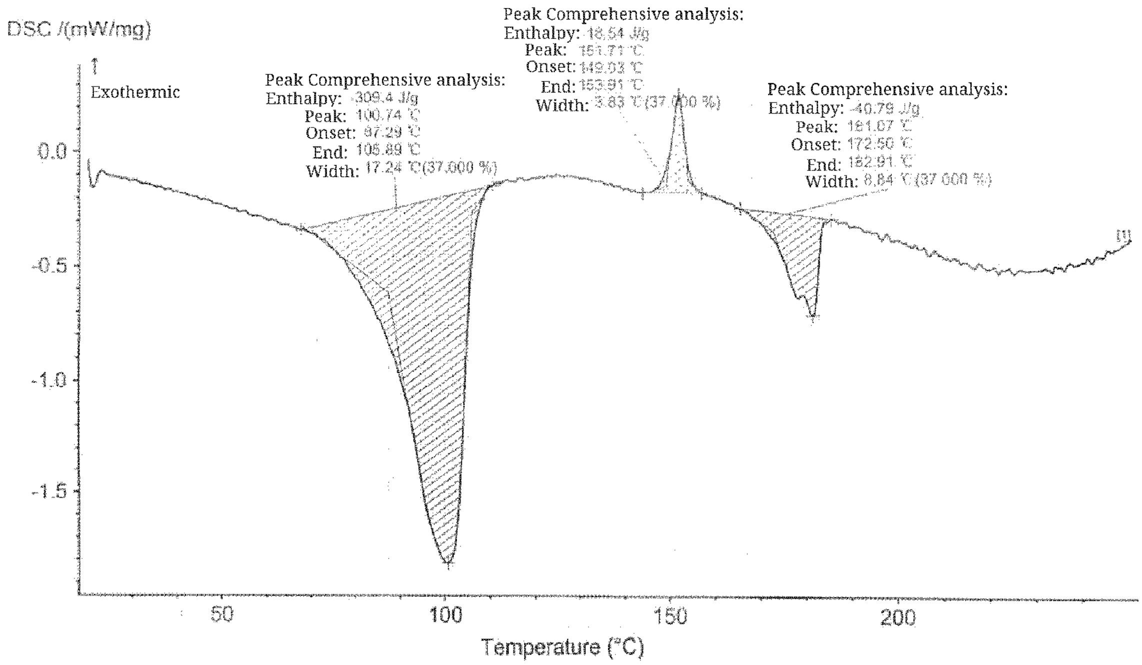


**Figure 34**

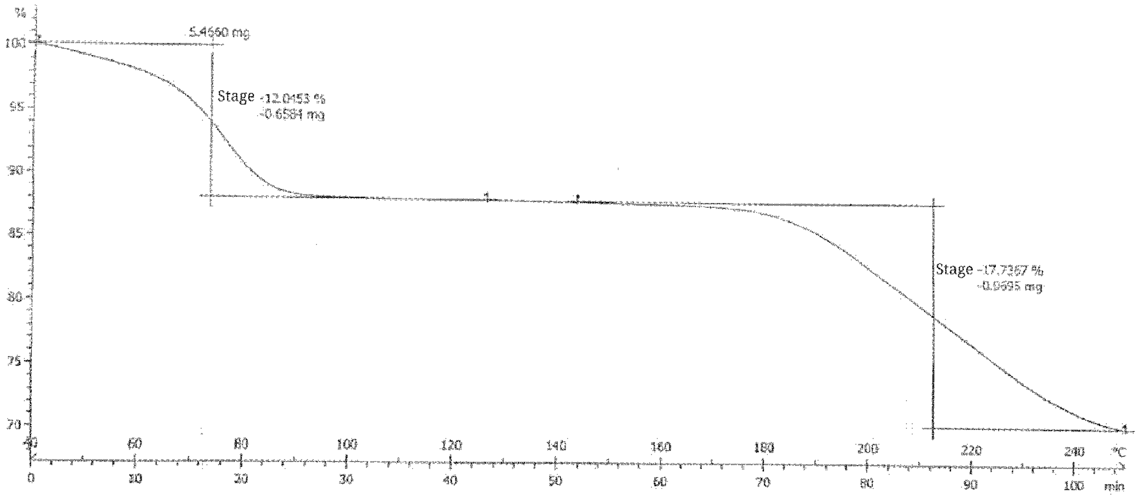


Figure 35

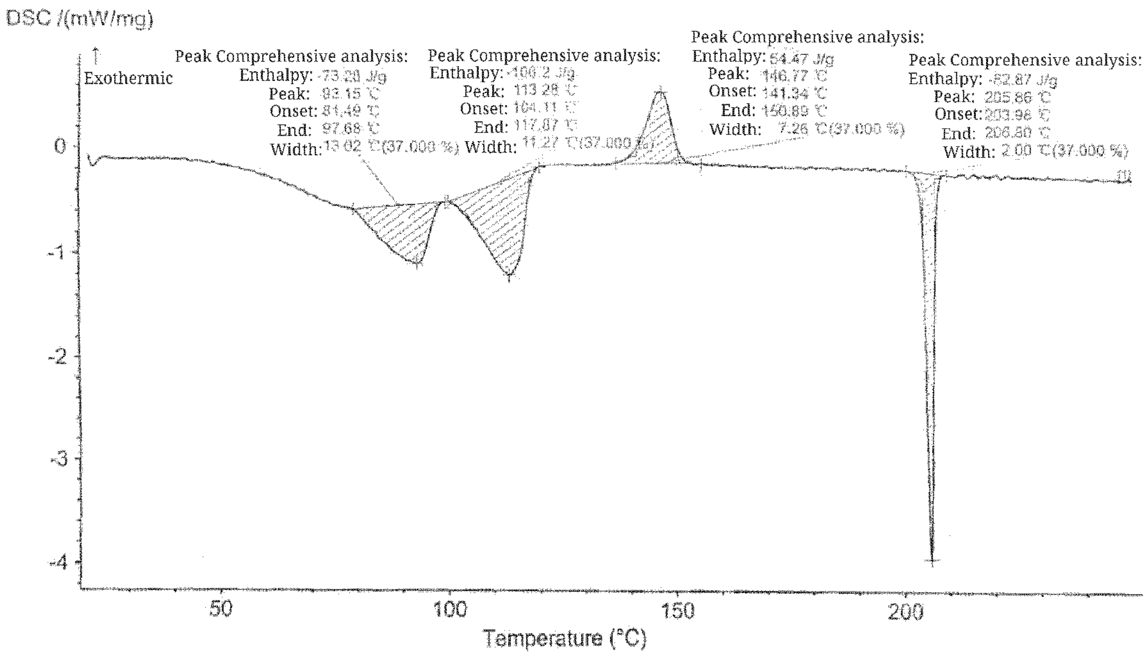


Figure 36

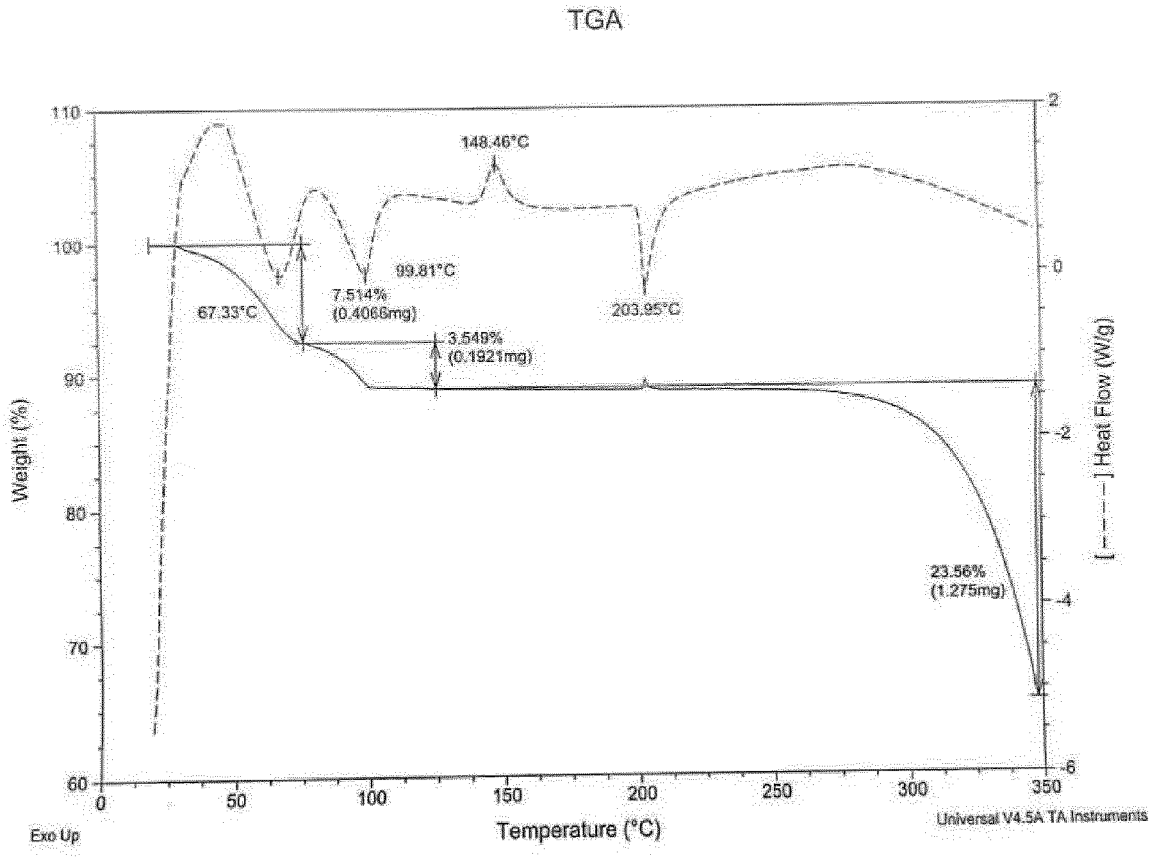


Figure 37

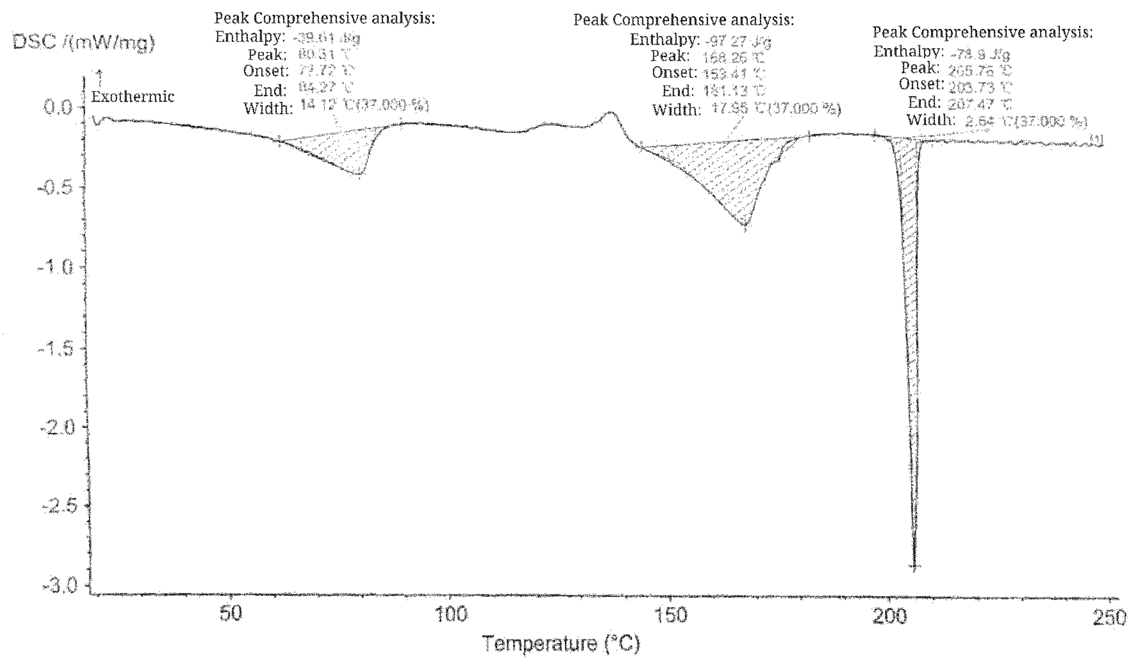


Figure 38

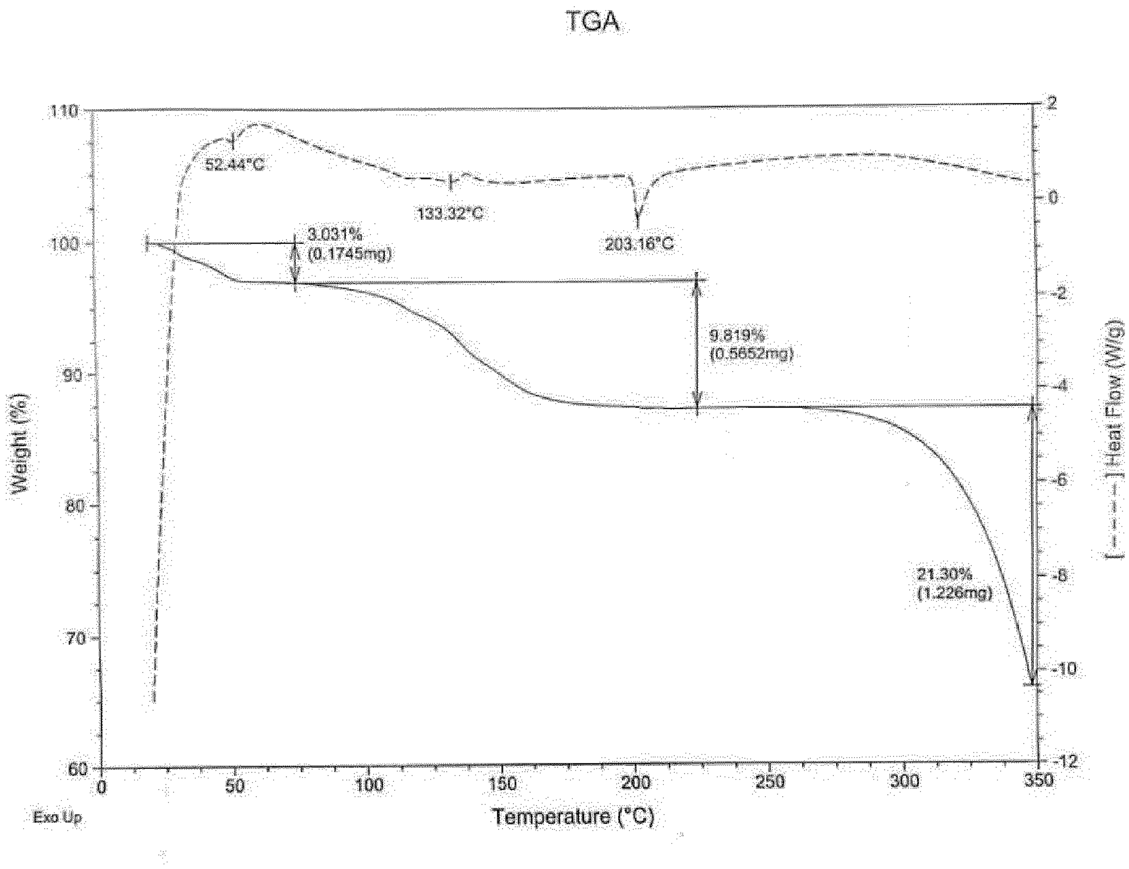


Figure 39

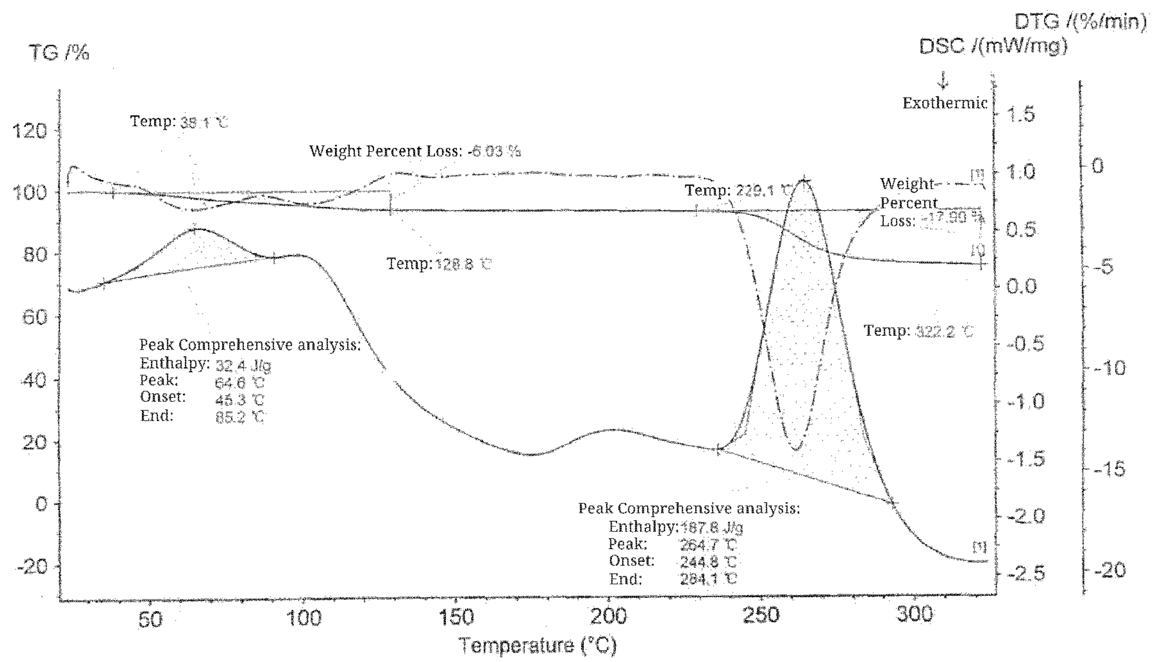


Figure 40

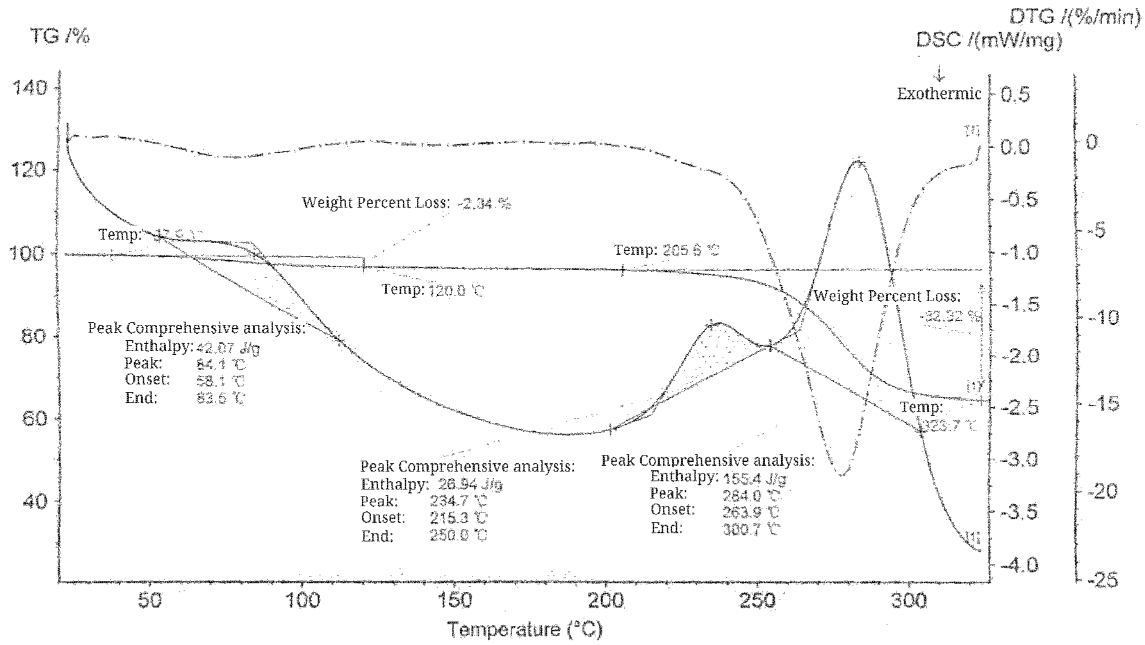


**Figure 41**

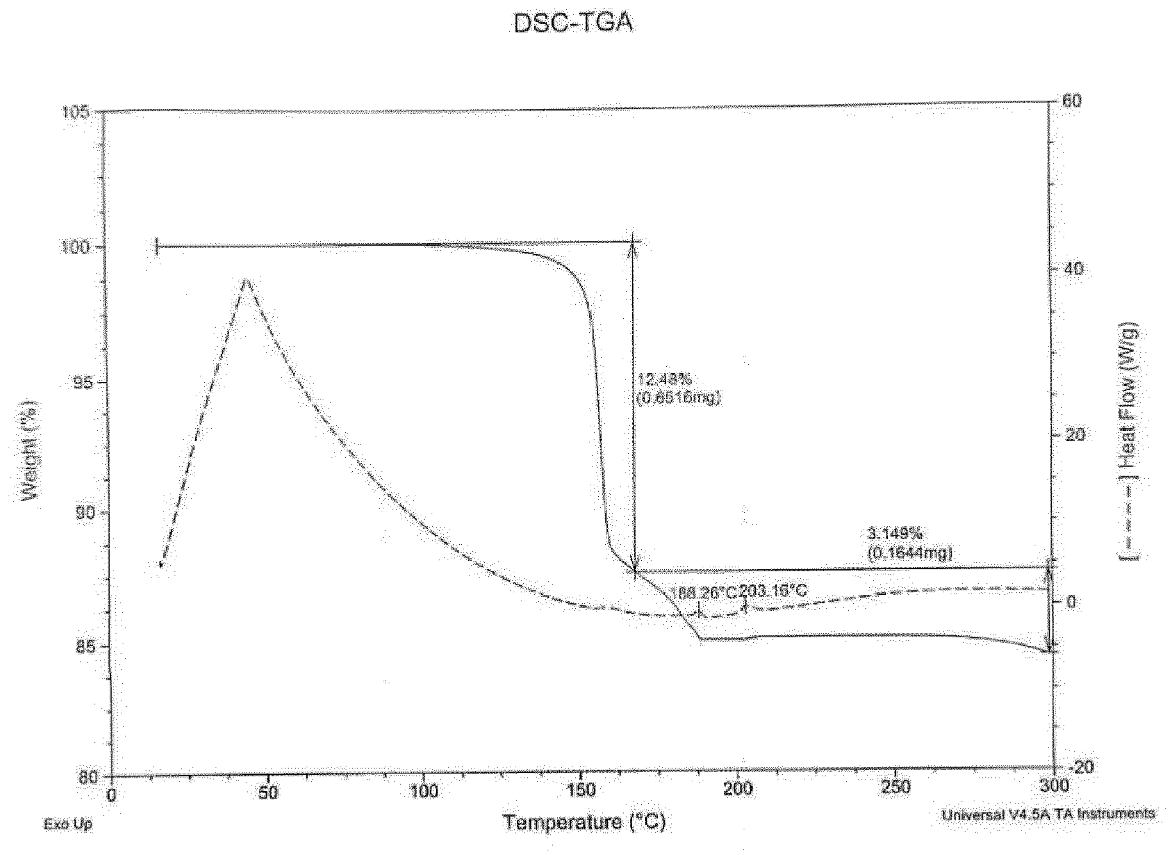


**Figure 42**

**INTERNATIONAL SEARCH REPORT**

International application No.
**PCT/CN2021/073285**

**A. CLASSIFICATION OF SUBJECT MATTER**

C07D 473/32(2006.01)i；A61K 31/52(2006.01)i；A61K 31/5377(2006.01)i；A61P 35/00(2006.01)i

According to International Patent Classification (IPC) or to both national classification and IPC

**B. FIELDS SEARCHED**

Minimum documentation searched (classification system followed by classification symbols)

C07D473/32，C07D473

Documentation searched other than minimum documentation to the extent that such documents are included in the fields searched

Electronic data base consulted during the international search (name of data base and, where practicable, search terms used)

CNABS, CNKI, VEN, STN(CAPLUS, REGISTRY): 石药集团, 芳胺基嘌呤, 芳胺基, 嘌呤, 药用盐, 盐, 水溶性, 水合物, arylamino, purine, salt, acid, solubility, soluble, hydrate, 1350544-93-2, 7732-18-5, specific compound structure search

**C. DOCUMENTS CONSIDERED TO BE RELEVANT**

| Category* | Citation of document, with indication, where appropriate, of the relevant passages | Relevant to claim No. |
|---|---|---|
| X | WO 2011147066 A1 (SI CHUAN UNIVERSITY et al.) 01 December 2011 (2011-12-01) description, pages 37, 79-80, 97-107 | 1-14 |
| A | 沈建民 等 (SHEN, Jianmin et al.). "三、成盐（离子-偶极型氢键）对有机药物溶解的影响 (non-official translation: 3, Effect of Salt (Ion - Dipole Hydrogen Bonding) on the Dissolution of Organic Drugs)" *《药物结构与制剂》 (non-official translation: Drug Structure and Formulation),* 31 August 1989 (1989-08-31), pp. 50-55 | 1-14 |
| A | 安宁 等 (AN, Ning et al.). "（二）溶解性和溶解度 (non-official translation: 2, Solubility and Dissolution)" *《标准化命题与学习方法指南高中化学》 (non-official translation: A Guide to the Study Methods of Standardized Test in High School Chemistry),* 31 December 1991 (1991-12-31), p. 8 | 1-14 |

☐ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **29 March 2021** | **12 April 2021** |
| Name and mailing address of the ISA/CN<br>**China National Intellectual Property Administration (ISA/CN)**<br>**No. 6, Xitucheng Road, Jimenqiao, Haidian District, Beijing 100088**<br>**China** | Authorized officer |
| Facsimile No. **(86-10)62019451** | Telephone No. |

Form PCT/ISA/210 (second sheet) (January 2015)

**INTERNATIONAL SEARCH REPORT**
**Information on patent family members**

International application No.
**PCT/CN2021/073285**

| Patent document cited in search report | Publication date (day/month/year) | Patent family member(s) | Publication date (day/month/year) |
|---|---|---|---|
| WO 2011147066 A1 | 01 December 2011 | CN 103003278 A | 27 March 2013 |
| | | JP 2013528164 A | 08 July 2013 |
| | | CN 102260263 A | 30 November 2011 |
| | | EP 2578584 B1 | 12 August 2020 |
| | | JP 5662564 B2 | 04 February 2015 |
| | | EP 2578584 A4 | 30 October 2013 |
| | | KR 101839915 B1 | 04 May 2018 |
| | | US 2013203986 A1 | 08 August 2013 |
| | | KR 20130109984 A | 08 October 2013 |
| | | US 9096601 B2 | 04 August 2015 |
| | | CN 105832740 A | 10 August 2016 |
| | | EP 2578584 A1 | 10 April 2013 |
| | | CN 103003278 B | 30 March 2016 |
| | | CN 105832740 B | 12 February 2019 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2011147066 A
- CN 2020127449 W


### Non-patent literature cited in the description


- Pharmaceutics. People's Medical Publishing House, 2003